# FASCICULE DE BREVET EUROPEEN

(11) **EP 3 221 459 B1**
(45) Date de publication et mention de la délivrance du brevet: **03.04.2019**
(21) Numéro de dépôt: 15805601.0
(22) Date de dépôt: 19.11.2015
(51) Int. Cl.: C12P 7/18, C12P 7/42

(54) **PROCÉDÉ DE PRODUCTION D'AU MOINS UN MÉTABOLITE D'INTÉRÊT PAR TRANSFORMATION D'UN PENTOSE DANS UN MICROORGANISME**
VERFAHREN ZUR HERSTELLUNG VON MINDESTENS EIN METABOLIT DURCH UMWANDLUNG EINER PENTOSE IN EINEM MIKROORGANISMUS
METHOD FOR PRODUCING A METABOLITE OF INTEREST BY MEANS OF A PENTOSE TRANSFORMING MICROORGANISM

(30) Priorité: 19.11.2014 FR 1461183
(43) Date de publication de la demande: 27.09.2017
(73) Titulaire: Institut National de la Recherche Agronomique (INRA), 75007 Paris (FR); Institut National des Science Appliquees - INSA, 31400 Toulouse (FR); Centre National de la Recherche Scientifique, 75794 Paris Cedex 16 (FR)
(72) Inventeur: WALTHER, Thomas, 31120 Lacroix-Falgarde (FR); CAM, Yvan, 31320 Castanet-Tolosan (FR); FRANCOIS, Jean-Marie, 31830 Plaisance du Touch (FR); ALKIM, Ceren, 31400 Toulouse (FR)
(74) Mandataire: Jacobacci Coralis Harle
(86) Numéro de dépôt international: PCT/FR2015/053138
(87) Numéro de publication internationale: WO 2016/079440

(56) Documents cités:
- WO-A1-2010/108909
- WO-A1-2013/126721
- JOSEFA BADIA ET AL: "Copyright C) 1991, American Society for Microbiology L-Lyxose Metabolism Employs the L-Rhamnose Pathway in Mutant Cells of Escherichia coli Adapted To Grow on L-Lyxose", JOURNAL OF BACTERIOLOGY, vol. 173, no. 16, 1 août 1991 (1991-08-01) , pages 5144-5150, XP055200009,
- HUAIWEI LIU ET AL: "Biosynthesis of ethylene glycol in Escherichia coli", APPLIED MICROBIOLOGY AND BIOTECHNOLOGY, vol. 97, no. 8, 1 avril 2013 (2013-04-01), pages 3409-3417, XP055080314, ISSN: 0175-7598, DOI: 10.1007/s00253-012-4618-7 cité dans la demande
- H. M. JAMES ET AL: "Models for the metabolic production of oxalate from xylitol in humans: A role for fructokinase and aldolase", AUST. J. EXP. BIOL. MED. SCI., vol. 60, 1982, pages 117-122, XP002749268,

## Description

### DOMAINE DE L'INVENTION

La présente invention se rapporte à un procédé de production d'au moins un métabolite d'intérêt par transformation d'un pentose dans un microorganisme. Elle fournit plus particulièrement un procédé de biosynthèse de l'éthylène glycol et/ou de l'acide glycolique dans un microorganisme exprimant une voie métabolique artificielle/synthétique d'assimilation des pentoses provenant avantageusement d'une source carbonée renouvelable.

### ETAT DE LA TECHNIQUE

L'éthylène glycol (EG) et l'acide glycolique (AG) sont des composés utilisés dans un large spectre d'applications industrielles dans l'industrie pétrochimique, en tant que précurseurs de polymères à base d'EG, comme le polyéthylène téréphtalate (PET) ou à base d'AG comme certaines résines thermoplastiques, mais aussi en tant que refroidissant dans les antigel pour automobiles pour l'EG ou, pour l'AG, dans l'industrie textile, dans l'industrie des huiles et gaz ainsi que dans un grand nombre de produits de beauté. Ces composés sont encore largement produits de façon pétrochimique. Néanmoins, l'ingénierie métabolique représente un secteur en fort développement depuis plus de 15 ans. Ce procédé comprend la conception de voies de biosynthèses artificielles et/ou l'optimisation de voies naturelles dans un organisme hôte et implique le transfert, l'expression et le couplage fonctionnel de multiples étapes enzymatiques au sein dudit organisme, pour permettre la production de molécules d'intérêt. La conception de voies de biosynthèses totalement artificielles dans le microorganisme hôte permet plus particulièrement de constituer un ensemble métabolique autonome de production de molécules d'intérêt. Ces méthodes de production présentent l'avantage d'utiliser, en tant que substrat, une source carbonée renouvelable, et représentent dorénavant une alternative réelle aux sources d'énergie fossiles. A cet égard, différentes voies métaboliques ont été décrites pour la bioproduction d'EG. Elles impliquent en particuliers des intermédiaires de la glycolyse. La société Genomatica a notamment décrit dans ses demandes de brevets WO2011130378 et WO2012177983 des voies métaboliques, principalement anaérobies, de synthèse de l'EG à partir de la sérine, 3-phosphohydroxypyruvate, du 3-phosphoglycérate ou du glyoxylate. Des techniques pour la bioproduction d'AG ont également été décrites. Ainsi, Mitsui Chemicals Inc. a décrit une méthode utilisant un microorganisme pour produire des acides hydrocarboxyliques à partir d'un alcool polyhydrique aliphatique ayant un groupe hydroxyle à son extrémité (EP 2 025 759 et EP 2 025 760). Il s'agit d'une méthode de bioconversion, comme celle décrite par Michihiko Kataoka dans un article sur la production d'AG, utilisant des microorganismes oxydant l'éthylène (Biosci. Biotechnol. Biochem., 2001). L'AG peut également être produit par bioconversion à partir du glyconitrile, en utilisant des nitrilases mutantes ayant une activité nitrilase augmentée, comme décrit par Dupont de Nemours dans les demandes WO2006/069110 et US7,445,917.

La biomasse lignocellulosique constitue une source de carbone durable et une alternative possible aux sources de carbone fossile. Elle génère un intérêt grandissant pour la biosynthèse de métabolites d'intérêt industriel. Dans ces matériaux carbonés, le D-xylose représente le deuxième sucre le plus abondant après le glucose. Or, les microorganismes n'utilisent pas préférentiellement les pentoses et lorsqu'ils le font, les rendements sont extrêmement faibles. Le développement de voies métaboliques assimilant ce sucre constitue donc un axe important de recherche pour l'industrie.

A l'heure actuelle, les principales sources d'utilisation des pentoses pour la production d'EG ou d'AG par des microorganismes, s'appuient sur l'utilisation de voies naturelles, optimisées pour la production de molécules d'intérêts et/ou sur l'ajout d'enzymes pour utiliser les produits de ces voies naturelles.

Ainsi une méthode de production biologique d'AG en optimisant les voies naturelles d'assimilation des pentoses est décrite dans les demandes WO2007140816 et WO2007141336 déposées par Metabolic Explorer. Ces demandes décrivent des organismes génétiquement modifiés à différents niveaux pour augmenter le flux de la voie glyoxylate, augmenter la conversion de glyoxylate en glycolate et/ou réduire le métabolisme du glycolate et de son intermédiaire, le glyoxylate. Une demande ultérieure de Metabolic Explorer (WO2010108909) décrit la possibilité d'agir en outre, pour optimiser la production d'AG, sur les voies de production du lactate (en atténuant les gènes codant pour la méthylglyoxal synthase et la D-lactate déshydrogénase), sur la transition du métabolisme aérobique/anaérobique en atténuant le gène ArcA régulant le contrôle de la voie respiratoire aérobie et/ou en atténuant les gènes codant pour des protéines important le glycolate.

Plus récemment des voies métaboliques de synthèse d'EG basées sur des voies métaboliques synthétiques d'assimilation des pentoses ont été élaborées. Ainsi Liu H et al. (Appl. Microbiol. Biotechnol, 2012, PMID :23233208), ont décrit des souches *E. coli* exprimant une voie métabolique de synthèse de l'éthylène glycol à partir de D-xylose et impliquant les enzymes (D)-xylose-déshydrogénase, (D)-xylonic-déshydratase, 2-déhydro-3déoxy-D-pentonate aldolase et glycolaldéhyde réductase. Néanmoins, les rendements en EG obtenus par cette méthode, de l'ordre de 275 mg par gramme de xylose, peuvent être améliorés.

Le Massachusetts Institute of Technology (MIT) a également, décrit dans la demande WO2013126721, une voie métabolique artificielle de production d'EG à partir de pentose. Cette voie implique la phosphorylation en position 1 du cycle du (D)-Ribulose ou du (L)-xylulose. Une telle voie nécessite cependant, pour l'assimilation des pentoses les plus abondants comme le (D)-Xylose et le (L)-Arabinose, l'expression de plusieurs isomérases et épimérases qui permettent la conversion du D-xylose en (D)-Xylulose puis en (D)-Ribulose et la conversion du (L)-Arabinose en (L)-Ribulose puis en (L)-Xylulose.

Il existe donc un besoin dans l'état de la technique pour de nouveaux procédés performants de production de métabolites d'intérêt par transformation de pentoses, notamment d'EG et/ou d'AG, avantageusement à partir de sources carbonées renouvelables et plus particulièrement basées sur l'assimilation directe des pentoses les plus abondants dans ces ressources naturelles comme le (D)-Xylose et le (L)-Arabinose.

### RESUME DE L'INVENTION

L'invention décrite dans la présente demande répond aux objectifs techniques mentionnés ci-dessus. Elle se rapporte à un procédé de transformation d'au moins un pentose dans un microorganisme pour la production d'au moins un métabolite d'intérêt, par assimilation dudit au moins un pentose dans un microorganisme. Elle fournit plus particulièrement un procédé de biosynthèse simple et économique pour produire de façon autonome, dans un microorganisme, un métabolite d'intérêt et notamment de l'éthylène glycol et/ou de l'acide glycolique, à partir d'une source carbonée renouvelable comme par exemple la lignocellulose et en particulier l'hémicellulose.

L'utilisation d'un tel substrat carboné renouvelable fournit une alternative durable à la production de métabolites d'intérêt (comme l'éthylène glycol et l'acide glycolique), qui sont des composés à haute valeur ajoutée pour l'industrie pétrochimique, et restent à ce jour largement produits par voie pétrochimique.

La nouvelle voie d'assimilation des pentoses décrite dans l'invention constitue une voie qui est parallèle aux voies naturelles d'assimilation des pentoses et qui n'existe pas à l'état naturel. Elle est ainsi largement indépendante des contraintes de régulation régissant les voies naturelles des cellules hôtes. Elle permet ainsi de s'affranchir des voies naturelles et de leurs régulations, pour produire des métabolites d'intérêts, comme l'éthylène glycol et/ou l'acide glycolique.

Une telle propriété permet sa portabilité de façon simplifiée vers un large spectre de microorganisme hôtes puisque leur métabolisme endogène n'interfère pas avec la voie synthétique de l'invention.

En outre, les calculs sur les rendements théoriques de la voie synthétique d'assimilation des pentoses décrite dans la présente demande, pour la production d'éthylène glycol et d'acide glycolique, permettent d'estimer une amélioration importante des rendements par rapport aux procédés de biosynthèse basés sur l'optimisation de voies naturelles et/ou une simplification de la mise en oeuvre et de la production.

Le procédé de l'invention est ainsi caractérisé en ce qu'il comprend les étapes suivantes:
(i) une opération de culture d'un microorganisme recombinant exprimant une voie synthétique d'assimilation des pentoses qui comprend au moins les étapes réactionnelles suivantes :
   a) phosphorylation en position 1 d'un pentose choisi parmi le (D)-Xylulose et/ou le (L)-Ribulose
   b) clivage du pentose-1-phosphate obtenu à l'issue de l'étape a), pour l'obtention de glycolaldéhyde et de dihydroxyacétone phosphate (DHAP), et
(ii) une opération de récupération d'au moins métabolite d'intérêt obtenu à l'issu de l'opération de culture (i).

De préférence, l'étape a) de la voie synthétique d'assimilation des pentoses peut être catalysée par une enzyme exprimée de façon recombinante choisie parmi le groupe constitué par la kétohexokinase C (Khk-C), la rhamnulose kinase (rhaB) et la fuculose kinase (fucK).

De préférence également, l'étape b) est catalysée par une aldolase, de préférence de classe I. Une aldolase de classe I selon l'invention est choisie typiquement parmi le groupe constitué par l'aldolase B (Aldo-B) et la Fructose-1,6 bisPphosphate aldolase (fructose 1,6 bP aldolase ou FbaB).

Selon un mode de réalisation le procédé de production est caractérisé en ce que :
- l'étape a) est catalysée par la KhkC, et
- l'étape b) est catalysée par l'aldolase B (Aldo-B) et/ou la fructose -1,6bP aldolase (FbaB)

Un mode particulier de réalisation du procédé de l'invention permet d'obtenir de l'éthylène glycol (EG) et/ou de l'acide glycolique (AG).

Dans un tel procédé, la voie synthétique d'assimilation des pentoses comprend en outre les étapes suivantes :
c) réduction du glycolaldéhyde obtenu à l'issue de l'étape b) en éthylène glycol, et/ou
c') oxydation du glycolaldéhyde obtenu à l'issue de l'étape b), en acide glycolique.

Les modes de réalisation décrits ci-après peuvent être combinés entre eux, sauf précision contraire dans le procédé de l'invention.

L'étape c) peut être catalysée par une glycolaldéhyde réductasenotamment choisie dans le groupe constitué par: l'aldéhyde réductase (YqhD), la glycérol déhydrogénase (GldA) et la propane-1,2-diol oxidoréductase (FucO).

L'étape c') peut être catalysée par une glycolaldéhyde déhydrogénase, notamment la lactaldéhyde déhydrogénase (AldA).

Dans un procédé de l'invention le microorganisme est préférentiellement mis en culture sur un milieu carboné contenant du (D)-Xylose et/ou du (L)-Arabinose.

Dans un mode de réalisation préféré du procédé de l'invention, le milieu de culture comprend un hydrolysat de biomasse comprenant de l'hémicellulose.
Typiquement, la voie synthétique d'assimilation des pentoses du procédé selon l'invention comprend, préalablement à l'étape a) au moins une des étapes suivantes :
- une étape de transport du (D)-xylose et/ou (L)-arabinose dans le microorganisme ;
- une étape de conversion du (D)-Xylose en (D)-Xylulose, et/ou
- une étape de conversion du (L)-Arabinose en (L)-Ribulose.

La conversion du (D)-Xylose en (D)-Xylulose peut être catalysée par une (D)-Xylose isomérase et/ou par l'action d'une (D)-xylose réductase et d'une xylitol déshydrogénase.

La conversion du (L)-Arabinose en (L)-Ribulose peut être catalysée par une (L)-arabinose isomérase et/ou par l'action d'une (L)-arabinose réductase et d'une arabitol déshydrogénase.

Selon le procédé de production selon l'invention le microorganisme recombinant peut être une bactérie de préférence choisie parmi le groupe constitué par *Enterobacteriaceae, Clostridiaceae, Bacillaceae, Streptomycetaceae, Streptococcaceae, Methylobacteriacae,* et *Corynebacteriaceae,* de préférence *Escherichia coli, Bacillus subtilis, Corynebacterium glutamicum, Clostridium acetobutylicum, Methylobacterium extorquens,* ou *Lactococcus lactis.* Le microorganisme peut également être une levure de préférence choisie parmi des *Saccharomycetaceae, Pichiaceae,* et *Schizosaccharomycetaceae,* de préférence *Saccharomyces cerevisiae, Schizosaccharomyces pombe, Kluyveromyces lactis, Kluyveromyces marxianus, Pichia jadinii, Scheffersomyces stipitis,* or *Pichia pastoris.* Le microorganisme de l'invention peut également être un champignon, de préférence choisi parmi le groupe constitué par *Penicillium, Aspergillus, Chrysosporium* ou *Trichoderma.*

De préférence, les activités (D)-Xylulose-5 kinase et/ou (L)-Ribulose-5 kinase du microorganisme utilisé dans le procédé de l'invention, ont été supprimées.

L'invention se rapporte également à un microorganisme recombinant qui exprime une voie synthétique d'assimilation des pentoses comprenant au moins les acides nucléique codant pour les enzymes suivantes :
i) une enzyme apte à phosphoryler en position 1 les pentoses choisis parmi le (D)-Xylulose et/ou le (L)-Ribulose.
ii) Une enzyme apte à cliver le dit pentose phosphorylé en position 1 en glycolaldéhyde et DHAP,

Avantageusement, les voies naturelles d'assimilation des pentoses d'un tel microorganisme ont été supprimées.

Généralement un microorganisme exprimant la voie synthétique d'assimilation de l'invention est en outre caractérisé en ce que les activités (D)-xylulose-5 kinase et/ou (L)-ribulose-5 kinase ont été supprimées, et/ou en ce qu'il porte au moins une des modifications suivantes :
- surexpression d'un gène codant pour une glyoxylate réductase ;
- surexpression d'un gène codant pour une isocitrate lyase ;
- délétion des gènes codant pour des malate synthases ;
- délétion des gènes codant pour des glyoxylate carboligases;
- délétion des gènes codant pour des gènes codant pour des glycolate oxydases et/ou des glycolates déshydrogénases
- délétion des gènes codant pour des 2-kéto-4-hydroxyglutarate aldolases, notamment la Entner-Doudouroff Aldolase et/ou des phosphogluconate déhydratases;
- délétion d'un gène répresseur de la réponse aérobie, notamment le gène *arcA* ;
- atténuation et notamment délétion de l'expression de l'isocitrate déhydrogénase ;
- délétion des gènes codants pour des systèmes d'internalisation de l'acide glycolique ;
- atténuation des voies métaboliques qui amènent à la production de produit secondaires comme l'acétate, le lactate ou l'éthanol ;
- surexpression d'au moins un gène codant pour un transporteur des sucres.

De préférence, le microorganisme recombinant comprend :
- au moins un acide nucléique exogène codant pour la kétohexokinase C et
- au moins un acide nucléique exogène codant pour l'aldolase B.

Avantageusement, ledit microorganisme peut comprendre les modifications suivantes :
- surexpression du gène codant pour la glycolaldéhyde réductase principale ;
- délétion du gène codant pour la glycolaldéhyde déshydrogénase.

En variante, le microorganisme peut comprendre au moins l'une des modifications suivantes pour optimiser la production d'acide glycolique :
- surexpression du gène codant pour la glycolaldéhyde déshydrogénase;
- délétion d'au moins un des gènes codant pour au moins l'une des sous-unités de la glycolate oxydase.

Typiquement un microorganisme adapté pour optimiser la production d'acide glycolique comprend les modifications suivantes :
- surexpression d'un gène codant pour une glyoxylate réductase ;
- surexpression d'un gène codant pour une isocitrate lyase, accompagné ou non de la délétion de son répresseur transcriptionnel ;
- délétion des gènes codant pour des malate synthases ;
- délétion des gènes codant pour des glyoxylate carboligases
- délétion des gènes codant pour des glycolate oxydases et/ou des glycolate déshydrogénases ;
- délétion des gènes codant pour des 2-kéto-4-hydroxyglutarate aldolases, notamment la Entner-Doudouroff Aldolase et/ou des phosphogluconate déhydratases;
- délétion d'un gène codant pour un répresseur des gènes impliqués dans le métabolisme respiratoire, notamment le gène *arcA* ;
- atténuation et notamment délétion de l'expression de l'isocitrate déhydrogénase ;
- éventuellement la surexpression d'un gène codant pour un transporteur des sucres.

De préférence encore, notamment dans le cas où le microorganisme recombinant pour la production de l'acide glycolique est *Escherichia coli,* le microorganisme comprend les modifications suivantes :
- la surexpression du gène *aldA* codant pour une glycolaldéhyde déshydrogénase ;
- la surexpression du gène *ghrA*
- la surexpression du gène *aceA* éventuellement accompagné de la délétion du gène *iclR* codant pour un répresseur transcriptionnel de la voie du glyoxylate ;
- la délétion des gènes *aceB* et *glcB*
- la délétion d'au moins un des gènes *glcDEFG* ;
- la délétion du gène *glc* ;
- la délétion des gènes *edd-eda* ;
- la délétion du gène *arcA* ;
- la délétion du gène *icd* ;
- la surexpression du gène *galP*

### FIGURES

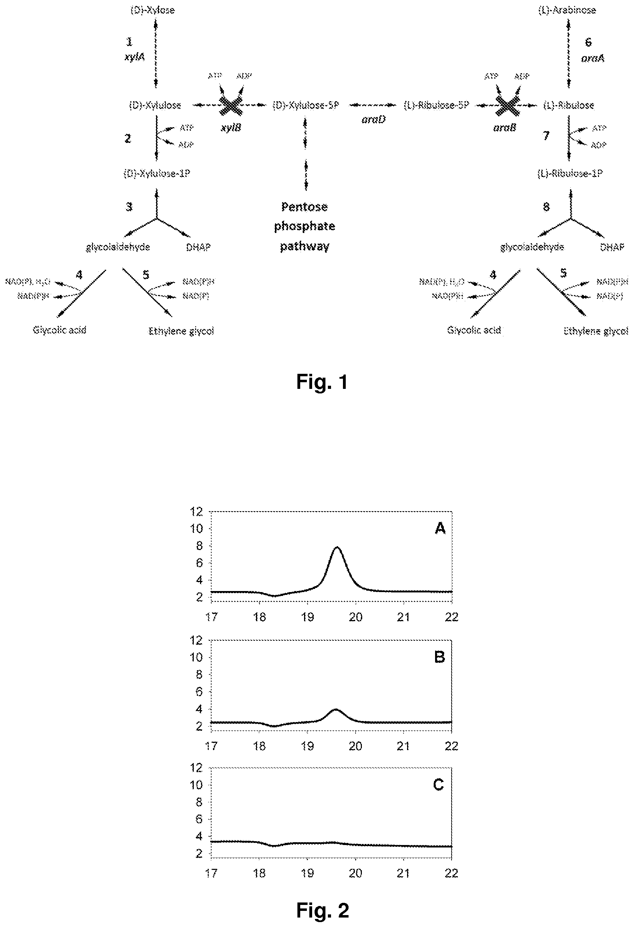

Figure 1. Voies naturelles et voies synthétiques d'assimilation du (D)-xylose et du (L)-arabinose. Les réactions catalysées par les enzymes naturelles sont représentées en pointillées. Les réactions catalysées par les enzymes synthétiques sont représentées en traits pleins. (1) (D)-xylose isomerase, (2) (D)-xylulose-1 kinase, (3)(D)- xylulose-1-phosphate aldolase, (4) glycolaldehyde dehydrogenase, (5) glycolaldehyde reductase, (6) (L)-arabinose isomerase, (7) (L)-ribulose-1 kinase, (8) (L)-ribulose-1-phosphate aldolase. Le nom des gènes sous les réactions correspondent aux gènes d'*Escherichia coli* codant pour l'enzyme avec l'activité correspondante.
Figure 2. Synthèse *in vitro* d'éthylène glycol par la voie synthétique d'assimilation du (D)-xylose. Chromatogrammes HPLC de (A) une solution à 10 mM d'éthylène glycol et en (B) d'un mélange réactionnel composé d'une (D)-xylulose-1 kinase (Khk-C, 0,005 Unité/ml), (D)-xylulose-1-phosphate aldolase (AldoB, 1 Unité/ml (Sigma-Aldrich-A6338)) et d'une glycolaldehyde reductase (GldA, 1 Unité/ml (Sigma-aldrich G3512-250U)) ou en (C) d'une (D)-xylulose-1 kinase (Khk-C, 0,005 Unité/ml)) et d'une glycolaldehyde reductase (GldA, 1 Unité/ml (Sigma-aldrich G3512-250U)). Les enzymes ont été incubées pendant 3 h à 37 °C dans un tampon de réaction Hépès contenant 100 mM Hepes ; 85 mM KCL ; 7,5 mM MgCl₂) à pH = 7 ; 2 mM ATP ; 5 mM ZnCl₂; 0,4 mM NADH. Les réactions ont été déclenchées par l'ajout de 5 mM de (D)-xylulose.
   Légende : Intensité (ordonnée) en fonction du temps en minutes (abscisse)
Figure 3. Synthèse *in vitro* d'éthylène glycol par la voie synthétique d'assimilation du (L)-arabinose. Chromatogrammes HPLC de (A) une solution à 1 mM d'éthylène glycol et en (B) d'un mélange réactionnel composé d'une (L)-ribulose-1 kinase, (L)-ribulose-1-phosphate aldolase et d'une glycolaldehyde reductase. Les enzymes ont été incubées pendant 3 h à 37 °C dans un tampon de réaction Hépès contenant 55 mM Hepes ; 45 mM KCL ; 4 mM MgCl₂) à pH = 7 ; 4 mM ATP ; 0,4 mM NADH. Les réactions sont déclenchées par l'ajout de 20 mM de (L)-ribulose.
   Légende : Intensité (ordonnée) en fonction du temps en minutes (abscisse)
Figure 4. Croissance de souches *d'E. coli* en milieu minimum contenant du (D)-xylose Légende : DO à 600 nm (ordonnée) en fonction du temps en minutes (abscisse)
Figure 5. Suivi de croissance et de production de métabolites sur xylose d'une souche de E coli MG1655 *ΔxylB* pEXT20 *khk-C-aldoB.*
   Légende : DO à 600 nm (ordonnée à gauche) et xylose en mM, éthylène glycol en mM et acide glycolique en mM (ordonnée à droite) en fonction du temps en heures (abscisse)
Figure 6. Production d'éthylène glycol (en mM sur l'ordonnée) par des mutants de glycolaldéhyde réductases candidates après incubation pendent 12 h en présence de 10 mM glycolaldéhyde.
Figure 7. Optimisation de la production d'éthylène glycol dans différents mutants selon l'invention exprimée en rendement en mol/mol de xylose (en ordonnée) via la voie métabolique synthétique d'assimilation du (D)-xylose.
Figure 8. Optimisation de la production d'acide glycolique dans différents mutants selon l'invention exprimée en rendement en mol/mol de xylose (en ordonnée) via la voie métabolique synthétique d'assimilation du (D)-xylose.
Figure 9. Croissance de la souche 905 en milieu minéral en présence de glucose et (D)-xylose. Légende : DO à 600nm (ordonnée à gauche) et glucose, xylose, acide glycolique et acétate en mM en mM (ordonnée à droite) en fonction du temps en heures (abscisse).
Figure 10. Croissance de la souche 979 en milieu minéral en présence de glucose et (D)-xylose.. Légende : DO à 600nm (ordonnée à gauche) et glucose, xylose, acide glycolique et acétate en mM en mM (ordonnée à droite) en fonction du temps en heures (abscisse).
Figure 11. Croissance en milieu minéral en présence de xylose comme seule source de carbone de souches CEN.PK-2-1. Légende : DO à 600nm (ordonnée à droite) et xylose en mM en mM (ordonnée à gauche) en fonction du temps en heures (abscisse).
Figure 12. Croissance en milieu minéral et xylose comme seule source de carbone de souches TMB3001. Légende : DO à 600nm en fonction du temps en heures.
Figure 13. Croissance en milieu minéral M9 et (L)-arabinose de souches ΔaraB avec ou sans voie synthétique. Légende : DO à 600nm en fonction du temps en heures.

### DEFINITIONS:

Sauf indication particulière, les termes techniques et scientifiques employés dans la présente demande ont la signification usuelle, entendue par l'homme du métier apte à mettre en oeuvre l'invention.

Sauf précision contraire également, les différents modes de réalisation décrits ci-après peuvent être combinés entre eux dans la mise en oeuvre de l'invention.

Par « voie d'assimilation des pentoses », on entend selon l'invention, une voie métabolique, c'est à dire un ensemble de réactions chimiques se déroulant dans le microorganisme, catalysées par une série d'enzymes qui agissent de manière séquentielle, utilisant les pentoses comme substrat initial et aboutissant à leur transformation pour la formation des métabolites d'intérêts.

Par voie naturelle d'assimilation des pentoses, on entend l'assimilation des pentoses impliquant leur phosphorylation en position 5, puis leur utilisation dans la voie métabolique dite des pentoses phosphates, qui existe de façon naturelle dans la plupart des cellules eucaryotes et procaryotes. Typiquement, le (L)-arabinose est isomérisé en (L)-ribulose, qui est alors phosphorylé en position 5. Le (L)-ribulose-5-phosphate obtenu est épimérisé sur le carbone C3 pour produire du (D)-xylulose-5-phosphate, substrat de la voie des pentoses phosphate.

L'expression « voie synthétique » ou « voie métabolique synthétique » signifie selon l'invention, que ladite voie métabolique n'est pas mise en oeuvre naturellement par le microorganisme. Cette condition est typiquement respectée lorsqu'au moins une des enzymes dudit microorganisme catalysant au moins une des étapes a) ou b) de la voie métabolique de l'invention n'est pas exprimée naturellement ou lorsque ladite au moins une enzyme lorsqu'elle est exprimée ne catalyse pas ladite au moins une étape a) ou b).

A titre d'exemples, (i) la voie métabolique impliquant la phosphorylation en position 1 d'un pentose choisi parmi le (D)-xylulose et le (L)-Ribulose, par la Khk-C d'origine humaine dans une cellule non humaine et typiquement dans un microorganisme, (ii) la voie métabolique impliquant la phosphorylation en position 1 d'un pentose choisi parmi le (D)-xylulose et le (L)-Ribulose, par la rhamnulose kinase RhaB dans un microorganisme et (iii) la voie métabolique impliquant la phosphorylation en position 1 d'un pentose choisi parmi le (D)-xylulose et le (L)-Ribulose, par la fuculose kinase FucK dans un microorganisme, sont des voies synthétiques selon l'invention.

L'expression des enzymes RhaB et FucK chez *E. coli* dépend de la présence de leur substrat naturel, soit respectivement le (L)-rhamnulose et le (L)-fucose. En l'absence de leur substrat naturel (ou lorsque celui est présent dans une concentration trop faible), l'expression de ces enzymes dans un microorganisme et notamment chez *E. coli* peut être obtenue de façon recombinante sous le contrôle d'un promoteur de façon inductible ou constitutive.

Le terme « transformation « ou « transfection » se réfère à l'acquisition de nouveaux gènes fonctionnels dans une cellule, après l'incorporation d'acides nucléiques exogènes.

Le terme « modification » ou « modifier » s'agissant du niveau de protéine ou d'activité enzymatique produit par une cellule hôte se réfère au contrôle des niveaux de protéine ou d'activité enzymatique produite durant la culture, de sorte que ces niveaux soient augmentés ou diminués comme souhaité.

Le terme « modifié », s'agissant d'un acide nucléique ou d'un polynucléotide signifie que l'acide nucléique a été modifié par rapport à la version sauvage contenue dans la cellule hôte, notamment par une mutation de type substitution, insertion, délétion d'une partie ou de la totalité dudit acide nucléique, ou que ledit acide nucléique a été lié de façon fonctionnelle à une région de contrôle de la transcription.

Par « gène », on entend selon l'invention, un segment d'ADN impliqué dans le codage d'ARN ribosomiques, d'ARN régulateurs, d'ARN de transfert, de séquences régulatrices (comprenant typiquement une région promoteur) liées de façon fonctionnelles à l'expression d'un peptide, d'un polypeptide ou d'une protéine, incluant les régions codantes (transcrites en ARN messager) et non codantes précédant ou terminant la région codante ainsi que les introns (régions non codantes séparant les régions codantes ou exons).

Le terme « lié de façon fonctionnelle » se rapporte à une juxtaposition d'éléments de telle sorte que leur disposition leur permette d'être fonctionnellement liés. Une séquence régulatrice, contenant typiquement une région promoteur est fonctionnellement liée à une région codante lorsqu'elle contrôle la transcription de la région codante, et un site de liaison d'un ribosome est fonctionnellement lié à une région codante lorsqu'il est positionné de façon à permettre la translation de l'ARNm.

Le terme « inactivation », ou « suppression » ou « atténuation » se rapporte à l'expression diminuée ou réduite ou significativement réduite d'un gène ou à l'activité diminuée ou réduite d'une protéine, ou du produit d'un gène, typiquement d'une enzyme. Différentes méthodes connues de l'homme du métier peuvent être employées à cette fin, comme :
- l'introduction d'une mutation dans le gène résultant en une expression réduite du gène ou en l'expression d'une protéine dont l'activité est réduite,
- le remplacement du promoteur naturel par un promoteur peu actif résultant en une faible expression du gène,
- l'utilisation d'élément déstabilisant l'ARMm correspondant à la protéine, ou
- la délétion du gène.

Typiquement, l'atténuation d'un gène ou d'une protéine est définie par une activité de la protéine exprimée par ledit gène diminuée d'au moins 50 %, de préférence d'au moins 60 %.

L'inactivation ou la délétion ou la suppression d'un gène ou d'une protéine est définie par une activité résiduelle de la protéine produit dudit gène inférieure à 20 %, notamment inférieure à 10 %, notamment inférieure à 5%.

Le terme « expression » correspond à la transcription et à la translation d'un gène vers une protéine, produit dudit gène.

Le terme « surexpression » correspond à une expression augmentée vis-à-vis de l'expression naturelle dudit gène dans la même cellule hôte. Typiquement la surexpression d'une protéine est définie par une activité d'au moins 200 % de ladite protéine par rapport à son expression naturelle dans la cellule hôte.

La surexpression d'une protéine peut être obtenue selon différentes technique connues de l'homme du métier, comme :
- la mutation d'une protéine, visant à obtenir une forme plus active ou résistante à l'inhibition,
- l'augmentation de l'expression du gène codant pour ladite protéine (par exemple par l'introduction d'un promoteur spécifique régulant l'expression du gène),
- l'addition de multiples copies du gène dans la cellule hôte, etc.

Des cellules hôtes compatibles avec l'invention peuvent exprimer une copie endogène d'un ou plusieurs gènes codant pour une protéine d'intérêt selon l'invention, ainsi qu'éventuellement une copie recombinante dudit gène.

Un acide nucléique codant pour une enzyme associée à la présente invention peut être introduite dans une cellule hôte selon toute technique standard connue de l'homme du métier. A titre d'exemple, les acides nucléiques peuvent être introduits par transformation (chimique ou électroporation), transfection, infection, transduction, etc.

Les gènes codant pour des protéines associées à l'invention peuvent être exprimés de façon extra-chromosomique dans un vecteur d'expression recombinant ou peuvent être intégré au sein des chromosomes.

On entend par « vecteur » selon l'invention, un acide nucléique au sein duquel est insérée une séquence d'intérêt, par restriction et ligation de façon à être liée de façon opérante à des séquences de régulation, pour l'expression en tant que transcrit d'ARNm dans une cellule hôte. Les vecteurs sont composés d'ARN ou de préférence d'ADN et incluent de façon non limitative les plasmides, phagemides, génomes viraux, bactériophages et chromosomes bactériens.

Un vecteur selon l'invention peut comprendre une ou plusieurs séquences marqueurs pour l'identification des cellules transformées avec ledit vecteur. De tels marqueurs incluent par exemple des gènes codant pour des protéines augmentant ou diminuant la résistance ou la sensibilité à des composés antibiotiques, des gènes codant pour des enzymes dont l'activité est détectable par des essais standards (par exemple, luciférase, galactosidase, phosphatase alcaline, etc.) ou des gènes modifiant le phénotype des cellules transformées (par exemple codant pour la GFP, *i.e.* : « green fluorescent protein »).

Les séquences régulatrices (promoteurs) utilisées dans l'expression des protéines recombinantes de l'invention, peuvent être des séquences régulatrices endogènes (c'est-à-dire le promoteur natif du gène auquel il est associé) ou exogènes. Le promoteur peut être inductible ou constitutif.

Par « cellule hôte », ou microorganisme « hôte », on entend tout type de cellule susceptible de subir une transformation, transfection, transduction, etc., avec une construction d'acide nucléique ou un vecteur d'expression comprenant un ou plusieurs polynucléotides, en particulier un ou plusieurs polynucléotide codant pour des enzymes décrites dans la demande.

Les kinases sont des enzymes du groupe des transférases catalysant les réactions de phosphorylation par l'ajout d'un ion phosphate à une molécule cible.

Les oxydoréductases sont des enzymes catalysant les réactions d'oxydoréduction en transférant les ions H⁺ et des électrons. Elles sont associées à des coenzymes d'oxydoréduction (NAD, FAD, FMN, etc.)

Une déshydrogénase(ou déhydrogénase) est une enzyme qui oxyde un substrat par le transfert d'un ou plusieurs ions (H+) à un accepteur, généralement un coenzyme type NAD+/NADP+ ou flavine comme le FAD ou le FMN.

Une aldéhyde déshydrogénase est une enzyme de type déshydrogénase qui catalyse l'oxydation des aldéhydes.

Lorsque les enzymes mentionnées dans la présente demande sont identifiées par leur activité spécifique, une telle définition inclut tous les polypeptides ayant la même activité spécifique et qui sont présents dans différentes cellules et notamment différents microorganismes. L'invention se rapporte donc également aux protéines homologues des protéines de références mentionnées dans la présente demande ayant la même activité que les protéines de référence, ainsi qu'aux gènes codant pour lesdites protéines homologues.

En l'absence de précision, les gènes et les protéines mentionnés dans la présente demande sont identifiés en référence à *E. coli* (notamment à la souche MG1655). La Khk-C et l'aldolase B sont identifiées en référence à *H. sapiens.* Néanmoins, des protéines et donc des gènes homologues aux protéines (et aux gènes les codants) identifiées dans la présente demande peuvent être retrouvées dans différents microorganismes.

Une protéine homologue d'une protéine de référence selon l'invention possède la même fonction, c'est-à-dire le cas échéant pour une enzyme, catalyse la même réaction que l'enzyme de référence. Un gène homologue d'un gène codant pour une protéine de référence selon la présente invention code pour une protéine homologue telle que définie ci-dessus.

Typiquement, à partir du nom de la protéine et de sa séquence, l'homme du métier est capable d'identifier dans d'autres organismes des équivalents des protéines mentionnées dans la présente demande. Ce travail de routine est usuellement effectué en utilisant des séquences consensus identifiées par des alignements de séquences avec d'autres protéines issues de différents organismes.

De préférence également, une protéine homologue d'une protéine de référence correspond à une enzyme ayant au moins 30 % d'identité de séquence, de préférence 40 %, 50 %, 55 %, 60 %, 65 %, 70 %, 75 %, 80 %, 85 %, 90 % ou 95 % d'identité avec la séquence de la protéine de référence.

Afin de déterminer le pourcentage d'identité de deux séquences d'acides aminés pour les besoins de l'invention, les séquences seront alignées pour permettre une comparaison optimale. Des espaces (gap) peuvent être introduits dans l'une ou l'autre des séquences à aligner afin de permettre un alignement optimal, et les séquences non homologues peuvent être ignorées pour la comparaison.

Le pourcentage d'identité des deux séquences d'acides aminés comparées peut être obtenu comme décrit dans le livre de D. Voet et J.G. Voet, Biochimie (2nd Edition, De Boeck & Larcier, 2005, section 7.4, paragraphe B). Les alignements sont réalisés avec le logiciel CLUSTAL W (version 1.82) avec les paramètres suivants : (1) CPU MODE = ClustalW mp ; (2) ALIGNMENT = « full » ; (3) OUTPUT FORMAT = « aln w/numbers » ; (4) OUTPUT ORDER = « aligned » ; (5) COLOR ALIGNMENT = « no » ; (6) KTUP (word size) = « default » ; (7) WINDOW LENGTH = « default » ; (8) SCORE TYPE = « percent » ; (9) TOPDIAG = « default » ; (10) PAIRGAP = « default » ; (11) PHYLOGENETIC TREE/TREE TYPE = « none » ; (12) MATRIX = « default » ; (13) GAP OPEN = « default » ; (14) END GAPS = « default » ; (15) GAP EXTENSION = « default » ; (16) GAP DISTANCES = « default » ; (17) TREE TYPE = « cladogram » et (18) TREE GRAP DISTANCES = « hide ».

La lignocellulose est composée de lignines, d'hémicelluloses et de cellulose en proportions variables. Les hémicelluloses sont un des trois composants principaux de la biomasse lignocellulosique, et représentent environ 20-40 % en poids de ladite biomasse.

Par « hémicellulose », on entend selon l'invention, un groupe de polysaccharides complexes qui se caractérisent par leur solubilité dans des solutions alcalines (par exemple KOH 1M) et leur insolubilité dans l'eau. Les hémicelluloses sont définies structurellement comme des polysaccharides dont le squelette est composé de résidus β-(1,4)-D-pyranose, où l'O4 est en position équatoriale. De courtes chaines latérales sont fixées sur le squelette. Les hémicelluloses comprennent des xylanes, des arabinoxylanes, des xyloglucanes, des glucuronoxylanes, et des glucomannanes. Leur hydrolysation, par exemple réalisée par la mise en contact d'un matériel lignocellulosique avec de l'acide sulfurique dilué à des pressions et des températures élevées, conduit à la libération des sucres monomériques. Selon la nature de la matière première et les conditions de l'hydrolyse, les pourcentages en xylose, glucose, et arabinose, varient respectivement de 60 à 80%, de 10 à 30 %, et de 10 à 30 % en poids par rapport au poids total de l'hydrolysat de lignocellulose.

Des lignocelluloses issues des bois durs (typiquement les arbres feuillus), des épis de maïs, des herbes, des feuilles, et des journaux sont particulièrement riches en sucres hémicellulosiques (Jorgensen H et al., Enzymatic conversion of lignocellulose into fermentable sugars: Challenges and opportunities. Biofuels, Bioprod. Bioref. 2007, 1, 119-134). Ils représentent des sources de matières premières préférées pour la mise en oeuvre des microorganismes modifiées selon l'invention. Par « amorce » ou « primer », on entend une courte séquence d'ADN, complémentaire du début d'une matrice, servant de point de départ à la synthèse du brin complémentaire de ladite matrice par une ADN polymérase.

### DESCRIPTION DETAILLEE

### Voie synthétique d'assimilation des pentoses

La présente invention se rapporte à un procédé de transformation d'un pentose dans un microorganisme recombinant exprimant une voie synthétique d'assimilation des pentoses, pour la production d'au moins un métabolite d'intérêt.

Ce procédé selon l'invention comprend :
(i) une opération de culture d'un microorganisme recombinant exprimant une voie synthétique d'assimilation des pentoses, illustrée de manière générale sur la figure 1, qui comprend au moins les étapes suivantes :
   a) la phosphorylation en position 1 d'un pentose choisi parmi le (D)-Xylulose et/ou le (L)-Ribulose, pour l'obtention respectivement de (D)-Xylulose-1P et/ou de (L)-Ribulose-1P,
   b) le clivage du pentose-1-phosphate obtenu à l'issue de l'étape a) ((D)-Xylulose-1P et/ou (L)-Ribulose-1P), pour l'obtention de glycolaldéhyde et de dihydroxyacétone phosphate (DHAP),
   ladite voie permettant d'obtenir au moins un métabolite d'intérêt, et
(ii) une opération de récupération dudit au moins un métabolite d'intérêt obtenu à l'issu de l'opération de culture (i).

Par « phosphorylation », on entend avantageusement une addition d'un groupe phosphate, en l'espèce un phosphoryl PO₃²⁻.

Par « métabolite d'intérêt », on entend notamment le glycolaldéhyde et le DHAP, mais aussi leurs dérivés susceptibles d'être obtenus par des réactions d'oxydation ou de réduction de ces composés, en particulier l'éthylène glycol (EG), l'acide glycolique (AG) et leurs dérivés.

Par « dérivés de de l'acide glycolique », on entend notamment :
- les esters de glycolate, comme l'éthyl ester glycolate ou le méthyl ester glycolate, ainsi que :
- les polymères contenant du glycolate comme l'acide polyglycolique.
- ainsi que l'acide glyoxylique issu d'une oxydation de l'acide glycolique.

A noter que dans la présente demande, les termes « acide glycolique » et « glycolate » ainsi que les termes « acide glyoxylique » et « glyoxylate » sont utilisés en tant que synonymes.

De préférence, la voie synthétique d'assimilation des pentoses exprimée par le microorganisme comprend donc en outre avantageusement les étapes suivantes :
c) réduction du glycolaldéhyde obtenu à l'issue de l'étape b) en éthylène glycol, ou
c') oxydation du glycolaldéhyde obtenu à l'issue de l'étape b), en acide glycolique.

Dans de tels modes de réalisation, les métabolites d'intérêts obtenus à l'issu de la voie synthétique d'assimilation des pentoses selon l'invention sont l'éthylène glycol et/ou l'acide glycolique, et leurs dérivés.

### Enzymes de l'invention :

La voie synthétique d'assimilation des pentoses, représentée sur la figure 1, est catalysée par un ensemble d'enzymes.

L'enzyme recombinante catalysant l'étape de phosphorylation a) de la voie synthétique d'assimilation des pentoses de l'invention, est une kinase phosphorylant le (D)-Xylulose ou le (L)-Arabinose en position 1.

Une telle enzyme est par exemple choisie parmi le groupe constitué par :
- la kétohexokinase, de préférence l'isoforme C de la kétohexokinase (KhK-C),
- la rhamnulose kinase (RhaB) et
- la fuculose kinase (FucK).

La kétohexokinase C est codée par le gène *khk,* typiquement retrouvé chez *H. sapiens.*

Dans un mode de réalisation préféré, on utilise le gène *H. sapiens* codant pour la *Khk-C,*
de séquence SEQ ID N°1.

La rhamnulose kinase ainsi que la fuculose kinase de l'invention sont codées respectivement par les gènes, *rhaB* et *fucK*, typiquement retrouvés chez *E. coli.* Ainsi, dans certains modes de réalisation on utilise le gène *E. coli rhaB* codant pour la rhamnulose kinase B (RhaB) de séquence SEQ ID N°6 ou le gène *E*. *coli fucK* codant pour la fuculose kinase (FucK) de séquence SEQ ID N°5.

L'enzyme catalysant l'étape de clivage b) est une aldolase clivant le (D)-xylulose-1P ou le (L)-ribulose-1P en glycolaldéhyde et DHAP.

Une aldolase selon l'invention peut être choisie parmi l'aldolase B, codée par le gène *aldoB*, typiquement retrouvé chez *Homo sapiens* et la fructose-1,6 bisphosphate aldolase B de E. coli, codée par le gène *fbaB* typiquement retrouvé chez *E. coli.*

Ainsi, dans certains modes de réalisation particuliers, on utilise le gène *H. sapiens aldoB* codant pour l'aldolase B, de séquence SEQ ID N°2 ou le gène *E. coli fbaB* codant pour la fructose-1,6 bisphosphate aldolase B, de séquence SEQ ID N°9.

L'enzyme catalysant l'étape de réduction c) est une glycolaldéhyde réductase.

Une glycolaldéhyde (ou aldéhyde) réductase convenant à l'invention peut par être exemple choisie parmi l'aldéhyde réductase codée par :
- le gène *yqhD*, typiquement retrouvé chez *E. coli,* codant pour l'aldéhyde réductase YqhD de séquence SEQ ID N°4,
- la glycérol déshydrogénase codée par le gène *gldA,* typiquement retrouvé chez *E. coli,* codant pour la glycérol déshydrogénase GldA et de séquence SEQ ID N°51° et
- la L-1,2-propanediol oxidoréductase codée par le gène *FucO,* typiquement retrouvé chez *E. coli,* codant pour la L-1,2-propanediol oxidoréductase FucO et de séquence SEQ ID N°52.

L'enzyme catalysant l'étape d'oxydation c') est une glycolaldéhyde déshydrogénase.

Une glycolaldéhyde déshydrogénase convenant à l'invention est par exemple la glycolaldehyde déshydrogénase codée par le gène *aldA*, typiquement retrouvé chez *E. coli,* codant pour la lactaldéhyde déshydrogénase AldA, de séquence SEQ ID N°3).

Les enzymes catalysent respectivement les étapes de réduction et d'oxydation avantageusement en présence de la forme réduite ou oxydée, respectivement, de nicotinamide adénine dinucléotide (phosphate) (NAD(P)H), coenzyme d'oxydoréduction.

Dans certains modes de réalisation, le microorganisme utilisé dans la présente invention exprime avantageusement, de façon native ou recombinante, au moins une des enzymes suivantes :
- une xylose isomérase, convertissant le (D)-Xylose en (D)-Xylulose, comme par exemple l'enzyme codée par le gène *xylA* de *E. coli*,
- une xylose réductase et une xylitol déshydrogénase, comme par exemple les enzymes codées par les gènes XYL1 et XYL2 de la levure *Scheffersomyces stipitis.*
- une L-Arabinose isomérase, convertissant le (L)-Arabinose en (L)-Ribulose, comme
   par exemple l'enzyme codée par le gène *E. coli araA,* et/ou
- une arabinose réductase et une arabitol déshydrogénase.

De préférence encore, le microorganisme de l'invention exprime, de façon native ou recombinante, au moins une protéine transportant les pentoses à l'intérieur de la cellule, et notamment :
- des protéines transportant le (L)-Arabinose, comme par exemple les enzymes codées par les gènes *araE, araF, araG ou araH* de *E. coli*; et/ou
- des protéines transportant le (D)-xylose, comme par exemple les protéines codées par les gènes *xylE, xylF, xylG ou xylH* de *E. coli* ou encore le gène *galP* codant pour une perméase des sucres, ou le gène *gal-2a* de *S cerevisiae*

### Microorganisme recombinant :

Par « microorganisme », on entend selon l'invention une cellule hôte choisie parmi les cellules procaryotes notamment les archéobactéries, les bactéries ou les microalgues procaryotes, et les cellules eucaryotes, notamment les champignons, les levures et les cellules végétales et les microalgues eucaryotes.

Par « microorganisme recombinant », ou « microorganisme génétiquement modifié », ou « microorganisme modifié », on entend selon l'invention, une cellule hôte ayant subi une modification de son génome, par exemple par addition d'un acide nucléique exogène (ou recombinant), ou par modification d'un acide nucléique endogène.

Des bactéries adaptées à l'invention peuvent être par exemple choisies parmi les familles des *Enterobacteriaceae, Clostridiaceae, Bacillaceae, Streptomycetaceae, Streptococcaceae, Methylobacteriacae,* et *Corynebacteriaceae.*

Des bactéries particulièrement adaptées à l'invention peuvent typiquement être choisies parmi le groupe constitué par *Escherichia coli, Bacillus subtilis, Corynebacterium glutamicum, Clostridium acetobutylicum, Methylobacterium extorquens,* et *Lactococcus lactis.*

Des levures adaptées à l'invention peuvent être par exemple choisies parmi les familles des *Saccharomycetaceae, Pichiaceae,* et *Schizosaccharomycetaceae.*

Des levures particulièrement adaptées à l'invention peuvent typiquement être choisies dans le groupe constitué par *Saccharomyces cerevisiae, Schizosaccharomyces pombe, Kluyveromyces lactis, Kluyveromyces marxianus, Pichia jadinii, Scheffersomyces stipitis,* et *Pichia pastoris.*

Des genres de champignons adaptés à l'invention peuvent typiquement être choisis dans le groupe constitué par *Penicillium, Aspergillus, Chrysosporium* et *Trichoderma.*

Dans un mode de réalisation préféré de l'invention, on utilise *Escherichia coli, Scheffersomyces stipitis* ou *Saccharomyces cerevisiae,* en tant que microorganisme hôte.

Avantageusement, on utilise un microorganisme naturellement capable d'assimiler le (D)-xylose et/ou le (L)-arabinose.

De préférence, la voie synthétique d'assimilation des pentoses selon l'invention implique qu'au moins une enzyme catalysant l'une des étapes de phosphorylation a), de clivage b), de réduction c) ou d'oxydation c') est exprimée de façon recombinante par le microorganisme.

Typiquement, au moins l'enzyme catalysant l'étape de phosphorylation a), et/ou au moins l'enzyme catalysant l'étape de clivage b), du procédé de l'invention est exprimée de façon recombinante.

Dans un mode de réalisation particulier les enzymes catalysant les étapes de phosphorylation a), de clivage b), et de réduction c) et/ou d'oxydation c') sont des enzymes recombinantes.

Dans certains modes de réalisation, au moins une desdites enzymes recombinantes est une enzyme codée par un gène hétérologue (c'est-à-dire non exprimé naturellement dans l'organisme hôte de référence), en particulier au moins une des enzymes catalysant les étapes a) et b) est codée par un gène hétérologue.

De préférence, le microorganisme exprime au moins la KhkC.

De préférence encore, le microorganisme exprime de façon recombinante :
- la KhkC (notamment KhkC, *H. sapiens* codée par le gène *khkC* de séquence SEQ ID N°1) catalysant l'étape a) de la voie d'assimilation des pentoses, et
- l'aldolase B, codée par le gène *aldo-B* (notamment par le gène *aldoB* de séquence SEQ ID N°2) ou la fructose-1,6, bis phosphate aldolase, codée par le gène *fbaB* (notamment le gène *fbaB* de séquence SEQ ID N°9).

Dans un mode de réalisation particulier, la glycolaldéhyde réductase et/ou la glycolaldéhyde déshydrogénase catalysant respectivement les étapes c) et c') de la voie synthétique d'assimilation des pentoses de l'invention sont des enzymes endogènes, naturellement exprimées par le microorganisme.

Dans certains modes de réalisation, les enzymes endogènes de la voie synthétique d'assimilation de l'invention peuvent être surexprimées, notamment les enzymes codant les étapes de réduction c) ou d'oxydation c'). En particulier, la glycolaldéhyde déshydrogénase peut être surexprimée pour stimuler l'étape c') d'oxydation. Par exemple chez *E. coli* on peut surexprimer le gène *aldA* codant pour une glycolaldéhyde déshydrogénase ;

Dans certains modes de réalisation de l'invention, les enzymes convertissant les (D)-xylose ou le (L)-Arabinose en (D)-xylulose ou en (L)-ribulose, respectivement, à savoir des isomérases ou épimérases telles que décrites précédemment, ou encore les protéines important le (D)-Xylose ou le (L)-Arabinose dans la cellule, sont surexprimées dans le microorganisme.

Dans un mode de réalisation de l'invention, les acides nucléiques codant pour les enzymes catalysant les étapes a) et b) sont clonés en opéron dans un vecteur d'expression sous le contrôle d'un même promoteur. Dans certains modes de réalisation les acides nucléiques codant pour les enzymes catalysant les étapes a), b), et c) et/ou c') sont clonées en opéron.

L'expression des protéines recombinantes est contrôlée par un promoteur inductible ou de préférence constitutif.

### Optimisation de la voie synthétique d'assimilation des pentoses :

Dans certains modes de réalisation, l'activité d'une ou plusieurs enzymes endogènes de la cellule hôte peut également être modifiée de façon à optimiser la production d'éthylène glycol et/ou d'acide glycolique.

Certaines modifications susceptibles d'être apportées à un microorganisme de l'invention sont décrites ci-dessous.
**A)** De préférence, le microorganisme utilisé est génétiquement modifié de façon à atténuer ou supprimer l'activité des enzymes endogènes impliquées dans les voies naturelles d'assimilation des pentoses phosphates, et notamment le ou les enzyme(s) catalysant la phosphorylation en position 5 du cycle des pentoses, et plus particulièrement la (L)-ribulose-5-kinase et/ou la (D)-xylulose-5- kinase.
   A titre d'exemple, les gènes *araB* et/ou *xylB,* codant pour la ribulose-5-kinase et la xylulose-5-kinase respectivement, typiquement retrouvés chez *E. coli,* peuvent être atténués, ou de préférence inactivés.
   Une telle modification permet d'orienter le flux carbone préférentiellement vers la voie synthétique d'assimilation des pentoses de l'invention et d'optimiser la production d'éthylène glycol et/ou d'acide glycolique par ladite voie synthétique.
   Dans un mode de réalisation de l'invention, on utilise un microorganisme dans lequel le gène *xylB,* et notamment le gène *xylB* codant pour la xylulose kinase de séquence SEQ ID N°53, est délété.
**B)** L'activité des enzymes de type glycolaldéhyde réductase et/ou glycolaldéhyde déhydrogénase peut également être modifiée afin d'orienter la voie synthétique d'assimilation de l'invention vers la production d'acide glycolique ou d'éthylène glycol.

A titre d'exemple, les enzymes codées par les gènes *aldA,* codant pour une glycolaldéhyde déshydrogénase, ainsi que les gènes *gldA, fucO* et/ou *yqhD* codant pour une glycolaldéhyde réductase, peuvent notamment être surexprimés pour favoriser la production de l'éthylène glycol ou atténués ou inactivés, pour favoriser la production de l'acide glycolique.

Avantageusement, la production d'éthylène glycol est optimisée en utilisant un microorganisme dans lequel au moins l'une (et de préférence les deux) des modifications suivantes sont en outre apportées quant à l'expression des enzymes endogènes :
- surexpression du gène codant pour au moins une glycolaldéhyde réductase, de préférence la glycolaldéhyde réductase principale, exprimée par le microorganisme ;
- inactivation ou délétion du gène codant pour une glycolaldéhyde déshydrogénase catalysant l'étape c', par exemple le gène *aldA.*

Avantageusement, la production d'acide glycolique peut être optimisée en utilisant un microorganisme dans lequel au moins une des modifications suivantes (et de préférence au moins les deux premières) sont apportées, quant à l'expression des enzymes endogènes :
- surexpression du gène codant pour la glycolaldéhyde déshydrogénase, par exemple la glycolaldéhyde déshydrogénase codée par le gène *aldA* ;
- réduction de la dégradation de l'acide glycolique, notamment par atténuation ou inactivation de la glycolate oxydase, par exemple par inactivation d'au moins un des gènes *glcDEFG,* codant pour au moins l'une des sous-unités de la glycolate oxydase ;
- éventuellement une inactivation du ou des gènes codant pour une glycoladéhyde réductase.

De préférence, un microorganisme selon l'invention comprend, outre les modifications propres à l'expression de la voie synthétiques d'assimilation des pentoses de l'invention, et notamment propres à la catalyse des étapes de phosphorylation a) et de clivage b) de cette voie, au moins une des modifications supplémentaires décrites aux points A) ou B) ci-dessus.

De préférence encore, le microorganisme comprend au moins les modifications décrites au point A). Selon les modes de réalisation, cette modification peut être combinée avec les modifications du point B) pour optimiser la production d'éthylène glycol ou d'acide glycolique.

Les modifications mentionnées ci-dessus peuvent être combinées.

En particulier :
- pour optimiser la production d'éthylène glycol, l'inactivation des enzymes catalysant la phosphorylation en position 5 des pentoses, notamment la (L)-ribulose-5-kinase et/ou la (D)-xylulose-5-kinase (comme les gènes *xylB* ou *araB*) peut être combinée à l'inactivation de la voie de synthèse de l'acide glycolique (notamment par inactivation du ou des gènes codant pour une glycolaldéhyde déshydrogénase, par exemple le gène *aldA*). Ces inactivations peuvent être combinées à une surexpression du gène codant pour la glycolaldéhyde réductase catalysant l'étape c) du procédé de l'invention.
- Pour optimiser la production d'acide glycolique, l'inactivation des enzymes catalysant la phosphorylation en position 5 des pentoses, notamment la (L)-ribulose-5-kinase et/ou la (D)-xylulose-5-kinase (notamment codées par les gènes *xylB* ou *araB*) peut être combinée à l'inactivation de la glycolate oxydase par inactivation d'au moins un des gènes *glcDEFG* codant pour ses sous-unités et/ou à la surexpression de la glycolaldéhyde déshydrogénase codée par le gène *aldA.* Eventuellement, le gène codant pour la glycolaldéhyde réductase catalysant l'étape c) du procédé de l'invention est également inactivé.

Par exemple, un microorganisme modifié pour optimiser la production d'éthylène glycol exprime au moins les activités de phosphorylation de clivage et de réduction correspondant aux étapes a), b) et c) décrites précédemment, de préférence les enzymes KhkC, aldolase B (notamment codée par le gène *aldoB*) ainsi qu'une glycoladéhyde réductase (comme l'aldéhyde réductase YqhD ou la glycérol déshydrogénase GldA ou encore l'enzyme codée par le gène *fucO*) et comprend en les modifications suivantes :
- la délétion du gène codant pour l'aldéhyde déshydrogénase, notamment du gène *aldA ;*
- la délétion des gène codant pour le ou les enzyme(s) catalysant la phosphorylation en position 5 du (D)-xylulose et/ou du (L)-ribulose, et plus particulièrement la (D)-xylulose-5-kinase et/ou la (L)-ribulose-5-kinase, notamment les gènes *araB* et/ou *xylB.*

**C)** Il est également possible d'utiliser un microorganisme génétiquement modifié afin de favoriser, *en sus* de la voie synthétique de l'invention, la production d'acide glycolique par les voies naturelles.

En effet, les inventeurs ont en outre découvert que la production d'acide glycolique peut également être encore augmentée en combinant le fonctionnement de la voie synthétique d'assimilation selon l'invention avec des modifications génétiques qui conduisent en parallèle à la production d'acide glycolique par la voie dite du glyoxylate.

Un microorganisme selon l'invention peut donc par exemple présenter des modifications favorisant la production d'acide glycolique à partir du glyoxylate, comme décrit dans la demande WO2010/108909.

Ainsi la dihydroxyacétone phosphate (DHAP ou glycérone phosphate), notamment obtenue à l'issue de l'étape de clivage b) pourrait intégrer les voies naturelles de la glycolyse, du cycle des acides tricarboxyliques (CAT) et de la voie du glyoxylate, un shunt du CAT (voir pour revue Neidhardt, F. C. (Ed. in Chief), R. Curtiss III, J. L. Ingraham, E. C. C. Lin, K. B. Low, B. Magasanik, W. S. Reznikoff, M. Riley, M. Schaechter, and H. E. Umbarger (eds). 1996. *Escherichia coli and Salmonella: Cellular and Molecular Biology. American Society for Microbiology*)*.*

L'optimisation de la voie du glyoxylate peut donc être obtenue par au moins l'une, et de préférence par une combinaison, des modifications suivantes :
i) surexpression d'un gène codant pour une glyoxylate réductase ;
ii) surexpression d'un gène codant pour une isocitrate lyase, éventuellement accompagnée de la délétion du gène codant pour son répresseur transcriptionnel ;
iii) délétion des gènes codant pour des malate synthases ;
iv) délétion des gènes codant pour des glyoxylate carboligases ;
v) délétion des gènes codant pour des gènes codant pour des glycolates oxidases ou des glycolates déshydrogénases
vi) délétion des gènes codant pour des 2-kéto-4-hydroxyglutarate aldolases, notamment la Entner-Doudouroff Aldolase et/ou pour des phosphogluconate déhydratases;
vii) délétion d'un gène répresseur de la réponse aérobie (c'est-à-dire un gène codant pour un répresseur des gènes impliqués dans le métabolisme respiratoire), notamment le gène *arcA* ;
viii) atténuation et notamment délétion de l'expression de l'isocitrate déhydrogénase ;
ix) éventuellement délétion des gènes codants pour des systèmes d'internalisation de l'acide glycolique ; et
x) éventuellement, atténuation des voies métaboliques qui amènent à la production de produit secondaires comme l'acétate, le lactate ou l'éthanol.

Dans le cas où le microorganisme recombinant, pour la production de l'acide glycolique, est *Escherichia coli,* les modifications décrites ci-dessus correspondent à
i) la surexpression du gène *ghrA,* et/ou *du gène ycdW ;*
ii) la surexpression du gène *aceA* accompagné ou non par la délétion de son répresseur transcriptionel, *iclR ;*
iii) la délétion des gènes *aceB* et *glcB*;
iv) la délétion du gène *gcl* ;
v) la délétion d'au moins un gène choisi parmi *glcD, glcE, glcF* ou *glcG*
vi) la délétion des gènes *edd-eda* ;
vii) la délétion du gène *arcA ;*
viii) l'atténuation et de préférence la délétion de l'expression du gène *icd* ;
ix) la délétion des gènes *glcA, lldP*, et/ou *yjcG ;*
x) la délétion des gènes *ackA-pta, poxB, ldha*, et/ou *adhE.* ;

Avantageusement, les modifications suivantes sont apportées pour optimiser la production d'acide glycolique dans un microorganisme selon la présente invention, par exemple chez *E.coli* :
i) la surexpression du gène *ghrA*
ii) la surexpression des gènes *aceA* éventuellement accompagné de la délétion du gène *iclR*;
iii) la délétion des gènes *aceB* et *glcB*
iv) la délétion du gène *glc* ;
v) la délétion d'au moins un gène choisi parmi *glcD, glcE, glcF* ou *glcG ;*
vi) la délétion des gènes *edd-eda* ;
vii) la délétion du gène *arcA*
viii) la délétion du gène *icd*

Dans un mode de réalisation, le microorganisme porteur des modifications ci-dessus n'exprime pas d'enzyme catalysant la phosphorylation en position 5 du (L)-ribulose et/ou du (D)-xylulose, et plus particulièrement la (L)-ribulose-5-kinase et/ou la (D)-xylulose-5- kinase. Ainsi, dans certains modes de réalisation on utilise un microorganisme dans lequel les gènse *xylB* et/ou *araB* sont délétés.

En variante, le microorganisme (avantageusement *E*. *coli*) exprime un ou plusieurs enzymes catalysant la phosphorylation en position 5 du (L)-ribulose et/ou du (D)-xylulose, et plus particulièrement la (L)-ribulose-5-kinase et/ou la (D)-xylulose-5- kinase. Typiquement, le microorganisme exprime le gène *xylB* et/ou le gène *araB.* Un tel microorganisme offre un excellent rendement en acide glycolique, lorsqu'il est cultivé sur un milieu contenant du glucose, notamment contenant au moins du glucose et du xylose ou du xylulose. De préférence le milieu contient majoritairement du glucose.

**D)** Il est enfin possible, pour augmenter la production en éthylène glycol ou en acide glycolique, d'utiliser un microorganisme dans lequel au moins un gène codant pour un transporteur des sucres (par exemple le gène *galP* codant pour une perméase des sucres et/ou le gène *gal-2a de S cerevisiae*) est surexprimé. Avantageusement un tel gène est exprimé de façon constitutive.

Dans un mode de réalisation particulièrement préféré de l'invention, on utilise pour la production d'acide glycolique un microorganisme combinant les modifications décrites aux paragraphes A) à D) ci-dessus. En particulier on utilise un microorganisme combinant les modifications rapportées dans les paragraphes, B), C) et D) ci-dessus pour la production d'acide glycolique.

Avantageusement un tel microorganisme exprime la KhkC et l'aldolase B et comporte les modifications suivantes :
- la surexpression du gène codant pour la glycolaldéhyde déshydrogénase, par exemple la glycolaldéhyde déshydrogénase codée par le gène *aldA ;*
- la surexpression du gène *ghrA*
- la surexpression du gène *aceA* éventuellement accompagné de la délétion du gène *iclR*;
- la délétion des gènes *aceB* et *glcB*
- la délétion du gène *glc*
- la délétion d'au moins un gène choisi parmi *glcD, glcE, glcF, ou glcG ;*
- la délétion des gènes *edd-eda* ;
- la délétion du gène *arcA* ;
- la délétion du gène *icd* ;
- la surexpression du gène *galP*

En fonction du substrat de culture choisi, un tel microorganisme peut également porter ou non une délétion du gène *xylB* et/ou du gène *araB.* De préférence l'expression du gène *xylB* et/ou du gène *araB* est conservée lorsque le microorganisme est cultivé sur un substrat comprenant du glucose.

De même en fonction du substrat de culture, on utilise un microorganisme exprimant, de façon recombinante ou non, des enzymes convertissant le (D)-xylose ou le (L)-Arabinose en (D)-xylulose ou en (L)-ribulose, respectivement, à savoir des (D)-xylulose isomérases ou des (L-arabinose isomérases, ou des (D)-xylose réductases/(D)-xylitol déshydrogénases, ou des (L)-arabinose réductases/(L)-arabitol déshydrogénases, telles que décrites précédemment.

De façon générale, sans être limitatif, le rendement théorique du procédé de l'invention pour l'éthylène glycol est d'environ 1 mol d'éthylène glycol par mol de xylose ou d'arabinose.

Le rendement théorique du procédé de l'invention pour l'acide glycolique est d'environ 1 mol d'acide glycolique par mol de xylose ou arabinose, sans activation du cycle glyoxylate. Lors d'un fonctionnement parallèle de la voie synthétique et du cycle glyoxylate, le rendement théorique est d'environ 2 mol d'acide glycolique par mol de xylose ou arabinose.

### Culture du microorganisme :

Les conditions de culture du microorganisme selon l'invention peuvent être adaptées selon les techniques classiques, connues de l'homme du métier.

Typiquement, les bactéries utilisées comme cellules hôtes dans la présente invention peuvent être cultivées dans des milieux de tous types et de toute composition.

Les milieux de culture sont typiquement des milieux carbonés comprenant, ou supplémentés, avec divers composés incluant notamment différentes sources de carbone, et notamment de pentoses, comme le (D)-glucose, le (D)-Xylose, le (L)-Arabinose, et/ou des hydrolysats de biomasse lignocellulosique, notamment de l'hémicellulose, de l'amidon et leurs dérivés.

Dans certains modes de réalisation, le milieu de culture comprend moins de 5 %, notamment moins de 4 %, moins de 3 %, moins de 2 % ou encore moins de 1 % de rhamnulose.

Par « hydrolysat de biomasse », on entend en particulier des hydrolysats lignocellulosiques, en particulier des hydrolysats comprenant au moins 20 % de de xylose et/ou au moins 5%, notamment au moins 10 % d'arabinose, en poids par rapport au poids total de l'hydrolysat. Dans un mode de réalisation préféré, on utilise ainsi des hydrolysats lignocellulosiques de bois durs, d'épis de maïs ou de papier.

D'autres paramètres relatifs aux conditions de culture peuvent être optimisés par des expériences de routine, comme le pH ou la température.

Dans certains modes de réalisation, la température de culture varie de 25 à 43 °C et dépend essentiellement du type de cellule hôte et du milieu de culture. A titre d'exemple lorsque la cellule hôte est *E. coli,* la température de culture optimale varie généralement entre 30 et 38 °C.

La durée de culture dépend également des paramètres de culture mentionnés ci-dessus. Typiquement, les cellules peuvent être cultivées entre 6 et 300 heures.

De préférence, le ou les métabolites d'intérêt obtenus à l'issu de l'opération de culture du microorganisme selon l'invention sont récupérés dans le milieu de culture.

La présente demande se rapporte également à un microorganisme recombinant tel que décrit dans la présente demande.

En particulier, la présente invention se rapporte à un microorganisme exprimant une voie synthétique d'assimilation des pentoses selon l'invention.

Selon différents modes de réalisation, les voies naturelles d'assimilation des pentoses sont conservées ou sont inactivées (par exemple par délétion des gènes codant pour la xylulose-5-kinase et/ou la ribulose-5-kinase).

Un microorganisme selon l'invention exprime au moins :
- un acide nucléique codant pour une enzyme apte à phosphoryler, en position 1, un pentose choisi parmi le (D)-Xylulose et/ou le (L)-Ribulose, et
- un acide nucléique codant pour une enzyme de type aldolase, apte à cliver le (D)-xylulose-1-phosphate et/ou le (L)-Ribulose-1-phosphate en glycolaldéhyde et DHAP, tels que décrit précédemment

De préférence, au moins une de ces enzymes est exprimée de façon recombinante.

Dans certains modes de réalisations, au moins une de ces enzymes est codée par un acide nucléique exogène et, de préférence, le microorganisme exprime au moins :
- un acide nucléique codant pour l'isoforme C de la kétohexokinase (typiquement retrouvée chez *H. sapiens*) et
- au moins un acide nucléique codant pour l'aldolase B.

Dans certains modes de réalisation, le microorganisme est en outre modifié tel que décrit dans les points A) à E) ci-dessus.

Par exemple un microorganisme adapté à la production d'acide glycolique peut avantageusement comprendre les modifications suivantes :
- la surexpression du gène codant pour la glycolaldéhyde déshydrogénase, par exemple la glycolaldéhyde déshydrogénase codée par le gène *aldA ;*
- la surexpression du gène *ghrA*
- la surexpression du gène *aceA* ;
- éventuellement la délétion du gène *iclR* ;
- la délétion du gène *glc* ;
- la délétion d'au moins un gène choisi parmi les gènes *glcD, glcE, glcF ou glcG* ;
- la délétion des gènes *aceB* et *glcB*
- la délétion des gènes *edd-eda* ;
- la délétion du gène *ArcA* ;
- la délétion du gène *icd* ;
- éventuellement la délétion des gènes *xylB et*/*ou araB.*

### EXEMPLES

### Composition des milieux

### Milieu Luria-Bertani (LB)

Pour un litre de milieu: 10 g de tryptone, 5 g d'extrait de levures, 5 g de chlorure de sodium dans un litre d'eau purifiée. Le milieu est autoclavé avant utilisation. Pour être utilisé en milieu solide, 2 % d'agar est rajouté au milieu avant autoclavage.

### Milieu minimal M9

Pour un litre de milieu : 18 g Na₂HPO₄*12 H₂O; 3 g KH₂PO₄; 0,5 g NaCl; 2 g NH₄Cl; 0,5 g MgSO₄ * 7 H₂O; 0,015 g CaCl₂ * 2 H₂O; 1 ml d'une solution d'éléments traces (contenant par litre 0,04 g NaEDTA * 2 H₂O, 0,18 g CoCl₂ * 6 H₂O; ZnCl₂SO₄ * 7 H₂O; 0,04 g Na₂MoO₄ * 2 H₂O, 0.01 g H₃BO₃, 0,12 g MnSO₄ * H₂O, 0,12 g CuCl₂ * H₂O) ; des quantités de (D)-glucose, (D)-xylose, et (L)-arabinose précisées dans le texte. Le milieu est ajusté à un pH de 7 et filtré.

### YPD

Le milieu YPD est utilisé comme milieu riche pour la croissance de *S. cerevisiae.* Pour un litre, 10 g d'extraits de levure, 20 g de bacto-peptone. Le milieu est autoclavé avant utilisation et 20 g de glucose filtré sont ajoutés. Pour être utilisé en milieu solide, 2 % d'agar est rajouté au milieu avant autoclavage.

### Milieu minimal SCD pour Saccharomyces cerevisiae

Pour un litre, 1,7 g de yeast nitrogen base sans acides aminés, 5 g de sulfate d'ammonium sans acides aminés, drop-out de 0,940 g des acides aminés essentiels exceptés de ceux utilisés pour mettre en évidence une auxotrophie, 900 ml d'eau puis le tout est autoclavé. En cas d'utilisation en milieu solide, 20 g de bacto-agar est rajouté. 100 ml d'une solution de sucres à 20% est rajoutée.

### Test de croissance en milieu M9 + xylose

Toutes les cultures ont été réalisées dans des flacons d'Erlenmeyer de 250 ml contenant 50 ml de milieu de culture et en agissant les cultures à 200 RPM.
Les cellules à tester sont mises en culture sur la nuit à 37 °C en milieu LB. Cette préculture est ensuite utilisée pour inoculer à DO₆₀₀ₙₘ∼0,2 du milieu M9 + 10 g/l glucose. En phase exponentielle de croissance (DO entre 0,6 et 1) l'IPTG est ajouté à 1 mM et les cultures sont ainsi incubées pendent 16 à 18 heures. Après cette période d'incubation les cellules sont lavées deux fois à l'eau stérile et réensemencées à DO₆₀₀ₙₘ∼0,2 dans du milieu M9 + IPTG 1 mM + glucose et/ou xylose et/ou arabinose en des quantités indiquées dans le texte. La DO₆₀₀ₙₘ est suivie et des aliquots sont prélevés, centrifugés et injectés en HPLC pour analyse des métabolites.

### Méthodes de construction de souches

### Transformation bactérienne

Les transformations bactériennes sont réalisées sur des cellules chimiocompétentes commerciales ou préparées au laboratoire. Les cellules rendues chimiocompétentes sont préparées selon le protocole au chlorure de calcium (Dagert and Ehrlich, 1979). La transformation s'effectue ensuite en laissant pendent 20 min l'ADN plasmidique à transformer au contact des bactéries compétentes sur de la glace, puis un choc thermique à 42 °C de 45 secondes est réalisé. Les cellules sont remises 5 minutes sur glace puis 1ml de milieu LB est rajouté avant de les incuber pendent 1 h à 37 °C. Les cellules sont ensuite étalées sur boite LB solide supplémentée avec le marqueur de sélection correspondant.

De manière générale, outre les plasmides développés dans le cadre de la présente invention, les plasmides suivants ont été utilisés : pACT3 (Dykxhoorn et al., 1996), pEXT20 (Dykxhoorn et al., 1997), pGEM-T (Promega), pET28a (Novagen), pCP20 (Cherepanov & Wackernagel, 1995), peX-A-aldoB (Eurofins) et pET11-KHK-C (Asipu et al., 2003).

### Délétion de gène par transduction d'une cassette kanamycine à partir d'une souche KEIO

Pour transférer une délétion de gène portée par une souche KEIO *d'E. coli* vers une souche réceptrice donnée de MG1655 d'*E*. *coli*, une transduction est réalisée.

A partir d'une souche Keio mise en culture en LB + 50 µM kanamycine à 37°C sur la nuit, un lysat de phage est généré. Sur une pré-culture de 10 ml de LB inoculée le matin à partir de 200 µl de la culture sur la nuit en présence de 2 g/L de glucose et 5 mM de CaCl₂, on ajoute 200 µl de phage P1. La culture est laissée 2 h le temps de la lyse cellulaire due au phage. La réaction est arrêtée avec 200 µl de chloroforme. Le tout est centrifugé 10 min à 4500 x g et 9 ml du surnageant contenant les phages récupéré et stocké avec 200 µl de chloroforme à 4 °C. La souche réceptrice est mise en préculture sur la nuit. De cette culture, 1,5 ml est récupéré et centrifugé. Le culot est repris dans 600 µl de MgSO₄ 10 mM + CaCl₂ 5 mM. La transduction est réalisée en mettant 100 µl de cellules en présence de 100 µl du lysat de phage. Le tout est incubé 30 minutes à 30 °C sans agitation. Ensuite, 100 µl de citrate de sodium 1 M est ajouté ainsi que 1ml de LB. L'expression phénotypique des souches ayant intégré la cassette kanamycine se fait en laissant les cellules pousser pendant 1 h à 37 °C sous agitation. Les cellules sont ensuite étalées sur boite de milieu LB contenant le marqueur de sélection et laissées à pousser sur la nuit. Le lendemain, les colonies formées sont testées par PCR pour la présence de la cassette de sélection et pour l'absence du gène délété.

### Protocole d'excision de cassette de sélection flanquée de séquence FRT

La cassette est excisée du chromosome en utilisant la recombinase FLP portée par le plasmide pCP20 (Cherepanov & Wackernagel, 1995) qui laisse une région cicatricielle contenant un site FRT. pCP20 est un plasmide portant la résistance à l'ampicilline et au chloramphénicol présentant une réplication thermosensible et une expression de la recombinase FLP thermo-induite. Les mutants marqueurs résistants contenant donc la cassette sont transformés avec pCP20 et les transformants ampicilline résistants portant la résistance du plasmide sont sélectionnés à 30 °C. Ils sont ensuite mis en culture à 37 °C sur LB solide puis testé pour la perte de la résistance à l'ampiciline. L'excision de la cassette de sélection est ensuite vérifiée par PCR avec les primers ayant servi pour l'amplifier avec la Taq polymerase (NEB). Les délétions multiples sont obtenues en répétant l'opération.

### Clonage de gènes sur plasmide chez S. cerevisiae

Le clonage d'un gène chez *S. cerevisiae* utilise les capacités de recombinaison génétique de la levure. Le gène à cloner est associé à une séquence promotrice et une séquence terminatrice donnant trois fragments à liguer dans un plasmide préalablement linéarisé. Pour cela, des régions homologues de 40 nucléotides sont désignés sur les primers. Ces 40 nucléotides d'homologie permettent aux systèmes de recombinaison de la levure de liguer l'ensemble des fragments après transformation. Chaque fragment est amplifié par PCR en utilisant la PhusionTM polymérase. L'ensemble des fragments et le vecteur d'accueil linéarisé est transformé dans une souche compétente de *S. cerevisiae* selon la méthode décrite par Gietz, et Woods (2002). Après croissance des transformants, les plasmides sont extraits selon la méthode décrite par Zeugin et Hartley (1985). Les plasmides sont ensuite utilisés pour transformer une souche DH10B d'*E*. *coli.* Les plasmides sont extraits d'*E*. *coli*, vérifiés par séquençage et utilisés pour transformer la souche réceptrice de *S. cerevisiae.*

### Extraction de plasmides chez S. cerevisiae

Après transformation, les colonies obtenues sont resuspendues dans de l'eau puis centrifugées en vue d'une extraction de plasmide. Le culot de cellules est resuspendues dans 400 µl d'un tampon à 4°C contenant 50 mM glucose, 10 mM EDTA, 25 mM Tris-HCl pH 8 et supplémenté de la RnaseA 0,1mg/ml. 400 µl d'une solution contenant 0,2 M NaOH et 1% SDS est utilisé pour faire lyser les cellules. Des billes de verre sont ensuite rajoutées à hauteur d'un tiers du volume total et les cellules sont vortexées à 4°C pendant 10 minutes. Cette étape est suivie d'une centrifugation de 60 secondes à 13000 RPM. 700µl du surnageant sont prélevés et mis dans un nouveau tube de 2 ml. 325 µl d'une solution à 4°C de 3 M KAc à pH 5,5 est ajouté. Le mélange est laissé à incuber 10 min sur glace avant d'être centrifugé pendant 10 min à 13000 RPM à 4°C. 700 µl du surnageant est prélevé et déposé dans un nouveau tube. 700 µl d'isopropanol est rajouté et le tout est agité intensément avant d'être laissé à incuber 10 min à température ambiante. Une centrifugation est ensuite réalisée à température ambiante pendant 30 min à 13000 RPM. Le surnageant est ensuite éliminé et le culot resuspendu dans 500 µl d'éthanol à 70% à -20°C puis centrifugé 5 min. Le surnageant est éliminé. Cette étape est répétée une nouvelle fois puis le culot est séché jusqu'à la disparition de l'éthanol. Ce culot est ensuite repris dans 30 µl d'H₂O.

De façon générale, les amorces utilisées pour l'expression de la voie chez *E. coli* sont listées dans le tableau 3, et les amorces utilisées pour l'expression de la voie chez *S.cerevisiae* sont listées dans le tableau 4.

### Expression et purification de protéines depuis un plasmide pET28a

La souche d'*E.coli* BL21(DE3) contenant le pET28a portant le gène d'intérêt est mise en culture sur la nuit à 37 °C dans 100 ml de milieu LB dans un erlenmeyer de 500 ml sous agitation 200 RPM.

Le lendemain, 10 à 50 ml de cette pré-culture d'*E.coli* BL21(DE3) contenant le pET28a portant le gène d'intérêt sont mis en culture dans du milieu LB supplémenté par 50 µg/ml de kanamycine à 37 °C.

L'expression protéique est déclenchée par le rajout de 1 mM IPTG aux cultures qui atteignent une DO_{6OOnm}∼0,7. Après 3 h d'incubation à 37 °C les cellules sont centrifugées, et les culots obtenus congelés à -80 °C. Pour la purification de la protéine ainsi exprimées, les cellules sont reprises dans 1ml de tampon de lyse (50 mmol/l Hépès [pH 7,5], 3 mol/L NaCl, 0,25 mmol/l) et gardées une heure dans la glace.

Les cellules sont ensuite soniquées et les débris retirés par centrifugation pendant 10 minutes à 13000 RPM à 4°C. Une résine Talon™ est ensuite préparée avec 0,3 mL de résine dans 3 mL de tampon de lyse. Toute la suspension est ensuite déposée sur la résine et incubés 20 minutes à température ambiante avant d'être centrifugés 5 minutes à 2500 RPM à 4°C. Le culot est lavé avec 10 fois le volume de résine avec du tampon de lyse et incubé pendent 10 minutes à température ambiante. L'opération est répétée avec le tampon de lyse qui contient 15 mM d'imidazole. Puis le culot est lavé avec 500 µl d'imidazole à 200 mM. Le tout est re-centrifugé pendant 5 minutes à 2500 RPM à 4 °C. Lesurnageant est récupéré donnant l'élution 1. L'opération est renouvelée donnant une élution 2. Les protéines purifiées se retrouvent dans les différentes élutions qui seront testées.

### Méthodes analytiques

Pour doser les produits de la voie synthétique à partir des surnageants de cultures, une HPLC (Ultimate 3000, Dionex) équipée d'un autosampler et d'un four (Shimadzu CTO-20A) et couplés au détecteur RID-10A (Shimadzu) et UV SPD-20A (Shimadzu) a été utilisée. Les composés ont été séparés sur une colonne Aminex HPX-87H (300mm x 7,8 mm) équipée d'une pré-colonne (Aminex). Les analyses ont été réalisées à une température de 35 °C avec un débit de 0,5 ml/min d'H₂SO₄ à 1.25 mM. Des échantillons de 20 µl ont été injectés dans l'appareil.

### Exemple 1 : Démonstration des activités (D)-xylulose-1-kinase et (L)-ribulose-1-kinase.

### Clonage des xylulokinases candidates : khkC, rhaB et fucK dans pET28a

Le clonage des gènes *khkC* (SEQ ID N°1), *rhaB* (SEQ ID N°6) et *fuck* (SEQ ID N°5) dans pET28a a été réalisée comme indiqué ci-dessous. Le gène *khk-C* a été digéré à partir du pET11a -khk-C (Asipu *et al.,* 2003) par NdeI/EcoRI. Il a été inséré par ligation grâce à la ligase T4 (Biolabs) à la suite du tag-histidine du pET28a préalablement digéré par ces mêmes enzymes. Le clonage de *rhaB* et *fucK* a été réalisé en amplifiant par PCR *rhaB* et *fucK* à partir de l'ADN génomique d'*E. coli* avec les primers suivant, respectivement P1/P2; P3/P4 listés dans tableau 3. Les fragments ont ensuite été clonés dans le vecteur pGEM-T (Invitrogen). Ils ont ensuite été digérés par NcoI et BamHI puis ligué dans le plasmide pET28a au niveau de MCS préalablement digéré par ces mêmes enzymes. Le produit de ligation est transformé dans une souche BL21(DE3) d'*E. coli.* Les vecteurs pET28-khk-C, pET28-rhaB, et pET28-fucK ainsi obtenus ont été vérifiés par séquençage de contenir les gènes avec les séquences correctes.

### Détermination des paramètres cinétiques des (D)-xylulokinases et de (L)-ribulokinase candidates

Les protéines ont été exprimées et purifiées comme décrit précédemment et les paramètres cinétiques des enzymes ont été déterminés par la réaction de couplage pyruvate kinase/lactate déhydrogénase reposant sur le principe suivant (l'exemple donné correspond au dosage d'une activité (D)-xylulose-1P aldolase):

| | |
|---|---|
| Xylulose kinase : | (D)-Xylulose + ATP → (D)- xylulose-1P + ADP |
| Pyruvate kinase: | phosphoénolpyruvate + ADP → pyruvate + ATP |
| Lactate déhydrogénase: | pyruvate + NADH → lactate + NAD |

La réaction a été réalisée dans le mélange suivant : NADH 0, 4 mM (Sigma), PEP 2 mM (Sigma), ATP 4 mM (Sigma) dans un tampon Hépès (90 mM Hepes, 77 mM KCI, 12 mM MgCl₂, ajusté à pH 7 avec une solution de KOH). Un volume de 1,25 µl d'un mélange enzymes pyruvate kinase/lactate déhydrogénase (Sigma) est ajouté dans un mélange réactionnel total de 250 µl. La réaction démarre avec l'ajout de 100 µl de (D)-xylulose (Carbosynth) ou de (L)-ribulose (Sigma) 10 mM. La consommation du NADH a été suivie par spectrofluorimètre à 340 nm.

**Tableau 1A. Paramètres cinétiques des kinases sur leur substrat naturel et sur (D)-xylulose. Les substrats naturels de Khk-C, RhaB et FucK sont respectivement le fructose, le rhamnulose et le fuculose.**

| Enzyme candidate | Substrat naturel | | (D)-xylulose | |
|---|---|---|---|---|
| | **Vmax** [U/mg] | **Km** [mM] | **Vmax** [U/mg] | **Km** [mM] |
| **Ketohexokinase Khk-C, *H*. *sapiens*** | 6 | 0,72 | 3,1 | 0,6 |
| **(L)-rhamnulose kinase RhaB, *E. coli*** | nd | nd | 18,7 | ns |
| **L-fuculokinase FucK, *E. coli*** | 20 | 0,06 | 0,1 | ns |

Ces résultats montrent que des kinases ayant la capacité de phosphoryler le (D)-xylulose en position 1 peuvent être identifiées.

La même approche a été utilisée pour caractériser ces paramètres sur (L)-ribulose:

**Tableau 1B. Paramètres cinétiques des kinases sur son substrat naturel et sur (L)-ribulose.**

| Enzyme candidate | Substrat naturel | | (L)-ribulose | |
|---|---|---|---|---|
| | **Vmax** [U/mg] | **Km** [mM] | **Vmax** [U/mg] | **Km** [mM] |
| **Ketohexokinase, Khk-C, *H*. *sapiens*** | 6,59 ± 1,4 | 0,31 ± 0,1 | 3,5 | 0,55 |

La Khk-C est fonctionnelle à la fois sur (D)-xylose et sur (L)-ribulose et présente des caractéristiques appropriées pour son utilisation dans la voie synthétique.

### Exemple 2 : Démonstration des activités (D)-xylulose-1P-aldolase et (L)-ribulose-1P-aldolase.

### Clonage des gènes codant pour les aldolases candidates AldoB, FbaB et AgaY

Le clonage des aldolases candidates s'est fait en amplifiant par PCR *aldoB*, *fbaB* et *agaY* (en utilisant respectivement les paires de primers P5/P6, P7/P8 et P11/P12 listés dans le tableau 3). Cette amplification a été réalisée à partir du plasmide peX-A-aldoB portant *aldoB* avec optimisation de codons (Eurofins) ou de l'ADN génomique d'*E. coli* pour *fbaB* et *agaY* respectivement. Les fragments ont ensuite été clonés dans pGEM (Invitrogen). Ils ont ensuite été digérés par BamHI et HindIII (pour *aldoB*) ou Ndel et BamHI (pour *fbaB* et *agaY*) puis ligués dans le plasmide pET28a au niveau de MCS préalablement digéré par BamHI et HindIII ou Ndel et BamHI pour cloner respectivement *aldoB* (SEQ ID N°2) ou *fbaB* (SEQ ID N°9) et *agaY* (SEQ ID N°8). Le produit de ligation est transformé dans la souche BL21(DE3) d'E. *coli.* Les vecteurs pET28-aldoB, pET28-fbaB, et pET28-agaY ainsi obtenues ont été vérifiés par séquençage de contenir les gènes avec les séquences correctes.

### Détermination des paramètres cinétiques des (D)-xylulose-1-phosphate aldolases et (L)-ribulo-1-phosphate aldolases candidates

Les protéines ont été exprimées et purifiées comme décrit précédemment et les paramètres cinétiques des enzymes ont été déterminés sur (D)-fructose-1,6bP, (D)-xylulose-1P et (L)-ribulose-1P reposant sur le principe suivant (l'exemple donné correspond au dosage d'une activité (L)-ribulose-1P aldolase).

| | |
|---|---|
| (L)-ribulose-1 kinase : | (L)-ribulose + ATP -> (L)-ribulose-1P + ADP |
| (L)-ribulose-1P aldolase: | (L)-ribulose-1P → DHAP + glycolaldehyde |
| Glycerol-3P dehydrogenase: | DHAP + NADH → Glycerol-3P + NAD |

La réaction a été réalisée dans le mélange suivant : NADH 0, 4 mM, PEP 2 mM, ATP 4 mM (tous Sigma) dans un tampon Hépès (90 mM Hepes, 77 mM KCI, 6, 8 mM MgCl₂, ajusté à pH 7 avec une solution de KOH). Les enzymes Khk-C purifiées et GldA (Glycerol déhydrogénase de Cellulomonas sp. Sigma) sont ajoutées à hauteur de 15 µl de Khk-C (0,005 U) et 4 µl d'une solution concentrée à 84 U/ mg de GldA pour un mélange réactionnel total de 250 µl. La réaction a été démarrée avec l'ajout de 100 µl de D-xylulose ou du (L)-ribulose (Sigma) 20 mM. La consommation du NADH a été suivie par spectrofluorimètre à 340 nm.

**Tableau 2A. Paramètres cinétiques d'aldolases sur (D)-fructose-1,6 bisphosphate et sur (D)-xylulose-1 -phosphate.**

| | (D)-Fructose-1,6bP | | (D)-Xylulose-1P | |
|---|---|---|---|---|
| Enzyme candidate | **Vmax** [U/mg] | **Km** [mM] | **Vmax** [U/mg] | **Km** [mM] |
| **Fructose-16bP aldolase, Aldo-B, *H. sapiens*** | 0.46 ± 0,05 | 0,03 ± 0,01 | 0.81 ± 0,2 | nd |
| **Fructose-16bP aldolase, FbaB, *E*. *coli*** | 0.52 ± 0,1 | 0,33 ± 0,07 | 0.18 ± 0,04 | nd |

**Tableau 2B. Paramètres cinétiques d'aldolases sur (D)-fructose-1,6 bisphosphate et sur (L)-ribulose-1-phosphate.**

| | (D)-Fructose-1,6bP | | (L)-Ribulose-1P | |
|---|---|---|---|---|
| Enzyme candidate | **Vmax** [U/mg] | **Km** [mM] | **Vmax** [U/mg] | **Km** [mM] |
| **Fructose-16bP aldolase Aldo-B, *H. sapiens*** | 0.46 ± 0,05 | 0,03 ± 0,01 | 0,00 | nd |
| **Fructose-16bP aldolase FbaB, *E. coli*** | 0.52 ± 0,1 | 0,33 ± 0,07 | 0,06 ± 0,01 | nd |

AldoB possède une activité sur (D)-xylulose-1-P et peut donc être utilisé pour la voie synthétique d'assimilation du xylose. FbaB possède une activité sur (D)-xylulose-1P et (L)-ribulose-1P et peut donc être utilisée pour la construction d'une voie synthétique pour l'assimilation du (D)-xylose ou du (L)-arabinose.

### Exemple 3 : Fonctionnement in vitro de la voie métabolique synthétique d'assimilation des pentoses.

### Fonctionnement in vitro de la voie métabolique synthétique d'assimilation du (D)-xylose

La voie métabolique d'assimilation du D-xylose a été reconstituée *in vitro* à partir d'enzymes purifiées (commerciales et exprimées puis purifiées à partir d'*E. coli*) à partir du (D)-xylulose pour démontrer son fonctionnement par la production de l'éthylène glycol (Figure 1).

Les enzymes utilisées pour la mise en oeuvre de la voie métabolique synthétique ont été les suivantes :
- Khk-C (Kétohexokinase /*H.sapiens*), codée par le gène *khkC* de séquence SEQ ID N°1 ou KHK-A (Prospecbio) ;
- Aldolase B (AldoB /*H.sapiens*), codée par le gène *aldoB* de séquence SEQ ID N°2 ou aldolase de lapin (Sigma-Aldrich-A2714) ;
- Glycérol déshydrogénase *Cellulomonas sp.* (Sigma-aldrich/ G3512-250U).

Le milieu réactionnel comprenait le tampon Hepes (90 mM Hepes ; 77 mM KCL ; 6, 8 mM MgCl₂) à pH = 7 ; 4 mM ATP ; 0,4 mM NADH ; 0,005 Unité/ml Khk-A (Prospecbio) ou Khk-C (purifiée depuis pET28a); 1 Unité/ml aldolase (AldoB, Sigma A6338), et 1 Unité/ml GldA (Sigma- G3512-250U). La réaction a été démarrée par l'ajout de 5 mM (D)-xylulose (Cabosynth). Après un temps d'incubation de 3 h, l'éthylène glycol produit au cours de la réaction a été quantifié par HPLC (Figure 2).

L'apparition de l'éthylène glycol dans la réaction qui contient la (D)-xylulose-1-kinase, la (D)-xylulose-1P aldolase et la glycolaldéhyde réductase démontre le fonctionnement de la voie synthétique.

### Fonctionnement in vitro de la voie métabolique d'assimilation du (L)-arabinose

La voie métabolique d'assimilation du (L)-arabinose a été reconstituée in vitro à partir d'enzymes purifiées (commerciales et exprimées puis purifiées à partir de *E. coli*) à partir du (L)-ribulose.

### Le fonctionnement de la voie a été vérifié par la mesure en HPLC de l'éthylène glycol produit.

Les enzymes utilisées pour la mise en oeuvre de la voie métabolique synthétique ont été les suivantes :
- Khk-C (Kétohexokinase /*H.sapiens*), codée par le gène *khkC* de séquence SEQ ID N°1 ;
- FbaB (Fructose 1,6-bisphosphate aldolase, *E. coli*) codée par le gène *fbaB*, de séquence SEQ ID N°9 ;
- GldA (Glycérol déhydrogénase *Cellulomonas sp.* (Sigma-aldrich/ G3512-250U).

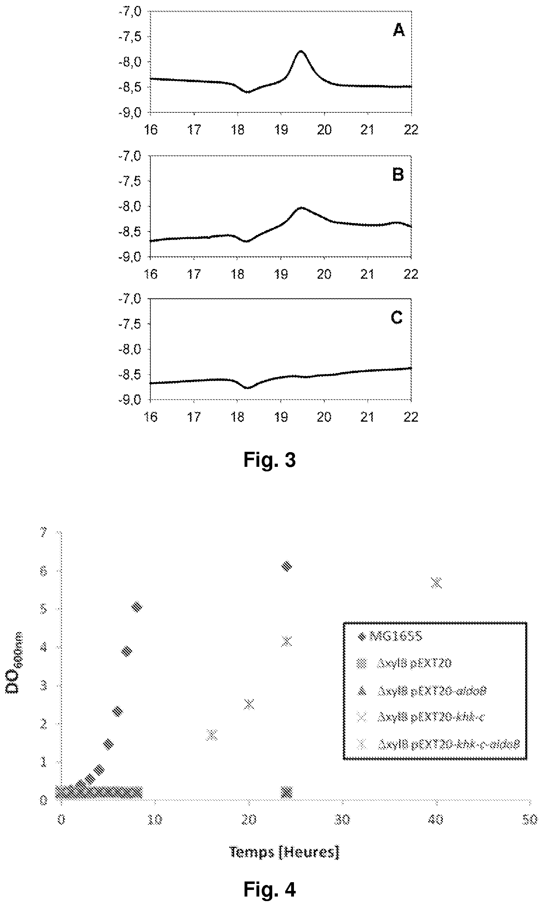

Les enzymes ont été incubées dans le milieu réactionnel contenant: 0.4 mM de NADH, 4 mM d'ATP, un tampon Hépès pH 7 (55 mM Hépès, 45 mM KCI, 4 mM MgCl₂ ajusté à pH 7 avec du KOH) pour un volume final de 500 µl. Khk-C a été rajouté à 0,005 Unité/ml tandis que FbaB à 100 µg/ml. GldA a été ajouté à 1 U/ml. La réaction a été démarrée par ajout de 20 mM de L-ribulose (Sigma-Aldrich). Après un temps d'incubation de 3 h, l'éthylène glycol produit au cours de la réaction a été quantifié par HPLC. Les résultats sont présentés en figure 3.

L'apparition de l'éthylène glycol dans la réaction qui contient la (L)-ribulose-1-kinase, la (L)-ribulose-1P aldolase et la glycolaldéhyde réductase démontre le fonctionnement de la voie synthétique.

### Exemple 4 : Fonctionnement in vivo de la voie métabolique synthétique d'assimilation du (D)-xylose.

### Clonage des gènes de la voie synthétique en opéron

Les gènes *H. sapiens khk-C,* codant pour isoforme C de l'enzyme kétohexokinase (Khk), de séquence SEQ ID N°1 et *aldoB* codant pour l'isoforme B de la fructose -1,6 aldolase de séquence SEQ ID N°2 ont été clonés en opéron sur unplasmide pEXT20 (Dykxhoorn, (1996).) sous contrôle d'un promoteur inductible à l'IPTG construit comme suit. Le gène humain *khk-C* a été fourni par le Dr. Asipu (Asipu *et al.,* 2003) et amplifié avec les amorces P13 et P14 (Tableau 3). L'aldolase a été synthétisée avec une optimisation de codons pour *E. coli* par Eurofins™ et amplifiée par PCR avec les amorces P15 et P16 (Tableau 3). Les amorces pour l'amplification des deux gènes ont été conçues pour donner des fragments PCR qui peuvent être utilisés avec le kit In-Fusion de Clonetech par l'addition d'une queue de 17 nt d'homologie avec le fragment adjacent. Un RBS canonique (AGGAGG) a été ajouté aux séquences de *khk-C* et *aldoB.* Le plasmide pEXT20 a été digéré par les enzymes de restriction BamHI et SacI. Le kit In-Fusion de Clonetech a été utilisé pour liguer par recombinaison les deux fragments de PCR et le pEXT20 linéarisé donnant le plasmide *pEXT20-khk-C-aldoB.* Le vecteur *pEXT20-khk-C-aldoB* ainsi obtenue a été verifié par séquençage de contenir les gènes avec les séquences correctes. Ce plasmide a été transformé dans une souche *ΔxylB* de *E. coli* MG1655, dans laquelle *xylB*, de séquence SEQ ID53, codant pour la xylulose-5-kinase était délété. Les deux gènes *khk-c* et *aldoB* ont également été clonés individuellement sur le pEXT20 en étant d'abord amplifié par P60 et P61 et P62 et P63 respectivement puis ligué dans pEXT20 par restriction avec les enzymes BamHI et SalI donnant les plasmides pEXT20-*khk-C* et pEXT20-*aldoB*.

### Test de la voie synthétique par suivi de la croissance d'une souche ΔxylB sur (D)-xylose

La croissance bactérienne ainsi que la production d'éthylène glycol a été testée en milieu liquide M9 comprenant 120 mM (D)-xylose en tant que seul source carbonée, en présence d'IPTG.

Pour contrôler la capacité de souches ne possédant pas la voie d'assimilation naturelle du (D)-xylose à pousser en présence de (D)-xylose, les souches MG1655, MG1655 *ΔxylB et* MG1655 *ΔxylB* portant le pEXT20- *khk-C,* pEXT20-aldoB ou *pEXT20-khk-C-aldoB* ont été testées.

Sans la voie synthétique, seule la souche sauvage pousse dans ces conditions. La perte de XylB ne permet pas la croissance sur xylose. De plus, ni la présence de Khk-C, ni la présence d'AldoB ne permet pas de restaurer une croissance par l'utilisation de la voie naturelle d'assimilation du xylose (Figure 4). En revanche la souche MG1655 *ΔxylB,* qui porte le plasmide pEXT20-khkC-aldoB, a été capable de pousser sur xylose.

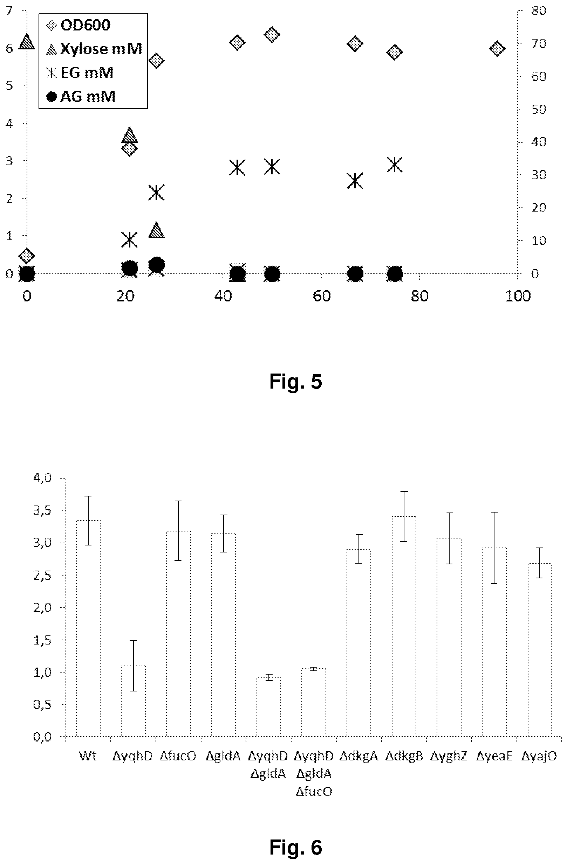

En suivant la production de métabolites durant la croissance par HPLC, l'éthylène glycol a été identifié comme produit majeur de la fermentation du xylose en passant par la voie synthétique produit avec un rendement de 0,45 mol par mol xylose (0.19 g EG par g xylose) (Figure 5).

La voie synthétique d'assimilation du (D)-xylose est donc fonctionnelle *in vivo* et restaure la croissance d'un mutant *ΔxylB* sur ce même sucre.

### Exemple 5 : Identification de la glycolaldéhyde réductase principale

L'optimisation de la production d'éthylène glycol dépend de l'identification et la surexpression de la glycolaldéhyde réductase responsable de la conversion du glycolaldéhyde en éthylène glycol. Plusieurs oxydoréductases ayant une activité sur glycolaldéhyde naturellement présentes chez *E. coli,* GldA, YqhD, FucO, DkgA, DkgB, YghZ, YeaE, YajO ont été identifiées (Lee et al., 2013). Des mutants de ces gènes récupérés de la collection KEIO pour déterminer si l'un de ces gènes code pour la glycolaldéhyde réductase principale. Pour cela, la capacité de ces mutants à générer de l'éthylène glycol à partir de glycolaldéhyde est testée. Ces souches ont été cultivées en milieu M9 en présence de 133 mM xylose et lorsque la DO₆₀₀ₙₘ atteint 1, les cellules ont été exposées à 10 mM de glycolaldéhyde. La quantité d'éthylène glycol a ensuite été mesurée par HPLC après 12 h de culture.

L'absence d'YqhD diminue drastiquement la production d'éthylène glycol à partir du glycolaldéhyde (Figure 6) suggérant qu'elle est la glycolaldéhyde réductase principale chez *E. coli* dans nos conditions de culture.

### Exemple 6 : Optimisation de la souche pour la production de l'éthylène glycol par la voie synthétique de l'invention

Pour améliorer la production d'éthylène glycol, la glycolaldéhyde réductase principale est surexprimée sur un plasmide pACT3. Pour cela, *yqhD* (SEQ ID N°4) a été amplifié avec P11 et P12 puis cloné dans le pACT3 par in fusion, préalablement digéré par Pstl et HindIII. L'insertion *d'yqhD* dans le pACT3 linéarisé a été effectuée par recombinaison grâce au kit In-Fusion (Clonetech), donnant le plasmide pACT3-yqhD. Le vecteur pACT3-yqhD ainsi obtenu a été vérifié par séquençage. Le produit de ligation a été ensuite transformé dans les souches MG1655 d'intérêt. De la même manière, *gldA* (SEQ ID 51) et *fucO* (SEQ ID 52) (respectivement amplifiés par P24 et P25 ; et P26 et P27), ont été clonés par In-Fusion dans le pACT3 préalablement digéré par Pstl et Hindlll afin de tester leur effet.

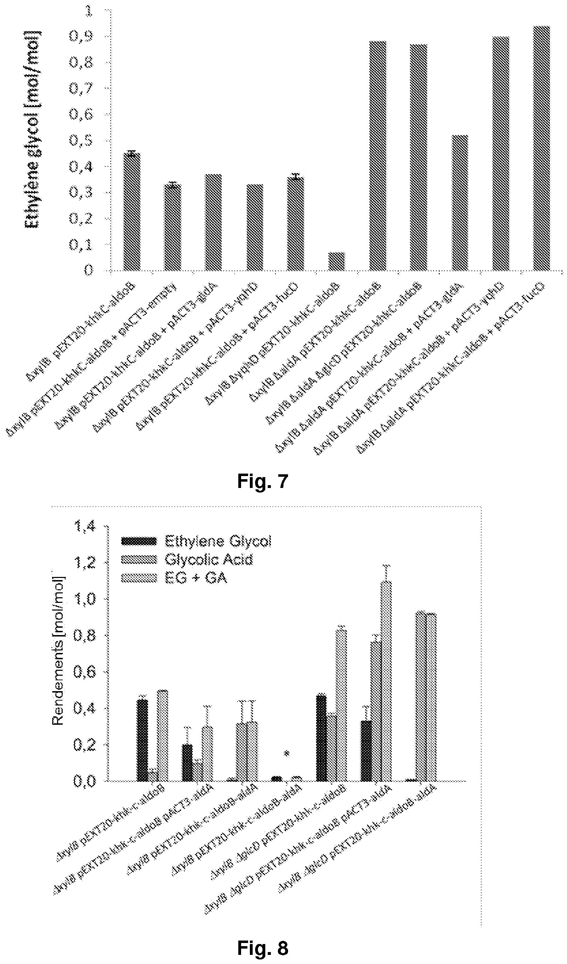

Dans nos conditions de culture, la réductase majeure du glycolaldéhyde est YqhD mais ni sa surexpression ni celle des autres réductases GldA et FucO n'augmentent la production d'éthylène glycol. Le rendement n'est en effet que de 0,45 mol/mol de xylose (0.19 g EG par g xylose), un rendement comparable à celui de la souche MG1655 *ΔxylB* qui exprime le plasmide pEXT20*-khkC-aldoB* (Figure 7).

Pour augmenter la production d'éthylène glycol, la voie de l'oxydation du glycolaldéhyde en acide glycolique doit être bloquée. Pour cela, l'impact de la délétion du gène de l'aldéhyde déhydrogénase *AldA* (SEQ ID N°3) a été testée. Pour quantifier la production résiduelle de l'acide glycolique, la reconsommation de cet acide a été bloquée par l'inactivation de la glycolate déhydrogénase (voir exemple 7) par la délétion de sa sous-unité GlcD (codée par le gène de séquence SEQ ID N°7). Les délétions de *aldA* et/ ou *glcD* dans une souche *ΔxylB* ont donc été entreprises par transduction depuis une souche KEIO. Ces constructions donnent les souches MG1655 *ΔxylB ΔaldA* et MG1655 *ΔxylB ΔaldA ΔglcD* qui sont ensuite transformées par *pEXT20-khkC-aldoB.*

Grâce à la délétion d'*aldA,* le rendement de l'EG augmente fortement. En effet, la souche MG1655 *ΔxylB ΔaldA* portant le plasmide *pEXT20-khkC-aldoB* produit de l'éthylène glycol jusqu'à un rendement de 0,88 mol par mol xylose (0.36 g EG par g xylose)(Figure 7). La surexpression de YqhD et FucO dans ces conditions permet d'atteindre un rendement de 0,9 et 0,94 respectivement mol/ mol (0.38 et 0.39 g EG par g xylose, respectivement). Ceci est très proche du rendement théorique maximal attendu qui est de 1 mol/ mol.

### Exemple 7 : Optimisation de la souche pour la production de l'acide glycolique par la voie synthétique d'assimilation du D-xylose

Pour augmenter la production de l'acide glycolique via la voie synthétique de l'assimilation de pentoses, la glycolaldéhyde déhydrogénase AldA de *E. coli* a été surexprimée. Pour cela, *aldA* a été amplifié depuis de l'ADN génomique de *E. coli* MG1655 en utilisant la paire de primers (P17 et P18, Tableau 3) et le fragment obtenu a été ligué dans pGEM-T (Promega) selon les instructions du fabricant. Le fragment a été ensuite digéré par les enzymes Kpnl et Hindlll puis ligué dans le pACT3 lui-même linéarisé par les deux mêmes enzymes. Le vecteur pACT3-aldA ainsi obtenue a été vérifié par séquençage de contenir le gène avec la séquence correcte. pACT3-aldA a été ensuite transformé dans la souche MG1655 *ΔxylB pEXT20-khk-C-aldoB* donnant la souche *ΔxylB pEXT20-khk-C-aldoB* pACT3-aldA. Lors de la mise en culture de cette souche sur un milieu M9 + 10 g/Ixylose, la production de l'éthylène glycol a diminuée significativement (rendement de 0,2 mol/mol) mais la production de l'acide glycolique a augmentée seulement transitoirement indiquant la reconsommation de l'acide glycolique produite (Figure 8).

Pour empêcher la reconsommation de l'acide glycolique, la glycolate oxydase, codée par *glcDEF*, a été atténuée. Pour cela, la souche MG1655 *ΔxylB ΔglcD* a été construite par délétion de *glcD* (SEQ ID N°7) via transduction de la mutation depuis une souche de la collection KEIO. La souche MG1655 *ΔxylB ΔglcD* a été transformée avec des plasmides pEXT20-khkC-aldoB, ou pEXT20-aldA-khkC-aldoB. Ce plasmide a été construit à partir du pEXT20-khkC-aldoB clivé par les enzymes de restriction EcoRI et Smal auquel a été cloné par la méthode in fusion le gène *aldA* avec un RBS en amont. Ce gène a été lui-même amplifié par PCR grâce aux primers P24 et P25. Lors de la mise en culture de cette souche contenant pEXT20-khkC-aldoB sur un milieu M9-xylose (10 g/l), la production de l'acide glycolique a augmentée significativement et atteint un rendement de 0,35 mol/mol (0.19 g AG par g xylose) (Figure 8). Lorsque *aldA* est surexprimé à partir du pEXT20, le rendement atteint 0,92 mol/ mol de xylose (0.47 g AG par g xylose).

La délétion de *glcD* permet une accumulation de l'acide glycolique grâce à la surexpression d'*aldA* en utilisant 92 % du flux de carbone provenant de la partie C2 du xylose.

### Exemple 8 : Optimisation de la souche pour la production de l'acide glycolique via le cycle du glyoxylate

La production de l'acide glycolique peut être encore augmentée en combinant le fonctionnement de la voie synthétique optimisée comme décrit ci-dessus avec des interventions génétiques qui conduisent à la production de l'acide glycolique via la voie de glyoxylate comme décrit dans les brevets : US20090155867 (Soucaille, 2009) et US20120315682 (Dischert et al., 2012). En s'appuyant sur ces données publiées, les gènes *aceB* et *glcB*, codant pour des malate synthases, le gène *glc* codant pour glyoxylate carboligase, le gène *arcA* codant pour un répresseur de la réponse aérobie et le gène *icd* codant pour une isocitrate deshydrogénase ont été délétés par le protocole de transduction de phage P1 dans la souche MG1655. L'opéron *glcDEFG* codant pour une glycolate oxidase, *edd-eda* codant pour respectivement une phosphogluconate dehydratase et la Entner-Doudoroff Aldolase ainsi que *iclR*, codant pour le répresseur transcriptionel de la voie du glyoxylate, ont été délétés grâce à la méthode de délétion de Datsenko (Datsenko *et al.,* 2000) en utilisant les primers P52 et P53 et P54 et P55 et 64 et 65 respectivement. Des plasmides pour la surexpression parallèle de l'isocitrate lyase, codée par *aceA* et de la glyoxylate reductase, codée par *ghrA* (ou *ycdW*) ont été construits pour améliorer la production d'acide glycolique via les cycles de Krebs et du glyoxylate. Pour cela, un plasmide pACT3 a été digéré par les enzymes BamHI et HindIII. Le gène *ghrA* a été amplifié par PCR comme décrit précédemment en utilisant la paire de primer P40 et P41 tandis que le gène *aceA* a été amplifié par la paire d'amorces P21 et P22. Les deux fragments amplifiés et le plasmide linéarisé ont été ligués ensemble par l'utilisation du kit In-Fusion (Clonetech). Cette construction a donné le plasmide pACT3-*ghrA-aceA.*

Ce plasmide est ensuite transformé dans la souche qui porte les délétions *ΔaceB ΔglcDEFGB Δgcl Δedd-eda ΔiclR ΔarcA Δicd.* La souche résultante est la souche 1054. Lorsque cette souche est mise en culture sur M9 + glucose, 1,17 mol/ mol d'acide glycolique (0,49 g AG par g glucose)sont produits sans production d'acétate (Tableau 6).

**Tableau 6. Production d'acide glycolique et d'acétate de souches d'E. coli ΔaceB ΔglcDEFGB Δgcl Δedd-eda ΔiclR avec des mutations additionnelles en milieu M9 + 1 % glucose**

| Souche | Mutations additionnelles | Plasmides | AG [mol/mol] | AG [g/g] | Acetate mol/mol |
|---|---|---|---|---|---|
| 1052 | - | *pACT3-ghrA aceA* | 0,15 ± 0,03 | 0,06 ± 0,01 | 0,02 |
| 1053 | *ΔarcA* | *pACT3-ghrA aceA* | 0,15 ± 0,02 | 0,06 ± 0,01 | 0,00 |
| 1054 | *ΔarcA Δicd* | *pACT3-ghrA aceA* | 1,17 | 0,49 | 0,00 |

### Exemple 9 : Optimisation de la souche pour la production d'acide glycolique via la co-utilisation du cycle du glyoxylate et de la voie synthétique d'assimilation du (D)-xylose sur glucose et D-xylose

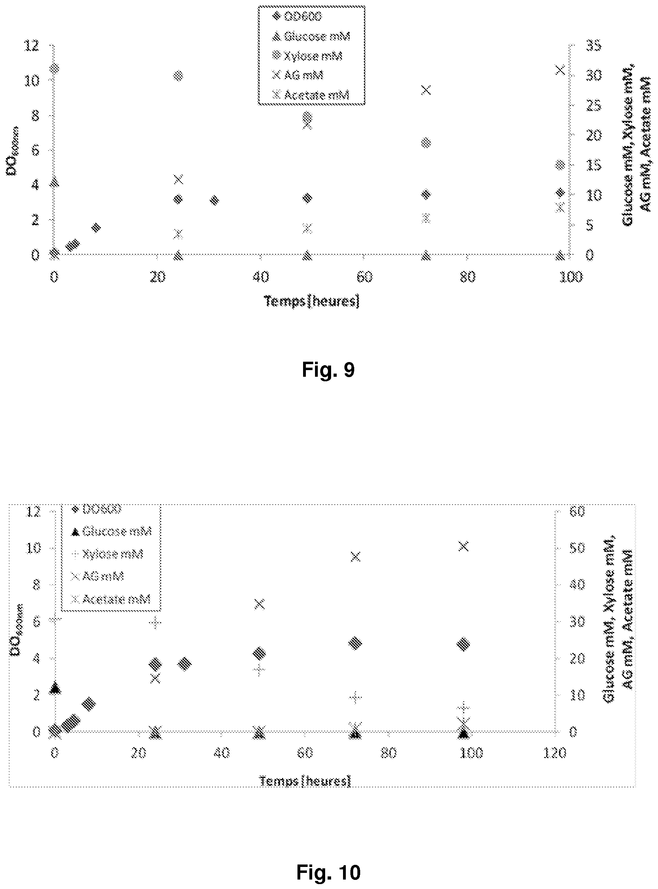

Pour l'application de la voie synthétique d'assimilation de xylose décrite dans ce document, il est préférable de réaliser la production de l'acide glycolique sur des hydrolysats cellulosiques ou hemicellulosiques qui contiennent typiquement du glucose et du xylose en pourcentages différents. Pour démontrer que le rendement de l'acide glycolique sur un substrat qui contient à la fois le sucres glucose et xylose peut être augmenté grâce à la production simultanée de l'acide glycolique via la voie synthétique et via le cycle glyoxylate une souche qui co-exprime les deux voies simultanément a été construite. La souche *E. coli ΔxylB ΔaceB ΔglcDEFGB Δgcl Δedd-eda ΔiclR ΔarcA Δicd* est co-transformée par les plasmides pACT3-*ghrA-aceA* et pEXT20-*khk-c-aldoB-aldA.* La souche ainsi obtenue est la souche 905. Le suivi de production d'acide glycolique par HPLC a été réalisé lors d'une croissance de cette souche sur milieu minéral M9 + 0,1% d'extraits de levures + 0,2% de tryptone et en présence de 0,25% glucose et de 0,5% xylose (Figure 9)

**Tableau 7. Production d'acide glycolique de souches d'E. coli en milieu M9 + 2.5 g/l glucose + 5 g/l (D)-xylose + 1 g/l extrait de levures + 2 g/l tryptone. proD :galP - galP surexprimé avec le promoteur proD constitutif (Davis et al., 2011)(**) Rendement calculé sur la base de sucre total consommé**

| Souche | Génotype | Plasmides | AG** [g/g] |
|---|---|---|---|
| 1054 | *ΔaceB ΔglcDEFGB Δgcl Δedd-eda ΔiclR ΔarcA Δicd* | *pACT3- aceA ghrA* | 0,4 |
| 1044 | *ΔaceB ΔglcDEFGB Δgcl Δedd-eda ΔiclR ΔarcA Δicd* | *pACT3- aceA ghrA pEXT20-khkC-aldoB- aldA* | 0,43 |
| 905 | *ΔxylB ΔaceB ΔglcDEFGB Δgcl Δedd-eda ΔiclR ΔarcA Δicd* | *pACT3- aceA ghrA pEXT20-khkC-aldoB- aldA* | 0,51 |
| 979 | *ΔxylB ΔaceB ΔglcDEFGB Δgcl Δedd-eda ΔiclR ΔarcA Δicd proD :galP* | *pACT3- aceA ghrA pEXT20-khkC-aldoB- aldA* | 0,66 |

La souche consomme d'abord le glucose et ensuite le xylose malgré l'absence de *xylB* montrant ainsi que la voie synthétique est active même dans ces conditions. Après 100 h de culture, 2,35 g/L d'acide glycolique sont produits par la souche avec un rendement sur le sucre utilisé total de 0, 51 g/g (tableau 7). Ce rendement est supérieur à celui obtenu avec une souche quasi-isogénique qui ne possédaient pas la voie synthétique d'assimilation du (D)-xylose (tableau 7, souche 1054), ou qui ne portait pas la délétion du gène *xylB* qui code pour l'enzyme qui catalyse l'entrée dans la voie naturelle d'assimilation du (D)-xylose (tableau 7, souche 1044).

Le taux d'assimilation de xylose après la consommation totale du glucose restait relativement faible avec une valeur d'environ 0.19 mmol/(l h). Pour accélérer l'assimilation du xylose, l'expression de la perméase de sucres *galP* a été rendue constitutive en utilisant la méthode suivante : un fragment d'ADN codant pour le promoteur constitutif *proD* décrit par Davis (Davis *et al.,* 2011) et synthétisé par Eurofins (SEQ ID 90) à été amplifié avec des amorces P56 et P57. Une cassette d'expression du gène *kan* à été amplifiée en utilisant les amorces P58 et P59 et le plasmide pKD4 (SEQ ID 95) comme matrice. Les deux fragments de PCR ont été fusionnés par un overlap extension PCR en utilisant les amorces P59 et P57. Le produit de PCR ainsi obtenu a été transformé dans la souche 905 en utilisant la méthode de Datsenko et Wanner (Datsenko et al., 2000). Des clones résistants à la kanamycine ont été récupérés et vérifiés comme contenant le promoteur synthétique et constitutif devant *galP.*

La nouvelle souche ainsi obtenue est co-transformée par pACT3 *aceA-ghrA* et pEXT20-*khk-C-aldoB-aldA* donnant la souche 979. Sa croissance et le suivi de production d'acide glycolique par HPLC ont été réalisés sur un milieu minéral M9 + 0,1 % extrait de levure + 0,2 % tryptone et en présence de 0,25 % glucose et de 0,5 % xylose (Figure 10).

La souche accumule 3,84 g/L d'acide glycolique avec un rendement de 0,66 g/g sur sucre total. Le taux d'assimilation de xylose après la consommation totale du glucose a été augmenté grâce à la surexpression de GalP et il atteint une valeur de 0.32 mmol/(l h).

### Exemple 10 : Expression de la voie synthétique d'assimilation du xylose chez Saccharomyces cerevisiae

Pour tester la portabilité de la voie synthétique d'assimilation du xylose, nous avons testé son expression dans un autre micro-organisme d'intérêt, la levure *Saccharomyces cerevisiae.*

*S. cerevisiae* ne possède pas de système enzymatique naturel pour convertir le (D)-xylose en (D)-xylulose, et n'est donc pas capable de pousser sur ce sucre. Deux voies métaboliques sont typiquement exprimées de façon hétérologue chez cette levure pour réaliser la conversion du (D)-xylose en (D)-xylulose et pour faciliter sa croissance sur xylose. La xylose isomerase (XI) catalyse la conversion de xylose en xylulose directe et de façon redox-neutre. Comme alternative, l'action séquentielle de la xylose reductase (XR) et de la xylitol déshydrogénase (XDH) permet aussi une conversion de xylose en xylulose, en utilisant les cofacteurs NADPH et NAD, respectivement. Pour montrer le fonctionnement de la voie synthétique d'assimilation du xylose chez la levure, cette voie synthétique a été complétée soit par une xylose isomerase, soit par le système XR/XDH.

Pour compléter la voie synthétique d'assimilation du xylose avec une XI, la XI de *Clostridium phytofermentans* codon optimisée pour S. *cerevisiae* conçue par l'équipe d'Eckhard Boles (Brat et al., 2009) a été utilisée. *khk-C* a été exprimé sous contrôle du promoteur de la triose phosphate isomérase Tpi (Ptpi: SEQ ID N°15) et utilisé le terminateur Trk1 (SEQ ID N°12).

Le gène de l'aldolase AldoB a été placé sous contrôle du promoteur de l'aldolase naturelle de *S*. *cerevisiae* (pFab: SEQ ID N°14) et un terminateur Tlk1 (SEQ ID N°13) a également été utilisé.

Ces gènes ont été clonés par recombinaison chez la levure dans le plasmide p425 linéarisé par KpnI et HindIII donnant p425-khk-aldoB en utilisant les primers suivant (voir également le tableau 4) : P1' et P2' pour *Ptp*i, P3' et P4' pour *khk-C,* P5' et P6' pour *Trk1*, P7' et P8' pour *pFab*, P'9 et P'10 pour *aldoB* et P'11 et P'12 pour *tlk1*.

Le plasmide p425-khk-aldoB a ensuite été utilisé dans une souche compétente incapable de pousser sur xylose, car muté pour sa xylulose kinase naturelle, Xks1 (SEQ ID 89).

Une co-transformation avec un plasmide construit par l'équipe d'Eckhard Boles contenant à la fois la xylulose isomérase et un transporteur xylose Gal-2 a ainsi été réalisée dans la souche CEN.PK-2 *xks1*-.

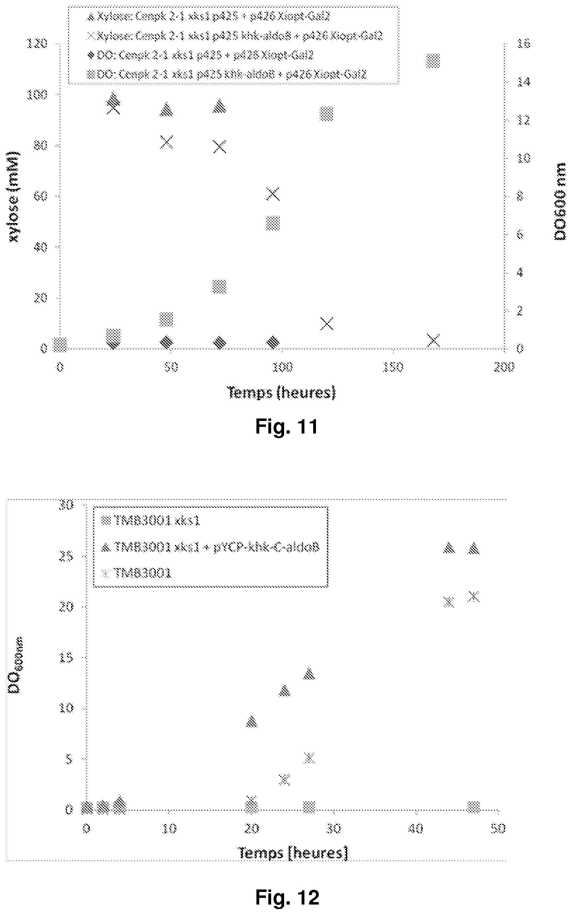

La capacité de la souche à pousser en milieu minimum contenant uniquement du xylose a été testé (Figure 11).

Dans ce cas, alors que le mutant *xks1* n'est pas capable de pousser sur D-xylose, le mutant *xks1* portant la voie synthétique voit sa croissance restaurée indiquant que la voie est fonctionnelle chez la levure.

L'utilisation d'une XI n'est pas le seul moyen possible pour assimiler le xylose en le convertissant en xylulose. En effet, suite à une réaction de réduction catalysée par une xylose réductase (XR) et à une déshydrogénation du xylitol ainsi obtenue catalysée par une xylitol déshydrogénase (XDH), du xylulose est obtenu. Nous avons donc utilisé la souche TMB3001 de (Eliasson *et al.,* 2000) exprimant le système XR/XDH pour tester si la voie synthétique est capable d'être utilisée dans ces conditions pour assimiler le xylose. Un mutant *xks1⁻* a été construit dans la souche TMB3001 en transformant un fragment PCR contenant des bords flanquants homologues aux extrémités non codantes de *xks1* pour générer une délétion par recombinaison homologue. Le fragment PCR est amplifié à partir de la souche BY de la Yeast collection knockout (YKO) (Winzeler *et al.* 1999) avec les primers P26' et P27' et contient une cassette *kan*MX pour la sélection des recombinants. La voie synthétique est éxprimée dans le plasmide pYCP-khk-C-aldoB construit de la façon suivante. Le plasmide pYCP-TPS1 (SEQ ID N°80) a été digéré par AgeI et XbaI pour en extraie la cassette TPS1. Des primers Fw pTpi et rev Tlk1 sont designés pour avoir une queue flottante de 40 nucléotides homologues au plasmide pYCP linéarisé par AgeI et XbaI afin de pouvoir se recombiner dans le plasmide. Nous avons ensuite exprimé *khk-C* codon optimisé pour la levure (SEQ ID N°83) amplifié par P15' et P16' sous contrôle du promoteur de la triose phosphate isomérase Tpi (amplifié par P13' et P14') et du terminateur Trk1 (amplifié par P17' et P18') et exprimé l'aldolase *aldoB* codon optimisé pour la levure (SEQ ID N°84) amplifé pa P21' et P22' sous contrôle du promoteur de l'aldolase naturelle de S. *cerevisiae* pFbaB1 (amplifié par P19' et P20') et du terminateur Tlk1 (amplifié par P23' et P24'). Ces constructions ont été clonées par recombinaison chez la levure dans le plasmide pYCP donnant pYCP-khk-C-aldoB. Ce plasmide a ensuite été utilisé dans la souche TMB3001 *xks1⁻* incapable de pousser sur xylose car mutée pour la xylulose kinase, Xks1.

Alors qu'un mutant TMB3001 *xks1⁻* a perdu sa capacité à pousser sur xylose contrairement à la souche TMB3001, la souche TMB3001 *xks1⁻* portant *pYCP-khk-C-aldoB* retrouve une croissance sur xylose (Figure 12). Ceci suggère que la voie synthétique est également fonctionnelle dans une souche assimilant le xylose via le système XR/XDH.

### Exemple 11 : Fonctionnement in vivo de la voie métabolique synthétique d'assimilation du (L)-arabinose.

### Clonage des gènes de la voie synthétique en opéron

Les gènes *H. sapiens khk-C,* codant pour isoforme C de l'enzyme kétohexokinase (Khk) de séquence SEQ ID N°1 et *fbaB* codant pour une fructose-1,6-bisphosphate aldolase isoforme B *d'E. coli* de séquence SEQ ID N°9 ont été clonées en opéron sur un plasmide pEXT20 (Dykxhoorn et al., 1996) sous contrôle d'un promoteur inductible à l'IPTG construit comme suit. Le gène humain *khk-C* a été fourni par le Dr. Asipu (Asipu *et al.,* 2003) et amplifié avec les amorces P13 et P14 (Tableau 3). L'aldolase a été amplifiée à partir d'ADN génomique de *E. coli* de la souche MG1655 avec les primers P28 et P29 (Tableau 3).

Les amorces pour l'amplification de ces deux gènes ont été conçues pour donner des fragments PCR qui peuvent être utilisés avec le kit In-Fusion de Clonetech par l'addition d'une queue de 17 nucléotides d'homologie avec le fragment adjacent. Des RBS ont été choisis grâce à logiciel RBS calculator (Salis *et al*., 2011). Le gène *fbaB* est donc précédé d'un RBS de séquence (TTAGGAGGTATACT) prédit pour donner une expression maximale et *khk-C* est précédé par un RBS de séquence (ACAGCTTTTAATTATACACTTTAAGGAGGACAGAC) prédit pour minimiser l'expression. Les amorces pour l'amplification des deux gènes avec les nouveaux RBS (P30 et P22 pour *khk-C* et P31 et P32 pour *fbaB*) ont été conçues pour donner des fragments PCR qui peuvent être utilisés avec le kit de fusion par l'addition d'une queue de 15 nucléotides d'homologie avec le fragment adjacent. Le plasmide pEXT20 a été digéré par les enzymes de restriction BamHI et SalI. Le kit In-Fusion de Clonetech a été utilisé pour liguer par recombinaison les deux fragments de PCR et le pEXT20 linéarisé donnant le plasmide pEXT20-khk-C-RBSmax-fbaB. Le plasmide pEXT20-*khk*-C-RBSmax-*fbaB* est transformé dans MG1655 *ΔaraB*. Le vecteur pEXT20-khk-C-RBSmax-fbaB ainsi obtenue a été vérifié par séquençage. De façon analogue les plasmides pEXT20-*khk*-*C* et pEXT20-*fbaB* ont été construits, en utilisant les amorces 83/84 et 33/34 (Tableau 3) par In-Fusion. Ces plasmides ont été transformés dans une souche Δ*araB* de *E. coli* MG1655, dans laquelle *araB*, de séquence SEQ ID88, codant pour la ribulo-5-kinase est délété.

### Test de la voie synthétique par suivi de la croissance d'une souche ΔaraB sur (L)-arabinose

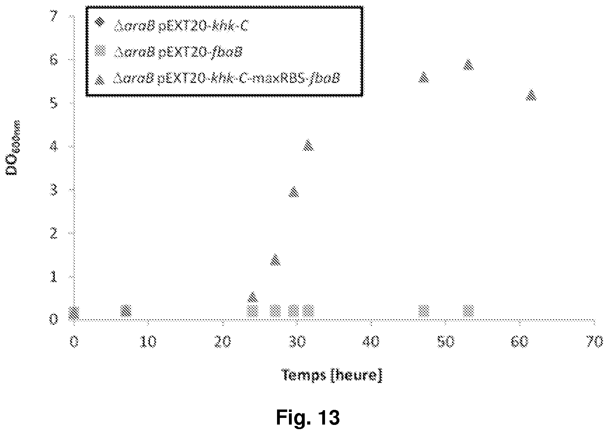

La croissance bactérienne a été testée en milieu liquide M9 comprenant 60 mM (L)-arabinose en tant que seule source carbonée, en présence d'IPTG. Pour contrôler la capacité des souches ne possédant pas la voie naturelle d'assimilation de l'arabinose à pousser en présence de (L)-arabinose en milieu M9, les souches MG1655 Δ*araB* et MG1655 Δ*araB* portant les plasmides pEXT20-*khk*-*C*, pEXT20-*fbaB*, ou pEXT20-khk-C-RBSmax-fbaB ont été testés. Une pré-culture over-night en LB + 100 µM IPTG+ 2 % L-arabinose a été effectuée puis les cellules ont été transférées en milieu M9 + glucose 2 % + arabinose 2 %+ 100 µM IPTG à DO₆₀₀ₙₘ=0,2 puis à DO₆₀₀ₙₘ= 1, les cellules ont été transférées en milieu M9 + L-arabinose 2 %+ 100 µM IPTG à DO₆₀₀ₙₘ= 0,2. Seule la souche qui exprime simultanément les enzymes KhkC et FbaB, ayant des activités (L)-ribulose-1 kinase et (L)-ribulose-1P aldolase, respectivement, pousse dans ces conditions (Figure 13). Ces résultats mettent en évidence le fonctionnement de la voie métabolique synthétique d'assimilation du (L)-arabinose.

**Tableau 3. Amorces utilisées pour l'expression de la voie chez E. coli**

| AA | Amorces | SEQ ID |
|---|---|---|
| P1 | YC-49 rhaB fw-ndeI | 16 |
| P2 | YC-50 rhaB rev-BamHI | 17 |
| P3 | FucokinasefwNcoI | 18 |
| P4 | FucokinaserevBamHISalI | 19 |
| P5 | AldoB_BamHI_Fw | 20 |
| P6 | AldoB_Hind3_Rv | 21 |
| P7 | fbaB-NdeI-fw | 22 |
| P8 | fbaB-BamHI-rev | 23 |
| P9 | agaY-NdeI-fw | 24 |
| P10 | agaY-BamHI-rev | 25 |
| P11 | YC73-Fw YqhD | 26 |
| P12 | YC74-Rev YqhD | 27 |
| P13 | YC52-fwkhkfu | 28 |
| P14 | YC53-revkhkfu | 29 |
| P15 | YC-76 fw AldoB | 30 |
| P16 | YC-75 fw AldoB | 31 |
| P17 | aldA_KpnI_Fw | 32 |
| P18 | aldA_Hind3_Rv | 33 |
| P19 | pACT ycdW-Fw | 34 |
| P20 | pACT ycdW-Rev | 35 |
| P21 | Operon aceA-Fw | 36 |
| P22 | pACT aceA-Rev | 37 |
| P23 | Operon ycdW-Rev | 38 |
| P24 | gldA_rbs_f | 54 |
| P25 | gldA_rbs_r | 55 |
| P26 | fucO_fw_inf | 56 |
| P27 | fucO_rev_inf | 57 |
| P28 | FbaB_oper_BamHI_F | 58 |
| P29 | FbaB_oper_SalI_R | 59 |
| P30 | khkrbsweak fw | 60 |
| P31 | fbabrbsmax fw | 61 |
| P32 | fbabrbsmax rev | 62 |
| P33 | FbaB inf BamHI F | 63 |
| P34 | FbaB inf Hind R | 64 |
| P40 | pACTaceA-Rev | 65 |
| P41 | Op aceA ghrA-Fw | 66 |
| P42 | pACT3 ghrA_XbaI-Rev | 67 |
| P51 | pACTghrA_XbaI-Rev | 68 |
| P52 | glcDEFGB_fw | 69 |
| P53 | glcDEFGB_rev | 70 |
| P54 | edd eda_fw | 71 |
| P55 | edd eda_rev | 72 |
| P56 | prom galP* fw | 73 |
| P57 | prom galP* rev | 74 |
| P58 | disruption k7 fw | 75 |
| P59 | disruption k7 rev | 76 |
| P60 | KHK-Cfw | 77 |
| P61 | KHK-Crev | 78 |
| P62 | AldoB_RBS_BaHI_F | 79 |
| P63 | RevAldoB | 80 |
| P64 | iclR-fw3 | 81 |
| P65 | iclR-rev2 | 82 |

**Tableau 4. Amorces utilisées pour l'expression de la voie chez S.cerevisiae**

| AA | Amorces | SEQ ID |
|---|---|---|
| P1' | YC1_pTPI fw | 39 |
| P2' | YC2_pTPI Rev | 40 |
| P3' | YC3_khk-C fw | 41 |
| P4' | YC4_khk-C rev | 42 |
| P5' | YC5_ter trki fw | 43 |
| P6' | YC6_ter trki rev | 44 |
| P7' | YC7_pFBA1 fw | 45 |
| P8' | YC8_pFBA1 rev | 46 |
| P9' | YC9_aldoB fw | 47 |
| P10' | YC10_aldoB fw | 48 |
| P11' | YC11_Tlk1 terminator fw | 49 |
| P12' | YC12_Tlk1 terminator rev | 50 |
| P13' | fw tpi ycp | 83 |
| P14' | Rev tpi | 84 |
| P15' | Fw khk-C opt | 85 |
| P16' | Rev khk-C opt | 86 |
| P17' | Trk1 fw | 87 |
| P18' | Term-trk1 rev | 88 |
| P19' | pFba1 fw | 89 |
| P20' | pfab1 rev | 90 |
| P21' | aldoB opt fw | 91 |
| P22' | aldoB opt rev | 92 |
| P23' | tlk1 fw | 93 |
| P24' | rev tlk1 ycp | 94 |
| P25' | fw deletion xks1 | 95 |
| P26' | rev deletion xks1 | 96 |

**Tableau 5 : liste de séquences des gènes**

| Espèce | Gène | SEQ ID N° |
|---|---|---|
| *H.sapiens* | *khkC* | 1 |
| *H.sapiens* | *aldoB* | 2 |
| *E.coli* | *aldA* | 3 |
| *E.coli* | *yqhD* | 4 |
| *E.coli* | *fucK* | 5 |
| *E.coli* | *rhaB* | 6 |
| *E.coli* | *glcD* | 7 |
| *E.coli* | *agaY* | 8 |
| *E.coli* | *fbaB* | 9 |
| *E.coli* | *ghrA* | 10 |
| *E.coli* | *aceA* | 11 |
| *S.cerevisiae* | *trk1* | 12 |
| *S.cerevisiae* | *tlk1* | 13 |
| *S.cerevisiae* | *pFab* | 14 |
| *S.cerevisiae* | *pTpi* | 15 |
| *E.coli* | *gldA* | 51 |
| *E.coli* | *fucO* | 52 |
| *E.coli* | *xylB* | 53 |
| *S.cerevisiae* | *Khk-C codon optimisé* | 97 |
| *S.cerevisiae* | *AldoB codon optimisé* | 98 |
| | *YCP-Tps1* | 99 |
| *E.coli* | *araB* | 100 |
| *S.cerevisiae* | *xks1* | 101 |
| *E. coli* | *proD* | 102 |

### Références

Asipu, A., B. E. Hayward, J. O'Reilly, and D. T. Bonthron. 2003. "Properties of normal and mutant recombinant human ketohexokinases and implications for the pathogenesis of essential fructosuria." Diabetes 52 (9):2426-32.
Baba, T., T. Ara, M. Hasegawa, Y. Takai, Y. Okumura, M. Baba, K. A. Datsenko, M. Tomita, B. L. Wanner, and H. Mori. 2006. "Construction of Escherichia coli K-12 in-frame, single-gene knockout mutants: the Keio collection." Mol Syst Biol 2:2006.0008. doi: 10.1038/msb4100050.
Bais, R., H. M. James, A. M. Rofe, and R. A. Conyers. 1985. "The purification and properties of human liver ketohexokinase. A role for ketohexokinase and fructose-bisphosphate aldolase in the metabolic production of oxalate from xylitol." Biochem J 230 (1):53-60.
Brat, D., E. Boles, and B. Wiedemann. 2009. "Functional expression of a bacterial xylose isomerase in Saccharomyces cerevisiae." Appl Environ Microbiol 75 (8):2304-11. doi: 10.1128/aem.02522-08.
Cherepanov, P. P., and W. Wackernagel. 1995. "Gene disruption in Escherichia coli: TcR and KmR cassettes with the option of Flp-catalyzed excision of the antibiotic-resistance determinant." Gene 158 (1):9-14.
Dagert, M., and S. D. Ehrlich. 1979. "Prolonged incubation in calcium chloride improves the competence of Escherichia coli cells." Gene 6 (1):23-8.
Datsenko, K. A., and B. L. Wanner. 2000. "One-step inactivation of chromosomal genes in Escherichia coli K-12 using PCR products." Proc Natl Acad Sci U S A 97 (12):6640-5. doi: 10.1073/pnas.120163297.
Davis, J. H., A. J. Rubin, and R. T. Sauer. 2011. "Design, construction and characterization of a set of insulated bacterial promoters." Nucleic Acids Res 39 (3):1131-41. doi: 10.1093/nar/gkq810.
Dischert, W., and P. Soucaille. 2012a. Method for producing high amount of glycolic acid by fermentation. Google Patents.
Dischert, W., and P. Soucaille. 2012b. Method for producing high amount of glycolic acid by fermentation. Google Patents.
Dykxhoorn, D. M., R. St Pierre, and T. Linn. 1996. "A set of compatible tac promoter expression vectors." Gene 177 (1-2):133-6.
Eliasson, A., C. Christensson, C. F. Wahlbom, and B. Hahn-Hägerdal. 2000. "Anaerobic xylose fermentation by recombinant Saccharomyces cerevisiae carrying XYL1, XYL2, and XKS1 in minerai medium chemostat cultures." Appl Environ Microbiol 66 (8):3381-6.
Gietz, R. D., and R. A. Woods. 2002. "Transformation of yeast by lithium acetate/single-stranded carrier DNA/polyethylene glycol method." Methods Enzymol 350:87-96.
Klamt, S., J. Saez-Rodriguez, J. A. Lindquist, L. Simeoni, and E. D. Gilles. 2006. "A methodology for the structural and functional analysis of signaling and regulatory networks." BMC Bioinformatics 7:56. doi: 10.1186/1471-2105-7-56.
Lee, C., I. Kim, and C. Park. 2013. "Glyoxal detoxification in Escherichia coli K-12 by NADPH dependent aldo-keto reductases." J Microbiol 51 (4):527-30. doi: 10.1007/s12275-013-3087-8.
Liu, H., K. R. Ramos, K. N. Valdehuesa, G. M. Nisola, W. K. Lee, and W. J. Chung. 2013. "Biosynthesis of ethylene glycol in Escherichia coli." Appl Microbiol Biotechnol97 (8):3409-17. doi: 10.1007/s00253-012-4618-7.
Salis, H. M. 2011. "The ribosome binding site calculator." Methods Enzymol 498:19-42. doi: 10.1016/B978-0-12-385120-8.00002-4.
Soucaille, P. 2009. Glycolic Acid Production by Fermentation from Renewable Resources. Google Patents.
Stephanopoulos, Gregory (4 Russet Lane, Winchester, MA, 01890, US), Pereira, Brian (11 Everett Street, Apt. Ng4Cambridge, MA, 02138, US), DE MEY, Marjan (Ursulinen Straat 4/1, Gent, Gent, BE), Dugar, Deepak (69 Chestnut Street, Cambridge, MA, 02139, US), Avalos, Jose, Luis (65 Walnut Street, Arlington, MA, 02476, US). 2013. Engineering microbes and metabolic pathways for the production of ethylene glycol. Massachusetts Institute of Technology (77 Massachusetts Avenue, Cambridge, MA, 02139, US
Winzeler, E. A., D. D. Shoemaker, A. Astromoff, H. Liang, K. Anderson, B. Andre, R. Bangham, R. Benito, J. D. Boeke, H. Bussey, A. M. Chu, C. Connelly, K. Davis, F. Dietrich, S. W. Dow, M. El Bakkoury, F. Foury, S. H. Friend, E. Gentalen, G. Giaever, J. H. Hegemann, T. Jones, M. Laub, H. Liao, N. Liebundguth, D. J. Lockhart, A. Lucau-Danila, M. Lussier, N. M'Rabet, P. Menard, M. Mittmann, C. Pai, C. Rebischung, J. L. Revuelta, L. Riles, C. J. Roberts, P. Ross-MacDonald, B. Scherens, M. Snyder, S. Sookhai-Mahadeo, R. K. Storms, S. Véronneau, M. Voet, G. Volckaert, T. R. Ward, R. Wysocki, G. S. Yen, K. Yu, K. Zimmermann, P. Philippsen, M. Johnston, and R. W. Davis. 1999. "Functional characterization of the S. cerevisiae genome by gene deletion and parallel analysis." Science 285 (5429):901-6.

## Revendications

1. Procédé pour la production d'au moins un métabolite d'intérêt par transformation d'un pentose dans un microorganisme, **caractérisé en ce que** ledit procédé comprend au moins :
(i) une opération de culture d'un microorganisme recombinant exprimant une voie synthétique d'assimilation des pentoses catalysée par un ensemble d'enzymes :
- une enzyme recombinante consistant en une kinase apte à phosphoryler en position 1 un pentose choisi parmi le (D)-Xylulose et/ou le (L)-Ribulose,
- une enzyme consistant en un aldolase apte à cliver le ou les pentoses-1-phosphate pour l'obtention de glycolaldéhyde et de dihydroxyacétone phosphate (DHAP),
et laquelle voie synthétique d'assimilation des pentoses comprend au moins les étapes suivantes :
a) phosphorylation en position 1 d'un pentose choisi parmi le (D)-Xylulose et/ou le (L)-Ribulose, catalysée par ladite kinase,
b) clivage du pentose-1-phosphate obtenu à l'issue de l'étape a), pour l'obtention de glycolaldéhyde et de dihydroxyacétone phosphate (DHAP), catalysé par ladite aldolase,
et
(ii) une opération de récupération dudit au moins un métabolite d'intérêt obtenu à l'issue de l'opération de culture (i).

2. Procédé pour la production d'au moins un métabolite d'intérêt, selon la revendication 1, **caractérisé en ce que** :
- l'étape de phosphorylation a) de la voie synthétique d'assimilation des pentoses est catalysée par une enzyme exprimée de façon recombinante choisie parmi le groupe constitué par la kétohexokinase C (KhkC), la rhamnulose kinase (rhaB) et la fuculose kinase (fucK), et/ou
- l'étape de clivage b) de la voie synthétique d'assimilation des pentoses est catalysée par une aldolase choisie parmi le groupe constitué par l'aldolase B (Aldo-B) et la Fructose-1,6 bisphosphate aldolase (FbaB).

3. Procédé pour la production d'au moins un métabolite d'intérêt, selon la revendication 2, **caractérisé en ce que** :
- l'étape a) est catalysée par la kétohexokinase C, et
- l'étape b) est catalysée par l'aldolase B (Aldo-B) et/ou la fructose -1,6 bisphosphate aldolase B.

4. Procédé pour la production d'au moins un métabolite d'intérêt, selon l'une des revendications 1 à 3, ledit métabolite d'intérêt étant choisi parmi l'éthylène glycol et/ou l'acide glycolique, **caractérisé en ce que** ledit microorganisme recombinant exprime une voie synthétique d'assimilation des pentoses catalysée en outre par les enzymes suivantes :
- une enzyme consistant en une glycolaldéhyde réductase, apte à catalyser la réduction du glycolaldéhyde obtenu à l'issue de l'étape b) en éthylène glycol, et/ou
- une enzyme consistant en une glycolaldéhyde déshydrogénase, apte à catalyser une oxydation du glycolaldéhyde obtenu à l'issue de l'étape b), en acide glycolique,
et **en ce que** ladite voie synthétique d'assimilation des pentoses comprend en outre les étapes suivantes :
c) réduction du glycolaldéhyde obtenu à l'issue de l'étape b) en éthylène glycol, catalysée par ladite glycolaldéhyde réductase et/ou
c') oxydation du glycolaldéhyde obtenu à l'issue de l'étape b), en acide glycolique, catalysée par ladite glycolaldéhyde déshydrogénase.

5. Procédé pour la production d'au moins un métabolite d'intérêt, selon la revendication 4, pour la production d'éthylène glycol et/ou d'acide glycolique, **caractérisé en ce que** :
- l'étape de réduction c) est catalysée par une glycolaldéhyde réductase choisie dans le groupe constitué par l'aldéhyde réductase (YqhD), la glycérol déshydrogénase (GldA) et la Propane-1,2-diol oxidoréductase (FucO), et/ou
- l'étape d'oxydation c') est catalysée par une glycolaldéhyde déshydrogénase consistant en la lactaldéhyde déshydrogénase (AldA).

6. Procédé pour la production d'au moins un métabolite d'intérêt, selon l'une quelconque des revendications précédentes, **caractérisé en ce que** le microorganisme est mis en culture sur un milieu carboné contenant du (D)-Xylose et/ou du (L)-Arabinose.

7. Procédé pour la production d'au moins un métabolite d'intérêt, selon la revendication 6, **caractérisé en ce que** le milieu de culture comprend un hydrolysat de biomasse comprenant de l'hémicellulose.

8. Procédé pour la production d'au moins un métabolite d'intérêt, selon l'une quelconque des revendications précédentes, **caractérisé en ce que** ladite voie synthétique d'assimilation des pentoses comprend, préalablement à l'étape a) :
- une étape de conversion du (D)-Xylose en (D)-Xylulose et/ou,
- une étape de conversion du (L)-Arabinose en (L)-Ribulose.

9. Procédé pour la production d'au moins un métabolite d'intérêt, selon l'une quelconque des revendications précédentes, **caractérisé en ce que** le microorganisme recombinant est choisi entre *E*. *coli*, *S*. *cerevisiae et Scheffersomyces stipitis.*

10. Procédé pour la production d'au moins un métabolite d'intérêt, selon l'une quelconque des revendications précédentes, **caractérisé en ce que** le microorganisme recombinant consiste en un microorganisme dont les activités (D)-xylulose-5 kinase et/ou (L)-ribulose-5 kinase ont été supprimées.

11. Microorganisme recombinant exprimant une voie synthétique d'assimilation des pentoses et comprenant au moins les acides nucléique codant pour les enzymes suivantes :
i) une enzyme consistant en une kinase apte à phosphoryler en position 1 un pentose choisi parmi le (D)-Xylulose et/ou le (L)-Ribulose,
ii) une enzyme consistant en une aldolase apte à cliver le ou les pentoses-1-phosphate obtenus à l'issu de l'étape (i), pour l'obtention de glycolaldéhyde et de dihydroxyacétone phosphate (DHAP),
**caractérisé en ce que** les activités (D)-xylulose-5 kinase et/ou (L)-ribulose-5 kinase dudit microorganisme ont été supprimées, et/ou le microorganisme est porteur d'au moins une des modifications suivantes :
- surexpression d'un gène codant pour une glyoxylate réductase ;
- surexpression d'un gène codant pour une isocitrate lyase accompagné ou non de la délétion de son répresseur transcriptionnel ;
- délétion des gènes codant pour des malate synthases ;
- délétion des gènes codant pour des glyoxylate carboligases ;
- délétion des gènes codant pour des glycolate oxydases et/ou des glycolate déshydrogénases ;
- délétion des gènes codant pour des 2-kéto-4-hydroxyglutarate aldolases, notamment la Entner-Doudouroff Aldolase et pour des phosphogluconate déhydratases ;
- délétion d'un gène codant pour un répresseur des gènes impliqués dans le métabolisme respiratoire, notamment le gène *arcA ;*
- atténuation et notamment délétion de l'expression de l'isocitrate déhydrogénase ;
- délétion des gènes codants pour des systèmes d'internalisation de l'acide glycolique ;
- atténuation des voies métaboliques qui amènent à la production de produit secondaires comme l'acétate, le lactate ou l'éthanol ;
- surexpression d'au moins un gène codant pour un transporteur des sucres.

12. Microorganisme recombinant, selon la revendication 11, **caractérisé en ce qu'**il comprend :
- au moins un acide nucléique exogène codant pour la kétohexokinase C et
- au moins un acide nucléique exogène codant pour l'aldolase B.

13. Microorganisme selon l'une des revendications 11 ou 12 comprenant en outre au moins l'une des modifications suivantes :
- surexpression du gène codant pour la glycolaldéhyde réductase principale ;
- délétion du gène codant pour la glycolaldéhyde déshydrogénase.

14. Microorganisme selon l'une des revendications 11 ou 12 comprenant en outre au moins l'une des modifications suivantes :
- surexpression du gène codant pour la glycolaldéhyde déshydrogénase;
- inactivation d'au moins un des gènes codant pour au moins l'une des sous-unités de la glycolate oxydase.

15. Microorganisme selon la revendication 14 comprenant les modifications suivantes :
- surexpression d'un gène codant pour une glyoxylate réductase ;
- surexpression d'un gène codant pour une isocitrate lyase accompagné ou non de la délétion de son répresseur transcriptionnel ;
- délétion des gènes codant pour des malate synthases ;
- délétion des gènes codant pour des glyoxylate carboligases ;
- délétion des gènes codant pour des glycolates oxydases et/ou des glycolates déshydrogénases ;
- délétion des gènes codant pour des 2-kéto-4-hydroxyglutarate aldolases, notamment la Entner-Doudouroff Aldolase et pour des phosphogluconate déhydratases;
- délétion d'un gène codant pour un répresseur des gènes impliqués dans le métabolisme respiratoire, notamment le gène *arcA* ;
- atténuation et notamment délétion de l'expression de l'isocitrate déshydrogénase ;
- éventuellement la surexpression d'un gène codant pour un transporteur des sucres.

16. Microorganisme selon la revendication 15, comprenant les modifications suivantes :
- la surexpression du gène *aldA* codant pour une glycolaldéhyde déshydrogénase ;
- la surexpression du gène *ghrA*
- la surexpression du gène *aceA* ;
- la délétion du gène *iclR* ;
- la délétion du gène *gcl* ;
- la délétion des gènes *aceB* et *glcB*
- la délétion d'au moins un gène choisi parmi *glcD, glcE, glcF ou glcG*;
- la délétion des gènes edd-eda ;
- la délétion du gène *arcA* ;
- la délétion du gène *icd* ;
- la surexpression du gène *galP*

## Patentansprüche

1. Verfahren zur Herstellung mindestens eines Metaboliten von Interesse durch Umwandlung einer Pentose in einen Mikroorganismus, **dadurch gekennzeichnet, dass** das Verfahren mindestens Folgendes umfasst:
(i) einen Arbeitsschritt der Kultur eines rekombinanten Mikroorganismus, der einen Syntheseweg zur Assimilation von Pentosen exprimiert, der durch eine Gruppe von Enzymen katalysiert wird:
- ein rekombinantes Enzym, das aus einer Kinase besteht, die eine Pentose, ausgewählt aus (D)-Xylulose und/oder (L)-Ribulose, an Position 1 phosphorylieren kann,
- ein Enzym, das aus einer Aldolase besteht, die das oder die Pentose(n)-1-phosphat(e) spalten kann, um Glykolaldehyd und Dihydroxyacetonphosphat (DHAP) zu erhalten,
und der Syntheseweg zur Assimilation von Pentosen mindestens die folgenden Schritte umfasst:
a) durch die Kinase katalysierte Phosphorylierung einer aus (D)-Xylulose und/oder (L)-Ribulose ausgewählten Pentose an Position 1,
b) durch die Aldolase katalysierte Spaltung des am Ende von Schritt a) erhaltenen Pentose-1-phosphats, um Glykolaldehyd und Dihydroxyacetonphosphat (DHAP) zu erhalten,
und
(ii) einen Arbeitsschritt der Gewinnung des mindestens einen Metaboliten von Interesse, der nach dem Kulturschritt (i) erhalten wird.

2. Verfahren zur Herstellung mindestens eines Metaboliten von Interesse nach Anspruch 1, **dadurch gekennzeichnet, dass**:
- der Phosphorylierungsschritt a) des Synthesewegs zur Assimilation von Pentosen durch ein rekombinant exprimiertes Enzym katalysiert wird, ausgewählt aus der Gruppe, bestehend aus Ketohexokinase C (KhkC), Rhamnulosekinase (rhaB) und Fuculosekinase (fucK), und/oder
- der Spaltungsschritt b) des Synthesewegs zur Assimilation von Pentosen durch eine Aldolase katalysiert wird, ausgewählt aus der Gruppe, bestehend aus Aldolase B (Aldo-B) und Fructose-1,6-bisphosphat-Aldolase (FbaB).

3. Verfahren zur Herstellung mindestens eines Metaboliten von Interesse nach Anspruch 2, **dadurch gekennzeichnet, dass**:
- Schritt a) durch Ketohexokinase C katalysiert wird und
- Schritt b) durch Aldolase B (Aldo-B) und/oder Fructose-1,6-bisphosphat-Aldolase B katalysiert wird.

4. Verfahren zur Herstellung mindestens eines Metaboliten von Interesse nach einem der Ansprüche 1 bis 3, wobei der Metabolit von Interesse aus Ethylenglykol und/oder Glykolsäure ausgewählt ist, **dadurch gekennzeichnet, dass** der rekombinante Mikroorganismus einen Syntheseweg zur Assimilation von Pentosen exprimiert, der außerdem durch die folgenden Enzyme katalysiert wird:
- ein Enzym, das aus einer Glykolaldehydreduktase besteht, die die Reduktion des am Ende von Schritt b) erhaltenen Glykolaldehyds zu Ethylenglykol katalysieren kann, und/oder
- ein Enzym, das aus einer Glykolaldehyddehydrogenase besteht, die eine Oxidation des am Ende von Schritt b) erhaltenen Glykolaldehyds zu Glykolsäure katalysieren kann,
- und dadurch, dass der Syntheseweg zur Assimilation von Pentosen außerdem die folgenden Schritte umfasst:
c) durch die Glykolaldehydreduktase katalysierte Reduktion des am Ende von Schritt b) erhaltenen Glykolaldehyds zu Ethylenglykol und/oder
c') durch die Glykolaldehyddehydrogenase katalysierte Oxidation des am Ende von Schritt b) erhaltenen Glykolaldehyds zu Glykolsäure.

5. Verfahren zur Herstellung mindestens eines Metaboliten von Interesse nach Anspruch 4 zur Herstellung von Ethylenglykol und/oder Glykolsäure, **dadurch gekennzeichnet, dass**:
- der Reduktionsschritt c) durch eine Glykolaldehydreduktase katalysiert wird, ausgewählt aus der Gruppe, bestehend aus Aldehydreduktase (YqhD), Glycerindehydrogenase (GldA) und Propan-1,2-diol-Oxidoreduktase (FucO), und/oder
- der Oxidationsschritt c') durch eine Glykolaldehyddehydrogenase katalysiert wird, die aus der Lactaldehyddehydrogenase (AldA) besteht.

6. Verfahren zur Herstellung mindestens eines Metaboliten von Interesse nach einem der vorhergehenden Ansprüche, **dadurch gekennzeichnet, dass** der Mikroorganismus auf einem kohlenstoffhaltigen Medium kultiviert wird, das (D)-Xylose und/oder (L)-Arabinose enthält.

7. Verfahren zur Herstellung mindestens eines Metaboliten von Interesse nach Anspruch 6, **dadurch gekennzeichnet, dass** das Kulturmedium ein Hemicellulose umfassendes Biomassehydrolysat umfasst.

8. Verfahren zur Herstellung mindestens eines Metaboliten von Interesse nach einem der vorhergehenden Ansprüche, **dadurch gekennzeichnet, dass** der Syntheseweg zur Assimilation von Pentosen vor Schritt a) Folgendes umfasst:
- einen Schritt der Umwandlung von (D)-Xylose in (D)-Xylulose und/oder
- einen Schritt der Umwandlung von (L)-Arabinose in (L)-Ribulose.

9. Verfahren zur Herstellung mindestens eines Metaboliten von Interesse nach einem der vorhergehenden Ansprüche, **dadurch gekennzeichnet, dass** der rekombinante Mikroorganismus aus *E. coli, S. cerevisiae* und *Scheffersomyces stipitis* ausgewählt wird.

10. Verfahren zur Herstellung mindestens eines Metaboliten von Interesse nach einem der vorhergehenden Ansprüche, **dadurch gekennzeichnet, dass** der rekombinante Mikroorganismus aus einem Mikroorganismus besteht, dessen (D)-Xylulose-5-Kinase- und/oder (L)-Ribulose-5-Kinase-Aktivitäten supprimiert wurden.

11. Rekombinanter Mikroorganismus, der einen Syntheseweg zur Assimilation von Pentosen exprimiert und mindestens die Nukleinsäuren umfasst, die für die folgenden Enzyme codieren:
i) ein Enzym, das aus einer Kinase besteht, die eine Pentose, ausgewählt aus (D)-Xylulose und/oder (L)-Ribulose, an Position 1 phosphorylieren kann,
ii) ein Enzym, das aus einer Aldolase besteht, die das oder die am Ende von Schritt (i) erhaltene(n) Pentose(n)-1-phosphat(e) spalten kann, um Glykolaldehyd und Dihydroxyacetonphosphat (DHAP) zu erhalten,
**dadurch gekennzeichnet, dass** die (D)-Xylulose-5-Kinase- und/oder (L)-Ribulose-5-Kinase-Aktivitäten des Mikroorganismus supprimiert wurden und/oder der Mikroorganismus mindestens eine der folgenden Modifikationen beherbergt:
- Überexpression eines für eine Glyoxylatreduktase codierenden Gens,
- Überexpression eines für eine Isocitratlyase codierenden Gens mit oder ohne gleichzeitige Deletion seines Transkriptionsrepressors,
- Deletion der für Malatsynthasen codierenden Gene,
- Deletion der für Glyoxylatcarboligasen codierenden Gene,
- Deletion der für Glykolatoxidasen und/oder Glykolatdehydrogenasen codierenden Gene,
- Deletion der für 2-Keto-4-hydroxyglutarat-Aldolasen, insbesondere Entner-Doudouroff-Aldolase, und Phosphogluconatdehydratasen codierenden Gene,
- Deletion eines Gens, das für einen Repressor der am Atmungsstoffwechsel beteiligten Gene codiert, insbesondere des Gens *arcA,*
- Attenuation und insbesondere Deletion der Expression von Isocitratdehydrogenase,
- Deletion der Gene, die für Glykolsäureinternalisierungssysteme codieren,
- Attenuation der Stoffwechselwege, die zur Produktion von Nebenprodukten, wie Acetat, Lactat oder Ethanol, führen,
- Überexpression mindestens eines Gens, das für einen Zuckertransporter codiert.

12. Rekombinanter Mikroorganismus nach Anspruch 11, **dadurch gekennzeichnet, dass** er Folgendes umfasst:
- mindestens eine exogene Nukleinsäure, die für Ketohexokinase C codiert, und
- mindestens eine exogene Nukleinsäure, die für Aldolase B codiert.

13. Mikroorganismus nach einem der Ansprüche 11 oder 12, der außerdem mindestens eine der folgenden Modifikationen umfasst:
- Überexpression des für die Haupt-Glykolaldehydreduktase codierenden Gens,
- Deletion des für die Glykolaldehyddehydrogenase codierenden Gens.

14. Mikroorganismus nach einem der Ansprüche 11 oder 12, der außerdem mindestens eine der folgenden Modifikationen umfasst:
- Überexpression des für die Glykolaldehyddehydrogenase codierenden Gens,
- Inaktivierung mindestens eines der Gene, die für mindestens eine der Untereinheiten der Glykolatoxidase codieren.

15. Mikroorganismus nach Anspruch 14, der die folgenden Modifikationen umfasst:
- Überexpression eines für eine Glyoxylatreduktase codierenden Gens,
- Überexpression eines für eine Isocitratlyase codierenden Gens mit oder ohne gleichzeitige Deletion seines Transkriptionsrepressors,
- Deletion der für Malatsynthasen codierenden Gene,
- Deletion der für Glyoxylatcarboligasen codierenden Gene,
- Deletion der für Glykolatoxidasen und/oder Glykolatdehydrogenasen codierenden Gene,
- Deletion der für 2-Keto-4-hydroxyglutarat-Aldolasen, insbesondere Entner-Doudouroff-Aldolase, und Phosphogluconatdehydratasen codierenden Gene,
- Deletion eines Gens, das für einen Repressor der am Atmungsstoffwechsel beteiligten Gene codiert, insbesondere des Gens *arcA*,
- Attenuation und insbesondere Deletion der Expression von Isocitratdehydrogenase,
- gegebenenfalls Überexpression eines Gens, das für einen Zuckertransporter codiert.

16. Mikroorganismus nach Anspruch 15, der die folgenden Modifikationen umfasst:
- Überexpression des *aldA*-Gens, das für eine Glykolaldehyddehydrogenase codiert,
- Überexpression des *ghrA*-Gens,
- Überexpression des *aceA*-Gens,
- Deletion des *iclR*-Gens,
- Deletion des *gcl*-Gens,
- Deletion des *aceB*- und des *glcB*-Gens,
- Deletion mindestens eines Gens, ausgewählt aus *gleD*, *gleE*, *glcF* oder *glcG*,
- Deletion der *edd-eda*-Gene,
- Deletion des *arcA*-Gens,
- Deletion des *icd*-Gens,
- Überexpression des *galP*-Gens.

## Claims

1. Process for producing at least one metabolite of interest by conversion of a pentose in a microorganism, **characterized in that** said process comprises at least:
(i) an operation of culturing a recombinant microorganism expressing a synthetic pathway for pentose assimilation catalyzed by a set of enzymes:
- a recombinant enzyme consisting in a kinase capable to phosphorylate in position 1 a pentose selected from (D)-Xylulose and/or (L)-Ribulose,
- a enzyme consisting in a aldolase capable to cleavage the pentose-1-phosphate in order to obtain glycolaldehyde and dihydroxyacetone phosphate (DHAP),
and wherein said synthetic pathway for pentose assimilation comprises at least the following steps:
a) phosphorylation in position 1 of a pentose selected from (D)-Xylulose and/or (L)-Ribulose, catalyzed by said kinase,
b) cleavage of the pentose-1-phosphate obtained at the end of step a) in order to obtain glycolaldehyde and dihydroxyacetone phosphate (DHAP), catalyzed by said aldolase, and
(ii) an operation of recovering said at least one metabolite of interest obtained at the end of the culturing operation (i).

2. Process for producing at least one metabolite of interest according to claim 1, **characterized in that**:
- the phosphorylation step a) of the synthetic pathway for pentose assimilation is catalyzed by a recombinantly expressed enzyme selected from the group consisting of ketohexokinase C (KhkC), rhamnulose kinase (rhaB) and fuculose kinase (fucK), and/or
- the cleavage step b) of the synthetic pathway for pentose assimilation is catalyzed by an aldolase selected from the group consisting of aldolase B (Aldo-B) and fructose-1,6 bisphosphate aldolase (FbaB).

3. Process for producing at least one metabolite of interest according to claim 2, **characterized in that**:
- the step a) is catalyzed by ketohexokinase C, and
- the step b) is catalyzed by aldolase B (Aldo-B) and/or fructose 1,6 bisphosphate aldolase B.

4. Process for producing at least one metabolite of interest according to any of claims 1 to 3, said metabolite of interest being selected from ethylene glycol and/or glycolic acid, **characterized in that** said recombinant microorganism expresses a synthetic pathway for pentose assimilation further catalyzed by the following enzymes:
- a enzyme consisting in glycolaldehyde reductase, capable to catalyze reduction of the glycolaldehyde obtained at the end of step b) to ethylene glycol, and/or
- a enzyme consisting in glycolaldehyde desydrogenase, capable to catalyze an oxidation of the glycolaldehyde obtained at the end of step b) to glycolic acid,
wherein said synthetic pathway for pentose assimilation further comprises the following steps:
c) reduction of the glycolaldehyde obtained at the end of step b) to ethylene glycol, catalyzed by said glycolaldehyde reductase and/or
c') oxidation of the glycolaldehyde obtained at the end of step b) to glycolic acid, catalyzed by said glycolaldehyde desydrogenase.

5. Process for producing at least one metabolite of interest according to claim 4, for the production of ethylene glycol and/or glycolic acid, **characterized in that**:
- the reduction step c) is catalyzed by a glycolaldehyde reductase selected from the group consisting of aldehyde reductase (YqhD), glycerol dehydrogenase (GIdA) and propane-1,2-diol oxidoreductase (FucO), and/or
- the oxidation step c') is catalyzed by a glycolaldehyde dehydrogenase consisting of the lactaldehyde dehydrogenase (AldA).

6. Process for producing at least one metabolite of interest according to any one of the preceding claims, **characterized in that** the microorganism is cultured on a carbon medium containing (D)-Xylose and/or (L)-Arabinose.

7. Process for producing at least one metabolite of interest according to claim 6, **characterized in that** the culture medium comprises a biomass hydrolysate comprising hemicellulose.

8. Process for producing at least one metabolite of interest according to any one of the preceding claims, **characterized in that** said synthetic pathway for pentose assimilation comprises, prior to step a):
- a step of converting (D)-Xylose into (D)-Xylulose, and/or
- a step of converting (L)-Arabinose into (L)-Ribulose.

9. Process for producing at least one metabolite of interest according to any one of the preceding claims, **characterized in that** the recombinant microorganism is selected from *E*. *coli*, *S. cerevisiae* and *Scheffersomyces stipitis.*

10. Process for producing at least one metabolite of interest according to anyone of the preceding claims, **characterized in that** the recombinant microorganism consists in a microorganism whose the (D)-xylulose-5 kinase and/or (L)-ribulose-5 kinase activities were suppressed.

11. Recombinant microorganism expressing a synthetic pathway for pentose assimilation and comprising at least the nucleic acids encoding the following enzymes:
i) an enzyme consisting in a kinase capable to phosphorylate in position 1 a pentose selected from (D)-Xylulose and/or (L)-Ribulose,
ii) an enzyme consisting in an aldolase capable to cleave the pentose(s)-1-phosphate obtained at the end of step (i) for obtaining glycolaldehyde and dihydroxyacetone phosphate (DHAP),
**characterized in that** the (D)-xylulose-5 kinase and/or (L)-ribulose-5 kinase activities of said microorganism were suppressed, and/or the microorganism carries at least one of the following modifications:
- overexpression of a gene encoding a glyoxylate reductase;
- overexpression of a gene encoding an isocitrate lyase with or without the deletion of its transcriptional repressor;
- deletion of the genes encoding malate synthases;
- deletion of the genes encoding glyoxylate carboligases;
- deletion of the genes encoding glycolate oxidases and/or glycolate dehydrogenases;
- deletion of the genes encoding 2-keto-4-hydroxyglutarate aldolases, including Entner-Doudouroff Aldolase, and phosphogluconate dehydratases;
- deletion of a gene encoding a repressor of the genes involved in the respiratory metabolism, including the *arcA* gene;
- attenuation and in particular, deletion of the isocitrate dehydrogenase expression;
- deletion of the genes encoding glycolic acid internalization systems;
- attenuation of the metabolic pathways which lead to the production of byproducts such as acetate, lactate or ethanol;
- overexpression of at least one gene encoding a sugar carrier.

12. Recombinant microorganism according to claim 11, **characterized in that** it comprises:
- at least an exogenous nucleic acid encoding the ketohexokinase C and
- at least an exogenous nucleic acid encoding the aldolase B.

13. Microorganism according to one of claims 11 or 12, further comprising at least one of the following modifications:
- overexpression of the gene encoding the main glycolaldehyde reductase;
- deletion of the gene encoding the glycolaldehyde dehydrogenase.

14. Microorganism according to one of claims 11 or 12, further comprising at least one of the following modifications:
- overexpression of the gene encoding the glycolaldehyde dehydrogenase;
- inactivation of at least one of the genes encoding at least one of the glycolate oxidase subunits.

15. Microorganism according to claim 14, comprising the following modifications:
- overexpression of a gene encoding a glyoxylate reductase;
- overexpression of a gene encoding an isocitrate lyase, with or without the deletion of its transcriptional repressor;
- deletion of the genes encoding malate synthases;
- deletion of the genes encoding glyoxylate carboligases;
- deletion of the genes encoding glycolate oxidases and/or glycolate dehydrogenases
- deletion of the genes encoding 2-keto-4-hydroxyglutarate aldolases, including Entner-Doudouroff Aldolase, and phosphogluconate dehydratases;
- deletion of a gene encoding a repressor of the genes involved in the respiratory metabolism, including the *arcA* gene;
- attenuation and in particular, deletion of the isocitrate dehydrogenase expression;
- optionally, overexpression of a gene encoding a sugar carrier.

16. Microorganism according to claim 15, comprising the following modifications:
- overexpression of the *aldA* gene encoding a glycolaldehyde dehydrogenase;
- overexpression of the *ghrA* gene;
- overexpression of the *aceA* gene;
- deletion of the *iclR* gene;
- deletion of the *gcl* gene;
- deletion of the *aceB* and *glcB* genes;
- deletion of at least one gene selected from *glcD*, *glcE*, *glcF* or *glcG*;
- deletion of the *edd-eda* genes;
- deletion of the *arcA* gene;
- deletion of the *icd* gene;
- overexpression of the *galP* gene.


### SEQUENCE LISTING

<110> INRA INSA Toulouse CNRS
<120> PROCEDE POUR LA PRODUCTION D'AU MOINS UN METABOLITE D'INTERET PAR TRANSFORMATION D'UN PENTOSE DANS UN MICROORGANISME
<130> Z376WO
<160> 102
<170> PatentIn version 3.5
<210> 1
   <211> 897
   <212> DNA
   <213> Homo sapiens
<400> 1
   atggaagaga agcagatcct gtgcgtgggg ctagtggtgc tggacgtcat cagcctggtg      60
   gacaagtacc ctaaggagga ctcggagata aggtgtttgt cccagagatg gcagcgcgga     120
   ggcaacgcgt ccaactcctg caccgttctc tccctgctcg gagccccctg tgccttcatg     180
   ggctcaatgg ctcctggcca tgttgctgac ttcctggtgg ccgacttcag gcggcggggc     240
   gtggacgtgt ctcaggtggc ctggcagagc aagggggaca cccccagctc ctgctgcatc    300
   atcaacaact ccaatggcaa ccgtaccatt gtgctccatg acacgagcct gccagatgtg     360
   tctgctacag actttgagaa ggttgatctg acccagttca agtggatcca cattgagggc     420
   cggaacgcat cggagcaggt gaagatgctg cagcggatag acgcacacaa caccaggcag     480
   cctccagagc agaagatccg ggtgtccgtg gaggtggaga agccacgaga ggagctcttc     540
   cagctgtttg gctacggaga cgtggtgttt gtcagcaaag atgtggccaa gcacttgggg     600
   ttccagtcag cagaggaagc cttgaggggc ttgtatggtc gtgtgaggaa aggggctgtg     660
   cttgtctgtg cctgggctga ggagggcgcc gacgccctgg gccctgatgg caaattgctc     720
   cactcggatg ctttcccgcc accccgcgtg gtggatacac tgggagctgg agacaccttc     780
   aatgcctccg tcatcttcag cctctcccag gggaggagcg tgcaggaagc actgagattc     840
   gggtgccagg tggccggcaa gaagtgtggc ctgcagggct ttgatggcat cgtgtga         897
<210> 2
   <211> 1095
   <212> DNA
   <213> Homo sapiens
<400> 2
   atggcacatc gctttccggc tctgacgcaa gaacagaaga aagaattgtc agagattgcg      60
   cagtccatcg tggcgaatgg caaaggtatt ctggccgcag atgagagcgt gggaaccatg     120
   ggtaatcggc ttcaacgcat caaagtggag aacaccgaag aaaatcggcg tcagtttcgc     180
   gaaatcctct ttagcgtcga cagtagcatc aatcagtcca ttggcggggt gattctgttt 240
   cacgaaacgc tgtaccagaa agactcgcaa gggaaactgt tccgcaacat tctgaaagag 300
   aaaggcattg tggtgggcat caaactggat cagggtggcg cacccttagc tggcactaat 360
   aaggagacaa cgattcaagg cctcgatggg ctttctgaac gttgtgccca gtataagaag 420
   gatggtgttg actttggcaa atggcgcgcg gtactgcgta tcgctgatca gtgccctagc 480
   agtctggcca ttcaggaaaa cgcgaatgcg ttagcacgct atgcgagcat ttgccaacag 540
   aacggcctgg ttcccattgt cgagccggaa gtaatcccgg atggcgatca tgacctggaa 600
   cactgccagt atgtcaccga gaaagttctg gcggcggtgt ataaagccct gaatgaccat 660
   cacgtgtact tggaaggtac cctcttgaaa ccgaacatgg taactgccgg acatgcctgc 720
   accaagaaat acacccctga acaagtcgcc atggcaacag ttacggcttt acaccgcact 780
   gttccagcgg cggttccggg tatttgcttc ctgtcaggtg gcatgtcgga agaagatgcc 840
   accctcaacc tgaatgcgat caacttgtgt ccactgccga agccgtggaa actgtcattc 900
   tcctatggac gtgcccttca ggccagtgcg ttagcggcat ggggtgggaa agcggcaaac 960
   aaagaagcta cccaggaagc cttcatgaaa cgtgcaatgg cgaactgtca agctgcgaaa 1020
   ggccagtacg tccataccgg ttctagcggt gctgcatcga cacaatctct gtttacggcc 1080
   tgttacacgt attaa 1095
<210> 3
   <211> 1440
   <212> DNA
   <213> Escherichia coli
<400> 3
   atgtcagtac ccgttcaaca tcctatgtat atcgatggac agtttgttac ctggcgtgga        60
   gacgcatgga ttgatgtggt aaaccctgct acagaggctg tcatttcccg catacccgat       120
   ggtcaggccg aggatgcccg taaggcaatc gatgcagcag aacgtgcaca accagaatgg       180
   gaagcgttgc ctgctattga acgcgccagt tggttgcgca aaatctccgc cgggatccgc       240
   gaacgcgcca gtgaaatcag tgcgctgatt gttgaagaag ggggcaagat ccagcagctg        300
   gctgaagtcg aagtggcttt tactgccgac tatatcgatt acatggcgga gtgggcacgg       360
   cgttacgagg gcgagattat tcaaagcgat cgtccaggag aaaatattct tttgtttaaa       420
   cgtgcgcttg gtgtgactac cggcattctg ccgtggaact tcccgttctt cctcattgcc       480
   cgcaaaatgg ctcccgctct tttgaccggt aataccatcg tcattaaacc tagtgaattt       540
   acgccaaaca atgcgattgc attcgccaaa atcgtcgatg aaataggcct tccgcgcggc       600
   gtgtttaacc ttgtactggg gcgtggtgaa accgttgggc aagaactggc gggtaaccca       660
   aaggtcgcaa tggtcagtat gacaggcagc gtctctgcag gtgagaagat catggcgact       720
   gcggcgaaaa acatcaccaa agtgtgtctg gaattggggg gtaaagcacc agctatcgta       780
   atggacgatg ccgatcttga actggcagtc aaagccatcg ttgattcacg cgtcattaat       840
   agtgggcaag tgtgtaactg tgcagaacgt gtttatgtac agaaaggcat ttatgatcag       900
   ttcgtcaatc ggctgggtga agcgatgcag gcggttcaat ttggtaaccc cgctgaacgc       960
   aacgacattg cgatggggcc gttgattaac gccgcggcgc tggaaagggt cgagcaaaaa      1020
   gtggcgcgcg cagtagaaga aggggcgaga gtggcgttcg gtggcaaagc ggtagagggg      1080
   aaaggatatt attatccgcc gacattgctg ctggatgttc gccaggaaat gtcgattatg      1140
   catgaggaaa cctttggccc ggtgctgcca gttgtcgcat ttgacacgct ggaagatgct      1200
   atctcaatgg ctaatgacag tgattacggc ctgacctcat caatctatac ccaaaatctg      1260
   aacgtcgcga tgaaagccat taaagggctg aagtttggtg aaacttacat caaccgtgaa      1320
   aacttcgaag ctatgcaagg cttccacgcc ggatggcgta aatccggtat tggcggcgca      1380
   gatggtaaac atggcttgca tgaatatctg cagacccagg tggtttattt acagtcttaa      1440
<210> 4
   <211> 1164
   <212> DNA
   <213> Escherichia coli
<400> 4
   atgaacaact ttaatctgca caccccaacc cgcattctgt ttggtaaagg cgcaatcgct        60
   ggtttacgcg aacaaattcc tcacgatgct cgcgtattga ttacctacgg cggcggcagc       120
   gtgaaaaaaa ccggcgttct cgatcaagtt ctggatgccc tgaaaggcat ggacgtgctg       180
   gaatttggcg gtattgagcc aaacccggct tatgaaacgc tgatgaacgc cgtgaaactg       240
   gttcgcgaac agaaagtgac tttcctgctg gcggttggcg gcggttctgt actggacggc       300
   accaaattta tcgccgcagc ggctaactat ccggaaaata tcgatccgtg gcacattctg       360
   caaacgggcg gtaaagagat taaaagcgcc atcccgatgg gctgtgtgct gacgctgcca       420
   gcaaccggtt cagaatccaa cgcaggcgcg gtgatctccc gtaaaaccac aggcgacaag       480
   caggcgttcc attctgccca tgttcagccg gtatttgccg tgctcgatcc ggtttatacc       540
   tacaccctgc cgccgcgtca ggtggctaac ggcgtagtgg acgcctttgt acacaccgtg       600
   gaacagtatg ttaccaaacc ggttgatgcc aaaattcagg accgtttcgc agaaggcatt       660
   ttgctgacgc taatcgaaga tggtccgaaa gccctgaaag agccagaaaa ctacgatgtg       720
   cgcgccaacg tcatgtgggc ggcgactcag gcgctgaacg gtttgattgg cgctggcgta       780
   ccgcaggact gggcaacgca tatgctgggc cacgaactga ctgcgatgca cggtctggat       840
   cacgcgcaaa cactggctat cgtcctgcct gcactgtgga atgaaaaacg cgataccaag       900
   cgcgctaagc tgctgcaata tgctgaacgc gtctggaaca tcactgaagg ttccgatgat       960
   gagcgtattg acgccgcgat tgccgcaacc cgcaatttct ttgagcaatt aggcgtgccg      1020
   acccacctct ccgactacgg tctggacggc agctccatcc cggctttgct gaaaaaactg      1080
   gaagagcacg gcatgaccca actgggcgaa aatcatgaca ttacgttgga tgtcagccgc      1140
   cgtatatacg aagccgcccg ctaa                                             1164
<210> 5
   <211> 1419
   <212> DNA
   <213> Escherichia coli
<400> 5
   atgaaacaag aagttatcct ggtactcgac tgtggcgcga ccaatgtcag ggccatcgcg 60
   gttaatcggc agggcaaaat tgttgcccgc gcctcaacgc ctaatgccag cgatatcgcg 120
   atggaaaaca acacctggca ccagtggtct ttagacgcca ttttgcaacg ctttgctgat 180
   tgctgtcggc aaatcaatag tgaactgact gaatgccaca tccgcggtat cgccgtcacc 240
   acctttggtg tggatggcgc tctggtagat aagcaaggca atctgctcta tccgattatt 300
   agctggaaat gtccgcgaac agcagcggtt atggacaata ttgaacggtt aatctccgca 360
   cagcggttgc aggctatttc tggcgtcgga gcctttagtt tcaatacgtt atataagttg 420
   gtgtggttga aagaaaatca tccacaactg ctggaacgcg cgcacgcctg gctctttatt 480
   tcgtcgctga ttaaccaccg tttaaccggc gaattcacta ctgatatcac gatggccgga 540
   accagccaga tgctggatat ccagcaacgc gatttcagtc cgcaaatttt acaagccacc 600
   ggtattccac gccgactctt ccctcgtctg gtggaagcgg gtgaacagat tggtacgcta 660
   cagaacagcg ccgcagcaat gctcggctta cccgttggca taccggtgat ttccgcaggt 720
   cacgataccc agttcgccct ttttggcgct ggtgctgaac aaaatgaacc cgtgctctct 780
   tccggtacat gggaaatttt aatggttcgc agcgcccagg ttgatacttc gctgttaagt 840
   cagtacgccg gttccacctg cgaactggat agccaggcag ggttgtataa cccaggtatg 900
   caatggctgg catccggcgt gctggaatgg gtgagaaaac tgttctggac ggctgaaaca 960
   ccctggcaaa tgttgattga agaagctcgt ctgatcgcgc ctggcgcgga tggcgtaaaa 1020
   atgcagtgtg atttattgtc gtgtcagaac gctggctggc aaggagtgac gcttaatacc 1080
   acgcgggggc atttctatcg cgcggcgctg gaagggttaa ctgcgcaatt acagcgcaat 1140
   ctacagatgc tggaaaaaat cgggcacttt aaggcctctg aattattgtt agtcggtgga 1200
   ggaagtcgca acacattgtg gaatcagatt aaagccaata tgcttgatat tccggtaaaa 1260
   gttctcgacg acgccgaaac gaccgtcgca ggagctgcgc tgttcggttg gtatggcgta 1320
   ggggaattta acagcccgga agaagcccgc gcacagattc attatcagta ccgttatttc 1380
   tacccgcaaa ctgaacctga atttatagag gaagtgtga 1419
<210> 6
   <211> 1470
   <212> DNA
   <213> Escherichia coli
<400> 6
   atgacctttc gcaattgtgt cgccgtcgat ctcggcgcat ccagtgggcg cgtgatgctg      60
   gcgcgttacg agcgtgaatg ccgcagcctg acgctgcgcg aaatccatcg ttttaacaat     120
   gggctgcata gtcagaacgg ctatgtcacc tgggatgtgg atagccttga aagtgccatt     180
   cgccttggat taaacaaggt gtgcgaggaa gggattcgta tcgatagcat tgggattgat     240
   acctggggcg tggactttgt gctgctcgac caacagggtc agcgtgtggg cctgcccgtt     300
   gcttatcgcg atagccgcac caatggccta atggcgcagg cacaacaaca actcggcaaa     360
   cgcgatattt atcaacgtag cggcatccag tttctgccct tcaatacgct ttatcagttg     420
   cgtgcgctga cggagcaaca acctgaactt attccacaca ttgctcacgc tctgctgatg     480
   ccggattact tcagttatcg cctgaccggc aagatgaact gggaatatac caacgccacg     540
   accacgcaac tggtcaatat caatagcgac gactgggacg agtcgctact ggcgtggagc     600
   ggggccaaca aagcctggtt tggtcgcccg acgcatccgg gtaatgtcat aggtcactgg     660
   atttgcccgc agggtaatga gattccagtg gtcgccgttg ccagccatga taccgccagc     720
   gcggttatcg cctcgccgtt aaacggctca cgtgctgctt atctctcttc tggcacctgg     780
   tcattgatgg gcttcgaaag ccagacgcca tttaccaatg acacggcact ggcagccaac     840
   atcaccaatg aaggcggggc ggaaggtcgc tatcgggtgc tgaaaaatat tatgggctta     900
   tggctgcttc agcgagtgct tcaggagcag caaatcaacg atcttccggc gcttatctcc     960
   gcgacacagg cacttccggc ttgccgcttc attatcaatc ccaatgacga tcgctttatt    1020
   aatcctgaga cgatgtgcag cgaaattcag gctgcgtgtc gggaaacggc gcaaccgatc    1080
   ccggaaagtg atgctgaact ggcgcgctgc attttcgaca gtctggcgct gctgtatgcc    1140
   gatgtgttgc atgagctggc gcagctgcgc ggtgaagatt tctcgcaact gcatattgtc    1200
   ggcggaggct gccagaacac gctgctcaac cagctatgcg ccgatgcctg cggtattcgg    1260
   gtgatcgccg ggcctgttga agcctcgacg ctcggcaata tcggcatcca gttaatgacg    1320
   ctggatgaac tcaacaatgt ggatgatttc cgtcaggtcg tcagcaccac cgcgaatctg    1380
   accaccttta cccctaatcc tgacagtgaa attgcccact atgtggcgca gattcactct    1440
   acacgacaga caaaggagct ttgcgcatga                                      1470
<210> 7
   <211> 1500
   <212> DNA
   <213> Escherichia coli
<400> 7
   atgagcatct tgtacgaaga gcgtcttgat ggcgctttac ccgatgtcga ccgcacatcg 60
   gtactgatgg cactgcgtga gcatgtccct ggacttgaga tcctgcatac cgatgaggag 120
   atcattcctt acgagtgtga cgggttgagc gcgtatcgca cgcgtccatt actggttgtt 180
   ctgcctaagc aaatggaaca ggtgacagcg attctggctg tctgccatcg cctgcgtgta 240
   ccggtggtga cccgtggtgc aggcaccggg ctttctggtg gcgcgctgcc gctggaaaaa 300
   ggtgtgttgt tggtgatggc gcgctttaaa gagatcctcg acattaaccc cgttggtcgc 360
   cgcgcgcgcg tgcagccagg cgtgcgtaac ctggcgatct cccaggccgt tgcaccgcat 420
   aatctctact acgcaccgga cccttcctca caaatcgcct gttccattgg cggcaatgtg 480
   gctgaaaatg ccggcggcgt ccactgcctg aaatatggtc tgaccgtaca taacctgctg 540
   aaaattgaag tgcaaacgct ggacggcgag gcactgacgc ttggatcgga cgcgctggat 600
   tcacctggtt ttgacctgct ggcgctgttc accggatcgg aaggtatgct cggcgtgacc 660
   accgaagtga cggtaaaact gctgccgaag ccgcccgtgg cgcgggttct gttagccagc 720
   tttgactcgg tagaaaaagc cggacttgcg gttggtgaca tcatcgccaa tggcattatc 780
   cccggcgggc tggagatgat ggataacctg tcgatccgcg cggcggaaga ttttattcat 840
   gccggttatc ccgtcgacgc cgaagcgatt ttgttatgcg agctggacgg cgtggagtct 900
   gacgtacagg aagactgcga gcgggttaac gacatcttgt tgaaagcggg cgcgactgac 960
   gtccgtctgg cacaggacga agcagagcgc gtacgtttct gggccggtcg caaaaatgcg 1020
   ttcccggcgg taggacgtat ctccccggat tactactgca tggatggcac catcccgcgt 1080
   cgcgccctgc ctggcgtact ggaaggcatt gcccgtttat cgcagcaata tgatttacgt 1140
   gttgccaacg tctttcatgc cggagatggc aacatgcacc cgttaatcct tttcgatgcc 1200
   aacgaacccg gtgaatttgc ccgcgcggaa gagctgggcg ggaagatcct cgaactctgc 1260
   gttgaagttg gcggcagcat cagtggcgaa catggcatcg ggcgagaaaa aatcaatcaa 1320
   atgtgcgccc agttcaacag cgatgaaatc acgaccttcc atgcggtcaa ggcggcgttt 1380
   gaccccgatg gtttgctgaa ccctgggaaa aacattccca cgctacaccg ctgtgctgaa 1440
   tttggtgcca tgcatgtgca tcacggtcat ttacctttcc ctgaactgga gcgtttctga 1500
<210> 8
   <211> 861
   <212> DNA
   <213> Escherichia coli
<400> 8
   atgagcatta tctccactaa atatctgtta caggacgccc aggccaatgg ctacgcggtg 60
   cctgctttta acattcataa cgccgagacg atccaagcga tcctcgaagt gtgcagtgaa 120
   atgcgatcgc cggtgatcct cgccggaacg ccggggacct ttaaacacat cgcgctggaa   180
   gagatctacg ccctgtgtag cgcctattcc acaacctaca acatgccact ggcgctgcat   240
   ctcgaccacc acgaatcgct ggatgatatt cgccgtaaag tccacgcagg tgtgcgcagt   300
   gcgatgatcg acggcagcca cttcccgttt gccgagaacg tgaagctggt gaaatcggtt   360
   gttgacttct gccactcaca agattgcagc gtggaagcag aactgggccg cctgggcggt   420
   gttgaagatg acatgagcgt tgacgccgaa agtgcattcc tgaccgatcc acaagaagct   480
   aaacgctttg tcgaactgac tggcgtcgac agcctggcgg tagcgattgg tacggcgcac   540
   ggcttataca gcaaaacgcc gaagattgat ttccagcggc tggcggaaat tcgtgaagtg   600
   gtggatgttc ctctggtgct gcatggtgcc agcgatgttc cggatgaatt tgtccgtcgc   660
   actattgaac ttggcgtcac aaaagtgaac gttgccacag aattaaaaat agccttcgct   720
   ggcgcggtta aagcctggtt tgcggaaaat ccgcagggta atgatcctcg ttattatatg   780
   cgcgtcggaa tggatgcgat gaaagaagtt gtcagaaata aaattaatgt ctgtggttca   840
   gcgaatcgaa tttcagcata a                                             861
<210> 9
   <211> 1053
   <212> DNA
   <213> Escherichia coli
<400> 9
   atgacagata ttgcgcagtt gcttggcaaa gacgccgaca accttttaca gcaccgttgt      60
   atgacaattc cttctgacca gctttatctc cccggacatg actacgtaga ccgcgtaatg     120
   attgacaata atcgcccgcc agcggtgtta cgtaatatgc agacgttgta caacaccggg     180
   cgtctggctg gcacaggata tctttctatt ctgccggttg accagggcgt tgagcactct     240
   gccggagctt catttgctgc taacccgctc tactttgacc cgaaaaacat tgttgaactg     300
   gcgatcgaag cgggctgtaa ctgtgtggcg tcaacttacg gcgtgctggc gtcggtatcg     360
   cggcgttatg cgcatcgcat tccattcctc gtcaaactta atcacaacga gacgctaagt     420
   tacccgaata cctacgatca aacgctgtat gccagcgtgg agcaggcgtt caacatgggc     480
   gcggttgcgg ttggtgcgac tatctatttt ggctcggaag agtcacgtcg ccagattgaa     540
   gaaatttctg cggcttttga acgtgcgcac gagctgggta tggtgacagt gctgtgggcc     600
   tatttgcgta actccgcctt taagaaagat ggcgttgatt accatgtttc cgccgacctg     660
   accggtcagg caaaccatct ggcggcaacc atcggtgcag atatcgtcaa acaaaaaatg     720
   gcggaaaata acggcggcta taaagcaatt aattacggtt acaccgacga tcgtgtttac     780
   agcaaattga ccagcgaaaa cccgattgat ctggtgcgtt atcagttagc taactgctat     840
   atgggtcggg ctgggttgat aaactccggc ggtgctgcgg gcggtgaaac tgacctcagc     900
   gatgcagtgc gtactgcggt tatcaacaaa cgcgcaggcg gaatggggct gattcttgga     960
   cgtaaagcgt tcaagaaatc gatggctgac ggcgtgaaac tgattaacgc cgtgcaggac    1020
   gtttatctcg atagcaaaat tactatcgcc tga                                1053
<210> 10
   <211> 939
   <212> DNA
   <213> Escherichia coli
<400> 10
   atggatatca tcttttatca cccaacgttc gatacccaat ggtggattga ggcactgcgc 60
   aaagctattc ctcaggcaag agtcagagca tggaaaagcg gagataatga ctctgctgat 120
   tatgctttag tctggcatcc tcctgttgaa atgctggcag ggcgcgatct taaagcggtg 180
   ttcgcactcg gggccggtgt tgattctatt ttgagcaagc tacaggcaca ccctgaaatg 240
   ctgaaccctt ctgttccact ttttcgcctg gaagataccg gtatgggcga gcaaatgcag 300
   gaatatgctg tcagtcaggt gctgcattgg tttcgacgtt ttgacgatta tcgcatccag 360
   caaaatagtt cgcattggca accgctgcct gaatatcatc gggaagattt taccatcggc 420
   attttgggcg caggcgtact gggcagtaaa gttgctcaga gtctgcaaac ctggcgcttt 480
   ccgctgcgtt gctggagtcg aacccgtaaa tcgtggcctg gcgtgcaaag ctttgccgga 540
   cgggaagaac tgtctgcatt tctgagccaa tgtcgggtat tgattaattt gttaccgaat 600
   acccctgaaa ccgtcggcat tattaatcaa caattactcg aaaaattacc ggatggcgcg 660
   tatctcctca acctggcgcg tggtgttcat gttgtggaag atgacctgct cgcggcgctg 720
   gatagcggca aagttaaagg cgcaatgttg gatgttttta atcgtgaacc cttaccgcct 780
   gaaagtccgc tctggcaaca tccacgcgtg acgataacac cacatgtcgc cgcgattacc 840
   cgtcccgctg aagctgtgga gtacatttct cgcaccattg cccagctcga aaaaggggag 900
   agggtctgcg ggcaagtcga ccgcgcacgc ggctactaa 939
<210> 11
   <211> 1305
   <212> DNA
   <213> Escherichia coli
<400> 11
   atgaaaaccc gtacacaaca aattgaagaa ttacagaaag agtggactca accgcgttgg 60
   gaaggcatta ctcgcccata cagtgcggaa gatgtggtga aattacgcgg ttcagtcaat 120
   cctgaatgca cgctggcgca actgggcgca gcgaaaatgt ggcgtctgct gcacggtgag 180
   tcgaaaaaag gctacatcaa cagcctcggc gcactgactg gcggtcaggc gctgcaacag 240
   gcgaaagcgg gtattgaagc agtctatctg tcgggatggc aggtagcggc ggacgctaac 300
   ctggcggcca gcatgtatcc ggatcagtcg ctctatccgg caaactcggt gccagctgtg 360
   gtggagcgga tcaacaacac cttccgtcgt gccgatcaga tccaatggtc cgcgggcatt 420
   gagccgggcg atccgcgcta tgtcgattac ttcctgccga tcgttgccga tgcggaagcc 480
   ggttttggcg gtgtcctgaa tgcctttgaa ctgatgaaag cgatgattga agccggtgca 540
   gcggcagttc acttcgaaga tcagctggcg tcagtgaaga aatgcggtca catgggcggc 600
   aaagttttag tgccaactca ggaagctatt cagaaactgg tcgcggcgcg tctggcagct 660
   gacgtgacgg gcgttccaac cctgctggtt gcccgtaccg atgctgatgc ggcggatctg 720
   atcacctccg attgcgaccc gtatgacagc gaatttatta ccggcgagcg taccagtgaa 780
   ggcttcttcc gtactcatgc gggcattgag caagcgatca gccgtggcct ggcgtatgcg 840
   ccatatgctg acctggtctg gtgtgaaacc tccacgccgg atctggaact ggcgcgtcgc 900
   tttgcacaag ctatccacgc gaaatatccg ggcaaactgc tggcttataa ctgctcgccg 960
   tcgttcaact ggcagaaaaa cctcgacgac aaaactattg ccagcttcca gcagcagctg 1020
   tcggatatgg gctacaagtt ccagttcatc accctggcag gtatccacag catgtggttc 1080
   aacatgtttg acctggcaaa cgcctatgcc cagggcgagg gtatgaagca ctacgttgag 1140
   aaagtgcagc agccggaatt tgccgccgcg aaagatggct ataccttcgt atctcaccag 1200
   caggaagtgg gtacaggtta cttcgataaa gtgacgacta ttattcaggg cggcacgtct 1260
   tcagtcaccg cgctgaccgg ctccactgaa gaatcgcagt tctaa 1305
<210> 12
   <211> 323
   <212> DNA
   <213> Saccharomyces cerevisiae
<400> 12
   tgcagatcaa aggcaaagac agaaaccgta gtaaaggttg acttttcaca acagtgtctc 60
   cattttttat attgtattat taaagctatt tagttatttg gatactgttt tttttccaga 120
   agttttcttt ttagtaaagt acaatccagt aaaaatgaag gatgaacaat cggtgtatgc 180
   agattcaaca ccaataaatg caatgtttat ttctttggaa cgtttgtgtt gttcgaaatc 240
   caggataatc cttcaacaag accctgtccg gataaggcgt tactaccgat gacacaccaa 300
   gaaccacaca tctatttcct tat 323
<210> 13
   <211> 311
   <212> DNA
   <213> Saccharomyces cerevisiae
<400> 13
   tattctgatc gtagatcatc agatttgata tgatattatt tgtgaaaaaa tgaaataaaa 60
   ctttatacaa cttaaataca acttttttta taaacgatta agcaaaaaaa tagtttcaaa 120
   cttttaacaa tattccaaac actcagtcct tttccttctt atattatagg tgtacgtatt 180
   atagaaaaat ttcaatgatt actttttctt tctttttcct tgtaccagca catggccgag 240
   cttgaatgtt aaacccttcg agagaatcac accattcaag tataaagcca ataaagaata 300
   taaaaaatta t 311
<210> 14
   <211> 514
   <212> DNA
   <213> Saccharomyces cerevisiae
<400> 14
   atgggtcatt acgtaaataa tgataggaat gggattcttc tatttttcct ttttccattc 60
   tagcagccgt cgggaaaacg tggcatcctc tctttcgggc tcaattggag tcacgctgcc 120
   gtgagcatcc tctctttcca tatctaacaa ctgagcacgt aaccaatgga aaagcatgag 180
   cttagcgttg ctccaaaaaa gtattggatg gttaatacca tttgtctgtt ctcttctgac 240
   tttgactcct caaaaaaaaa aaatctacaa tcaacagatc gcttcaatta cgccctcaca 300
   aaaacttttt tccttcttct tcgcccacgt taaattttat ccctcatgtt gtctaacgga 360
   tttctgcact tgatttatta taaaaagaca aagacataat acttctctat caatttcagt 420
   tattgttctt ccttgcgtta ttcttctgtt cttctttttc ttttgtcata tataaccata 480
   accaagtaat acatattcaa aaagaaacat aaat 514
<210> 15
   <211> 391
   <212> DNA
   <213> Saccharomyces cerevisiae
<400> 15
   tatttaaact gtgaggacct taatacattc agacacttct gcggtatcac cctacttatt 60
   cccttcgaga ttatatctag gaacccatca ggttggtgga agattacccg ttctaagact 120
   tttcagcttc ctctattgat gttacacctg gacacccctt ttctggcatc cagtttttaa 180
   tcttcagtgg catgtgagat tctccgaaat taattaaagc aatcacacaa ttctctcgga 240
   taccacctcg gttgaaactg acaggtggtt tgttacgcat gctaatgcaa aggagcctat 300
   atacctttgg ctcggctgct gtaacaggga atataaaggg cagcataatt taggagttta 360
   gtgaacttgc aacatttact attttccctt c 391
<210> 16
   <211> 23
   <212> DNA
   <213> Artificial Sequence
<220>
   <223> Amorce/Primer
<400> 16
   catatgacct ttcgcaattg tgt 23
<210> 17
   <211> 26
   <212> DNA
   <213> Artificial Sequence
<220>
   <223> Amorce/Primer
<400> 17
   ggatcctcat gcgcaaagct cctttg 26
<210> 18
   <211> 52
   <212> DNA
   <213> Artificial Sequence
<220>
   <223> AMORCE/PRIMER
<400> 18
   ccatggatgc accatcacca tcaccatatg ttatccggct atattgcagg ag 52
<210> 19
   <211> 38
   <212> DNA
   <213> Artificial Sequence
<220>
   <223> AMORCE/PRIMER
<400> 19
   ggatccgtcg acattaacgg cgaaattgtt tcagcatt 38
<210> 20
   <211> 40
   <212> DNA
   <213> Artificial Sequence
<220>
   <223> AMORCE/PRIMER
<400> 20
   ttggatccag gaggattcat atggcacatc gctttccggc 40
<210> 21
   <211> 28
   <212> DNA
   <213> Artificial Sequence
<220>
   <223> AMORCE/PRIMER
<400> 21
   ttaagctttt aatacgtgta acaggccg 28
<210> 22
   <211> 29
   <212> DNA
   <213> Artificial Sequence
<220>
   <223> AMORCE/PRIMER
<400> 22
   catatgacag atattgcgca gttgcttgg 29
<210> 23
   <211> 29
   <212> DNA
   <213> Artificial Sequence
<220>
   <223> AMORCE/PRIMER
<400> 23
   ggatcctcag gcgatagtaa ttttgctat 29
<210> 24
   <211> 29
   <212> DNA
   <213> Artificial Sequence
<220>
   <223> AMORCE/PRIMER
<400> 24
   catatgagca ttatctccac taaatatct 29
<210> 25
   <211> 29
   <212> DNA
   <213> Artificial Sequence
<220>
   <223> AMORCE/PRIMER
<400> 25
   ggatccttat gctgaaattc gattcgctg 29
<210> 26
   <211> 56
   <212> DNA
   <213> Artificial Sequence
<220>
   <223> AMORCE/PRIMER
<400> 26
   cctctagagt cgacctgcag aggaggattc atatgaacaa ctttaatctg cacacc 56
<210> 27
   <211> 34
   <212> DNA
   <213> Artificial Sequence
<220>
   <223> AMORCE/PRIMER
<400> 27
   gccaaaacag aagcttttag cgggcggctt cgta 34
<210> 28
   <211> 42
   <212> DNA
   <213> Artificial Sequence
<220>
   <223> AMORCE / PRIMER
<400> 28
   cggtacccgg ggatcaggag gcacacgatg gaagaagcag at 42
<210> 29
   <211> 25
   <212> DNA
   <213> Artificial Sequence
<220>
   <223> AMORCE / PRIMER
<400> 29
   tcacacgatg ccatcaaagc cctgc 25
<210> 30
   <211> 54
   <212> DNA
   <213> Artificial Sequence
<220>
   <223> AMORCE / PRIMER
<400> 30
   cctctagagt cgacctgcag aggaggattc atatggcaca tcgctttccg gctc 54
<210> 31
   <211> 36
   <212> DNA
   <213> Artificial Sequence
<220>
   <223> AMORCE / PRIMER
<400> 31
   gccaaaacag aagcttttaa tacgtgtaac aggccg 36
<210> 32
   <211> 44
   <212> DNA
   <213> Artificial Sequence
<220>
   <223> AMORCE / PRIMER
<400> 32
   ttggtaccaa gaaggagaat tcatatgtca gtacccgttc aaca 44
<210> 33
   <211> 28
   <212> DNA
   <213> Artificial Sequence
<220>
   <223> AMORCE / PRIMER
<400> 33
   ttaagctttt aagactgtaa ataaacca 28
<210> 34
   <211> 55
   <212> DNA
   <213> Artificial Sequence
<220>
   <223> AMORCE / PRIMER
<400> 34
   gagctcggta cccggggatc caggaggcac acgatggata tcatctttta tcacc 55
<210> 35
   <211> 49
   <212> DNA
   <213> Artificial Sequence
<220>
   <223> AMORCE / PRIMER
<400> 35
   ctcatccgcc aaaacagaag cttttagtag ccgcgtgcgc ggtcgactt 49
<210> 36
   <211> 52
   <212> DNA
   <213> Artificial Sequence
<220>
   <223> AMORCE / PRIMER
<400> 36
   gcacgcggct actaaaggag gaaccgtatg aaaacccgta cacaacaaat tg 52
<210> 37
   <211> 47
   <212> DNA
   <213> Artificial Sequence
<220>
   <223> AMORCE / PRIMER
<400> 37
   ctcatccgcc aaaacagaag cttttagaac tgcgattctt cagtgga 47
<210> 38
   <211> 42
   <212> DNA
   <213> Artificial Sequence
<220>
   <223> AMORCE / PRIMER
<400> 38
   tcatacggtt cctcctttag tagccgcgtg cgcggtcgac tt 42
<210> 39
   <211> 65
   <212> DNA
   <213> Artificial Sequence
<220>
   <223> AMORCE / PRIMER
<400> 39
   ggaacaaaag ctggagctcg taggaacaat ttcgggcctt aaactgtgag gaccttaata 60
   cattc 65
<210> 40
   <211> 42
   <212> DNA
   <213> Artificial Sequence
<220>
   <223> AMORCE / PRIMER
<400> 40
   atctgcttct cttccatttt tagtttatgt atgtgttttt tg 42
<210> 41
   <211> 44
   <212> DNA
   <213> Artificial Sequence
<220>
   <223> AMORCE / PRIMER
<400> 41
   cacatacata aactaaaaat ggaagagaag cagatcctgt gcgt 44
<210> 42
   <211> 42
   <212> DNA
   <213> Artificial Sequence
<220>
   <223> AMORCE / PRIMER
<400> 42
   tgtctttgcc tttgatctgc tcacacgatg ccatcaaagc cc 42
<210> 43
   <211> 45
   <212> DNA
   <213> Artificial Sequence
<220>
   <223> AMORCE / PRIMER
<400> 43
   gatggcatcg tgtgagcaga tcaaaggcaa agacagaaac cgtag 45
<210> 44
   <211> 56
   <212> DNA
   <213> Artificial Sequence
<220>
   <223> AMORCE / PRIMER
<400> 44
   attcctatca ttatttacgt aatgacccat cttggtgtgt catcggtagt aacgcc 56
<210> 45
   <211> 49
   <212> DNA
   <213> Artificial Sequence
<220>
   <223> AMORCE / PRIMER
<400> 45
   ccgatgacac accaagatgg gtcattacgt aaataatgat aggaatggg 49
<210> 46
   <211> 55
   <212> DNA
   <213> Artificial Sequence
<220>
   <223> AMORCE / PRIMER
<400> 46
   tgcgtcagag ccggaaagcg atgtgccatt ttgaatatgt attacttggt tatgg 55
<210> 47
   <211> 44
   <212> DNA
   <213> Artificial Sequence
<220>
   <223> AMORCE / PRIMER
<400> 47
   ccaagtaata catattcaaa atggcacatc gctttccggc tctg 44
<210> 48
   <211> 46
   <212> DNA
   <213> Artificial Sequence
<220>
   <223> AMORCE / PRIMER
<400> 48
   ctgatgatct acgatcagaa tttaatacgt gtaacaggcc gtaaac 46
<210> 49
   <211> 45
   <212> DNA
   <213> Artificial Sequence
<220>
   <223> AMORCE / PRIMER
<400> 49
   gttacacgta ttaaattctg atcgtagatc atcagatttg atatg 45
<210> 50
   <211> 60
   <212> DNA
   <213> Artificial Sequence
<220>
   <223> AMORCE / PRIMER
<400> 50
   cgcgcgtaat acgactcact atagggcgaa ttggatattc tttattggct ttatacttga 60
<210> 51
   <211> 1104
   <212> DNA
   <213> Escherichia coli
<400> 51
   atggaccgca ttattcaatc accgggtaaa tacatccagg gcgctgatgt gattaatcgt      60
   ctgggcgaat acctgaagcc gctggcagaa cgctggttag tggtgggtga caaatttgtt     120
   ttaggttttg ctcaatccac tgtcgagaaa agctttaaag atgctggact ggtagtagaa     180
   attgcgccgt ttggcggtga atgttcgcaa aatgagatcg accgtctgcg tggcatcgcg     240
   gagactgcgc agtgtggcgc aattctcggt atcggtggcg gaaaaaccct cgatactgcc     300
   aaagcactgg cacatttcat gggtgttccg gtagcgatcg caccgactat cgcctctacc     360
   gatgcaccgt gcagcgcatt gtctgttatc tacaccgatg agggtgagtt tgaccgctat     420
   ctgctgttgc caaataaccc gaatatggtc attgtcgaca ccaaaatcgt cgctggcgca     480
   cctgcacgtc tgttagcggc gggtatcggc gatgcgctgg caacctggtt tgaagcgcgt     540
   gcctgctctc gtagcggcgc gaccaccatg gcgggcggca agtgcaccca ggctgcgctg     600
   gcactggctg aactgtgcta caacaccctg ctggaagaag gcgaaaaagc gatgcttgct     660
   gccgaacagc atgtagtgac tccggcgctg gagcgcgtga ttgaagcgaa cacctatttg     720
   agcggtgttg gttttgaaag tggtggtctg gctgcggcgc acgcagtgca taacggcctg     780
   accgctatcc cggacgcgca tcactattat cacggtgaaa aagtggcatt cggtacgctg     840
   acgcagctgg ttctggaaaa tgcgccggtg gaggaaatcg aaaccgtagc tgcccttagc     900
   catgcggtag gtttgccaat aactctcgct caactggata ttaaagaaga tgtcccggcg     960
   aaaatgcgaa ttgtggcaga agcggcatgt gcagaaggtg aaaccattca caacatgcct    1020
   ggcggcgcga cgccagatca ggtttacgcc gctctgctgg tagccgacca gtacggtcag    1080
   cgtttcctgc aagagtggga ataa                                            1104
<210> 52
   <211> 1149
   <212> DNA
   <213> Escherichia coli
<400> 52
   atggctaaca gaatgattct gaacgaaacg gcatggtttg gtcggggtgc tgttggggct      60
   ttaaccgatg aggtgaaacg ccgtggttat cagaaggcgc tgatcgtcac cgataaaacg     120
   ctggtgcaat gcggcgtggt ggcgaaagtg accgataaga tggatgctgc agggctggca     180
   tgggcgattt acgacggcgt agtgcccaac ccaacaatta ctgtcgtcaa agaagggctc     240
   ggtgtattcc agaatagcgg cgcggattac ctgatcgcta ttggtggtgg ttctccacag     300
   gatacttgta aagcgattgg cattatcagc aacaacccgg agtttgccga tgtgcgtagc     360
   ctggaagggc tttccccgac caataaaccc agtgtaccga ttctggcaat tcctaccaca     420
   gcaggtactg cggcagaagt gaccattaac tacgtgatca ctgacgaaga gaaacggcgc     480
   aagtttgttt gcgttgatcc gcatgatatc ccgcaggtgg cgtttattga cgctgacatg     540
   atggatggta tgcctccagc gctgaaagct gcgacgggtg tcgatgcgct cactcatgct     600
   attgaggggt atattacccg tggcgcgtgg gcgctaaccg atgcactgca cattaaagcg     660
   attgaaatca ttgctggggc gctgcgagga tcggttgctg gtgataagga tgccggagaa     720
   gaaatggcgc tcgggcagta tgttgcgggt atgggcttct cgaatgttgg gttagggttg     780
   gtgcatggta tggcgcatcc actgggcgcg ttttataaca ctccacacgg tgttgcgaac     840
   gccatcctgt taccgcatgt catgcgttat aacgctgact ttaccggtga gaagtaccgc     900
   gatatcgcgc gcgttatggg cgtgaaagtg gaaggtatga gcctggaaga ggcgcgtaat     960
   gccgctgttg aagcggtgtt tgctctcaac cgtgatgtcg gtattccgcc acatttgcgt    1020
   gatgttggtg tacgcaagga agacattccg gcactggcgc aggcggcact ggatgatgtt    1080
   tgtaccggtg gcaacccgcg tgaagcaacg cttgaggata ttgtagagct ttaccatacc    1140
   gcctggtaa                                                            1149
<210> 53
   <211> 1455
   <212> DNA
   <213> Escherichia coli
<400> 53
   atgtatatcg ggatagatct tggcacctcg ggcgtaaaag ttattttgct caacgagcag      60
   ggtgaggtgg ttgctgcgca aacggaaaag ctgaccgttt cgcgcccgca tccactctgg     120
   tcggaacaag acccggaaca gtggtggcag gcaactgatc gcgcaatgaa agctctgggc     180
   gatcagcatt ctctgcagga cgttaaagca ttgggtattg ccggccagat gcacggagca     240
   accttgctgg atgctcagca acgggtgtta cgccctgcca ttttgtggaa cgacgggcgc     300
   tgtgcgcaag agtgcacttt gctggaagcg cgagttccgc aatcgcgggt gattaccggc     360
   aacctgatga tgcccggatt tactgcgcct aaattgctat gggttcagcg gcatgagccg     420
   gagatattcc gtcaaatcga caaagtatta ttaccgaaag attacttgcg tctgcgtatg     480
   acgggggagt ttgccagcga tatgtctgac gcagctggca ccatgtggct ggatgtcgca     540
   aagcgtgact ggagtgacgt catgctgcag gcttgcgact tatctcgtga ccagatgccc     600
   gcattatacg aaggcagcga aattactggt gctttgttac ctgaagttgc gaaagcgtgg     660
   ggtatggcga cggtgccagt tgtcgcaggc ggtggcgaca atgcagctgg tgcagttggt     720
   gtgggaatgg ttgatgctaa tcaggcaatg ttatcgctgg ggacgtcggg ggtctatttt     780
   gctgtcagcg aagggttctt aagcaagcca gaaagcgccg tacatagctt ttgccatgcg     840
   ctaccgcaac gttggcattt aatgtctgtg atgctgagtg cagcgtcgtg tctggattgg     900
   gccgcgaaat taaccggcct gagcaatgtc ccagctttaa tcgctgcagc tcaacaggct     960
   gatgaaagtg ccgagccagt ttggtttctg ccttatcttt ccggcgagcg tacgccacac    1020
   aataatcccc aggcgaaggg ggttttcttt ggtttgactc atcaacatgg ccccaatgaa    1080
   ctggcgcgag cagtgctgga aggcgtgggt tatgcgctgg cagatggcat ggatgtcgtg    1140
   catgcctgcg gtattaaacc gcaaagtgtt acgttgattg ggggcggggc gcgtagtgag     1200
   tactggcgtc agatgctggc ggatatcagc ggtcagcagc tcgattaccg tacggggggg    1260
   gatgtggggc cagcactggg cgcagcaagg ctggcgcaga tcgcggcgaa tccagagaaa    1320
   tcgctcattg aattgttgcc gcaactaccg ttagaacagt cgcatctacc agatgcgcag    1380
   cgttatgccg cttatcagcc acgacgagaa acgttccgtc gcctctatca gcaacttctg    1440
   ccattaatgg cgtaa                                                     1455
<210> 54
   <211> 45
   <212> DNA
   <213> Artificial Sequence
<220>
   <223> amorce/primer
<400> 54
   cctctagagt cgacctgcag aggaggattc atatggaccg catta 45
<210> 55
   <211> 35
   <212> DNA
   <213> artificial sequence
<220>
   <223> amorce/primer
<400> 55
   gccaaaacag aagcttttat tcccactctt gcagg 35
<210> 56
   <211> 40
   <212> DNA
   <213> artificial sequence
<220>
   <223> amorce/primer
<400> 56
   ttggatccag gaggattcat atggctaaca gaatgattct 40
<210> 57
   <211> 27
   <212> DNA
   <213> artificial sequence
<220>
   <223> amorce/primer
<400> 57
   ttaagctttt accaggcggt atggtaa 27
<210> 58
   <211> 52
   <212> DNA
   <213> artiticial sequence
<400> 58
   gatggcatcg tgtgaaggag gaaccgtatg acagatattg cgcagttgct tg 52
<210> 59
   <211> 44
   <212> DNA
   <213> artificial sequence
<220>
   <223> amorce/primer
<400> 59
   atgcctgcag gtcgacaatt gtcaggcgat agtaattttg ctat 44
<210> 60
   <211> 68
   <212> DNA
   <213> artificial sequence
<220>
   <223> amorce/primer
<400> 60
   cggtacccgg ggatcggaaa tactataaac ggttaaataa acaaaccata atggaagaga 60
agcagatc 68
<210> 61
   <211> 70
   <212> DNA
   <213> artificial sequence
<220>
   <223> amorce/primer
<400> 61
   gatggcatcg tgtgaacagc ttttaattat acactttaag gaggacagac atgacagata 60
   ttgcgcagtt 70
<210> 62
   <211> 35
   <212> DNA
   <213> artificial sequence
<220>
   <223> amorce/primer
<400> 62
   atgcctgcag gtcgagcgat agtaattttg ctatc 35
<210> 63
   <211> 46
   <212> DNA
   <213> artificial sequence
<220>
   <223> amorce/primer
<400> 63
   cggtacccgg ggatccagga ggattcatat gacagatatt gcgcag 46
<210> 64
   <211> 39
   <212> DNA
   <213> artificial sequence
<220>
   <223> amorce/primer
<400> 64
   tccgccaaaa cagaagcttt caggcgatag taattttgc 39
<210> 65
   <211> 47
   <212> DNA
   <213> artificial sequence
<220>
   <223> amorce/primer
<400> 65
   ctcatccgcc aaaacagaag cttttagaac tgcgattctt cagtgga 47
<210> 66
   <211> 49
   <212> DNA
   <213> artificial sequence
<220>
   <223> amorce/primer
<400> 66
   gaagaatcgc agttctaaag gaggcacacg atggatatca tcttttatc 49
<210> 67
   <211> 53
   <212> DNA
   <213> artificial sequence
<220>
   <223> amorce/primer
<400> 67
   catccgccaa aacagaagct ttctagatta gtagccgcgt gcgcggtcga ctt 53
<210> 68
   <211> 53
   <212> DNA
   <213> artificial sequence
<220>
   <223> amorce/primer
<400> 68
   catccgccaa aacagaagct ttctagatta gtagccgcgt gcgcggtcga ctt 53
<210> 69
   <211> 102
   <212> DNA
   <213> artificial sequence
<220>
   <223> amorce/primer
<400> 69
   gcgtcttgat ggcgctttac ccgatgtcga ccgcacatcg gtactgatgg cactgcgtga 60
   gcatgtccct ggacttgaga tcgtgtaggc tggagctgct tc 102
<210> 70
   <211> 99
   <212> DNA
   <213> artificial sequence
<220>
   <223> amorce/primer
<400> 70
   cgcgtaaacg ccaggcgtgt aataacggtt cggtatagcc gtttggctgt ttcacgccga 60
   ggaagattaa atcgctggcc atatgaatat cctccttag 99
<210> 71
   <211> 102
   <212> DNA
   <213> artificial sequence
<220>
   <223> amorce/primer
<400> 71
   cgcgcgagac tcgctctgct tatctcgccc ggatagaaca agcgaaaact tcgaccgttc 60
   atcgttcgca gttggcatgc gggtgtaggc tggagctgct tc 102
<210> 72
   <211> 100
   <212> DNA
   <213> artificial sequence
<220>
   <223> amorce/primer
<400> 72
   gcttagcgcc ttctacagct tcacgcgcca gcttagtaat gcggtcgtaa tcgcccgctt 60
   ccagcgcatc tgccggaacc catatgaata tcctccttag 100
<210> 73
   <211> 20
   <212> DNA
   <213> artificial sequence
<220>
   <223> amorce/primer
<400> 73
   cacagctaac accacgtcgt 20
<210> 74
   <211> 82
   <212> DNA
   <213> artificial sequence
<220>
   <223> amorce/primer
<400> 74
   acgtcattgc cttgtttgac cgcccctgtt ttttagcgtc aggcatataa tacctcctaa 60
   agttaaacaa aattatttgt ag 82
<210> 75
   <211> 70
   <212> DNA
   <213> artificial sequence
<220>
   <223> amorce/primer
<400> 75
   ccgcccgcac aataacatca ttcttcctga tcacgtttca ccgcagatta gtgtaggctg 60
   gagctgcttc 70
<210> 76
   <211> 47
   <212> DNA
   <213> artificial sequence
<220>
   <223> amorce/primer
<400> 76
   gatagggacg acgtggtgtt agctgtgcat atgaatatcc tccttag 47
<210> 77
   <211> 47
   <212> DNA
   <213> artificial sequence
<220>
   <223> amorce/primer
<400> 77
   ggatccgtcg acaggaggta acatatggaa gagaagcaga tcctgtg 47
<210> 78
   <211> 35
   <212> DNA
   <213> artificial sequence
<220>
   <223> amorce/primer
<400> 78
   ctgcagtcta gaagatctca cacgatgcca tcaaa 35
<210> 79
   <211> 44
   <212> DNA
   <213> artificial sequence
<220>
   <223> amorce/primer
<400> 79
   ttggatccaa gaaggagaat tcatatggca catcgctttc cggc 44
<210> 80
   <211> 21
   <212> DNA
   <213> artificial sequence
<220>
   <223> amorce/primer
<400> 80
   ttaatacgtg taacaggccg t 21
<210> 81
   <211> 101
   <212> DNA
   <213> artificial sequence
<220>
   <223> amorce/primer
<400> 81
   cgcacccatt cccgcgaaac gcggcagaaa acccgccgtt gccaccgcac cagcgactgg 60
   acaggttcag tctttaacgc gtgtaggctg gagctgcttc g 101
<210> 82
   <211> 100
   <212> DNA
   <213> artificial sequence
<220>
   <223> amorce/primer
<400> 82
   gcgcattcca ccgtacgcca gcgtcacttc cttcgccgct ttaatcacca tcgcgccaaa 60
   ctcggtcacg cggtcatcgg catatgaata tcctccttag 100
<210> 83
   <211> 63
   <212> DNA
   <213> artificial sequence
<220>
   <223> amorce/primer
<400> 83
   gaattgctgg cccgctgcgg cataaaaatt aattggtacc ggttatttaa actgtgagga 60
   cct 63
<210> 84
   <211> 46
   <212> DNA
   <213> artificial sequence
<220>
   <223> amorce/primer
<400> 84
   gatttgcttt tcttccattt ttagtttatg tatgtgtttt tttgta 46
<210> 85
   <211> 40
   <212> DNA
   <213> artificial sequence
<220>
   <223> amorce/primer
<400> 85
   cacatacata aactaaaaat ggaagaaaag caaatcttgt 40
<210> 86
   <211> 45
   <212> DNA
   <213> artificial sequence
<220>
   <223> amorce/primer
<400> 86
   gtttctgtct ttgcctttga tctgcttaaa cgataccgtc gaaac 45
<210> 87
   <211> 42
   <212> DNA
   <213> artificial sequence
<220>
   <223> amorce/primer
<400> 87
   ggtatcgttt aagcagatca aaggcaaaga cagaaaccgt ag 42
<210> 88
   <211> 56
   <212> DNA
   <213> artificial sequence
<220>
   <223> amorce/primer
<400> 88
   attcctatca ttatttacgt aatgacccat cttggtgtgt catcggtagt aacgcc 56
<210> 89
   <211> 49
   <212> DNA
   <213> artificial sequence
<220>
   <223> amorce/primer
<400> 89
   ccgatgacac accaagatgg gtcattacgt aaataatgat aggaatggg 49
<210> 90
   <211> 45
   <212> DNA
   <213> artificial sequence
<220>
   <223> amorce/primer
<400> 90
   gctgggaatc tgtgagccat tttgaatatg tattacttgg ttatg 45
<210> 91
   <211> 42
   <212> DNA
   <213> artificial sequence
<220>
   <223> amorce/primer
<400> 91
   tgatctacga tcagaattta gtaagtgtaa caagcagtga ac 42
<210> 92
   <211> 42
   <212> DNA
   <213> artificial sequence
<220>
   <223> amorce/primer
<400> 92
   gtaatacata ttcaaaatgg ctcacagatt cccagctttg ac 42
<210> 93
   <211> 49
   <212> DNA
   <213> artificial sequence
<220>
   <223> amorce/primer
<400> 93
   gcttgttaca cttactaaat tctgatcgta gatcatcaga tttgatatg 49
<210> 94
   <211> 66
   <212> DNA
   <213> artificial sequence
<220>
   <223> amorce/primer
<400> 94
   ggtgacccgg cggggacgag gcaagctaaa cagatctcta gaataatttt ttatattctt 60
tattgg 66
<210> 95
   <211> 25
   <212> DNA
   <213> artificial sequence
<220>
   <223> amorce/primer
<400> 95
   gagattttgt tcttctgagc ttctg 25
<210> 96
   <211> 25
   <212> DNA
   <213> artificial sequence
<220>
   <223> amorce/primer
<400> 96
   gaaattgcac ttagcatttt tgaat 25
<210> 97
   <211> 897
   <212> DNA
   <213> Saccharomyces cerevisiae
<400> 97
   atggaagaaa agcaaatctt gtgtgttggt ttggttgttt tggacgttat ctctttggtt        60
   gacaagtacc caaaggaaga ctctgaaatc agatgtttgt ctcaaagatg gcaaagaggt       120
   ggtaacgctt ctaactcttg tactgttttg tctttgttgg gtgctccatg tgctttcatg       180
   ggttctatgg ctccaggtca cgttgctgac ttcttggttg ctgacttcag aaggagaggt       240
   gttgacgttt ctcaagttgc ttggcaatct aagggtgaca ctccatcttc ttgttgtatc       300
   atcaacaact ctaacggtaa cagaactatc gttttgcacg acacttcttt gccagacgtt       360
   tctgctactg acttcgaaaa ggttgacttg actcaattca agtggatcca catcgaaggt       420
   agaaacgctt ctgaacaagt taagatgttg caaagaatcg acgctcacaa cactagacaa       480
   ccaccagaac aaaagatcag agtttctgtt gaagttgaaa agccaagaga agaattgttc       540
   caattgttcg gttacggtga cgttgttttc gtttctaagg acgttgctaa gcacttgggt       600
   ttccaatctg ctgaagaagc tttgagaggt ttgtacggta gagttagaaa gggtgctgtt       660
   ttggtttgtg cttgggctga agaaggtgct gacgctttgg gtccagacgg taagttgttg       720
   cactctgacg ctttcccacc gccaagagtt gttgacactt tgggtgctgg tgacactttc       780
   aacgcttctg ttatcttctc tttgtctcaa ggtagatctg ttcaagaagc tttgagattc       840
   ggttgtcaag ttgctggtaa gaagtgtggt ttgcaaggtt tcgacggtat cgtttaa          897
<210> 98
   <211> 1095
   <212> DNA
   <213> saccharomyces cerevisiae
<400> 98
   atggctcaca gattcccagc tttgactcaa gaacaaaaga aggaattgtc tgaaatcgct 60
   caatctatcg ttgctaacgg taagggtatc ttggctgctg acgaatctgt tggtactatg 120
   ggtaacagat tgcaaagaat caaggttgaa aacactgaag aaaacagaag acaattcaga 180
   gaaatcttgt tctctgttga ctcttctatc aaccaatcta tcggtggtgt tatcttgttc 240
   cacgaaactt tgtaccaaaa ggactctcaa ggtaagttgt tcagaaacat cttgaaggaa 300
   aagggtatcg ttgttggtat caagttggac caaggtggtg ctccattggc tggtactaac 360
   aaggaaacta ctatccaagg tttggacggt ttgtctgaaa gatgtgctca atacaagaag 420
   gacggtgttg acttcggtaa gtggagagct gttttgagaa tcgctgacca atgtccatct 480
   tctttggcta tccaagaaaa cgctaacgct ttggctagat acgcttctat ctgtcaacaa 540
   aacggtttgg ttccaatcgt tgaaccagaa gttatcccag acggtgacca cgacttggaa 600
   cactgtcaat acgttactga aaaggttttg gctgctgttt acaaggcttt gaacgaccac 660
   cacgtttact tggaaggtac tttgttgaag ccaaacatgg ttactgctgg tcacgcttgt 720
   actaagaagt acactccaga acaagttgct atggctactg ttactgcttt gcacagaact 780
   gttccagctg ctgttccagg tatctgtttc ttgtctggtg gtatgtctga agaagacgct 840
   actttgaact tgaacgctat caacttgtgt ccattgccaa agccatggaa gttgtctttc 900
   tcttacggta gagctttgca agcttctgct ttggctgctt ggggtggtaa ggctgctaac 960
   aaggaagcta ctcaagaagc tttcatgaag agagctatgg ctaactgtca agctgctaag 1020
   ggtcaatacg ttcacactgg ttcttctggt gctgcttcta ctcaatcttt gttcactgct 1080
   tgttacactt actaa 1095
<210> 99
   <211> 10023
   <212> DNA
   <213> Saccharomyces cerevisiae
<400> 99
   ggtaccttta cgatagagcc agagacgatg ccgacgaaga tgaagaagat cccgacacaa 60
   gaagctccgg taagaagaag gacgccagcc aagaagaatc tctaatctaa gaggacggtt 120
   gctgaagaaa aaggcttttt ttattttgtc cgtttttttt ttgtaaaacc caaagatctg 180
   aatctaaagc ttttttaaac gtatatagat gtctacatgt gtgtttttgt ttttttacgt 240
   acgtataccc acctatatat gcataatccg taattgaaaa aaaaaaaaga aaaagatcaa 300
   ggaacacatc accctgggca catcaagcgt gaggaatgcc gtccaactgg tggagacgct 360
   tgatttgctc tttttgttct gggtccaacc cggtctcgaa gaacatcagc accacgcccg 420
   caacgacaaa gaacattgca atacacttgc atatgtgagc atagtcgagc ggtccgttct 480
   gtggttgatg ctgttgttct ttcttctgtt tgtcaggggt gatagccata tcttcgtgct 540
   cttgttgcga ttgttctgtt ccatctgcac cagaacaaag aacaaaagaa caaggaacaa 600
   agtccaagca cgtcagcgct gtttataagg ggattgacga gggatcgggc ctagagtgcc 660
   agcgcgccag ggagagggag ccccctgggc cctcatccgc aggctgatag gggtcacccc 720
   gctgggcagg tcagggcagg ggctctcagg ggggcgccat ggacaaactg cactgaggtt 780
   ctaagacaca tgtattattg tgagtatgta tatatagaga gagattaagg cgtacagccg 840
   ggtggtagag attgattaac ttggtagtct tatcttgtca attgagtttc tgtcagtttc 900
   ttcttgaaca agcacgcagc taagtaagca acaaagcagg ctaacaaact aggtactcac 960
   atacagactt attaagacat agaactatga ctacggataa cgctaaggcg caactgacct 1020
   cgtcttcagg gggtaacatt attgtggtgt ccaacaggct tcccgtgaca atcactaaaa 1080
   acagcagtac gggacagtac gagtacgcaa tgtcgtccgg agggctggtc acggcgttgg 1140
   aagggttgaa gaagacgtac actttcaagt ggttcggatg gcctgggcta gagattcctg 1200
   acgatgagaa ggatcaggtg aggaaggact tgctggaaaa gtttaatgcc gtacccatct 1260
   tcctgagcga tgaaatcgca gacttacact acaacgggtt cagtaattct attctatggc 1320
   cgttattcca ttaccatcct ggtgagatca atttcgacga gaatgcgtgg ttggcataca 1380
   acgaggcaaa ccagacgttc accaacgaga ttgctaagac tatgaaccat aacgatttaa 1440
   tctgggtgca tgattaccat ttgatgttgg ttccggaaat gttgagagtc aagattcacg 1500
   agaagcaact gcaaaacgtt aaggtcgggt ggttcctgca cacaccattc ccttcgagtg 1560
   aaatttacag aatcttacct gtcagacaag agattttgaa gggtgttttg agttgtgatt 1620
   tagtcgggtt ccacacatac gattatgcaa gacatttctt gtcttccgtg caaagagtgc 1680
   ttaacgtgaa cacattgcct aatggggtgg aataccaggg cagattcgtt aacgtagggg 1740
   ccttccctat cggtatcgac gtggacaagt tcaccgatgg gttgaaaaag gaatccgtac 1800
   aaaagagaat ccaacaattg aaggaaactt tcaagggctg caagatcata gttggtgtcg 1860
   acaggctgga ttacatcaaa ggtgtgcctc agaagttgca cgccatggaa gtgtttctga 1920
   acgagcatcc agaatggagg ggcaaggttg ttctggtaca ggttgcagtg ccaagtcgtg 1980
   gagatgtgga agagtaccaa tatttaagat ctgtggtcaa tgagttggtc ggtagaatca 2040
   acggtcagtt cggtactgtg gaattcgtcc ccatccattt catgcacaag tctataccat 2100
   ttgaagagct gatttcgtta tatgctgtga gcgatgtttg tttggtctcg tccacccgtg 2160
   atggtatgaa cttggtttcc tacgaatata ttgcttgcca agaagaaaag aaaggttcct 2220
   taatcctgag tgagttcaca ggtgccgcac aatccttgaa tggtgctatt attgtaaatc 2280
   cttggaacac cgatgatctt tctgatgcca tcaacgaggc cttgactttg cccgatgtaa 2340
   agaaagaagt taactgggaa aaactttaca aatacatctc taaatacact tctgccttct 2400
   ggggtgaaaa tttcgtccat gaattataca gtacatcatc aagctcaaca agctcctctg 2460
   ccaccaaaaa ctgatgaacc cgatgcaaat gagacgatcg tctattcctg gtccggtttt 2520
   ctctgccctc tcttctattc acttttttta tactttatat aaaattatat aaatgacata 2580
   actgaaacgc cacacgtcct ctcctattcg ttaacgcctg tctgtagcgc tgttactgaa 2640
   gctgcgcaag tagttttttc accgtatagg atgacaaccc ttaatataac ttcgtataat 2700
   gtatgctata cgaagttatt aggtctagag atctgtttag cttgcctcgt ccccgccggg 2760
   tcacccggcc agcgacatgg aggcccagaa taccctcctt gacagtcttg acgtgcgcag 2820
   ctcaggggca tgatgtgact gtcgcccgta catttagccc atacatcccc atgtataatc 2880
   atttgcatcc atacattttg atggccgcac ggcgcgaagc aaaaattacg gctcctcgct 2940
   gcagacctgc gagcagggaa acgctcccct cacagacgcg ttgaattgtc cccacgccgc 3000
   gcccctgtag agaaatataa aaggttagga tttgccactg aggttcttct ttcatatact 3060
   tccttttaaa atcttgctag gatacagttc tcacatcaca tccgaacata aacaaccatg 3120
   ggtaaggaaa agactcacgt ttcgaggccg cgattaaatt ccaacatgga tgctgattta 3180
   tatgggtata aatgggctcg cgataatgtc gggcaatcag gtgcgacaat ctatcgattg 3240
   tatgggaagc ccgatgcgcc agagttgttt ctgaaacatg gcaaaggtag cgttgccaat 3300
   gatgttacag atgagatggt cagactaaac tggctgacgg aatttatgcc tcttccgacc 3360
   atcaagcatt ttatccgtac tcctgatgat gcatggttac tcaccactgc gatccccggc 3420
   aaaacagcat tccaggtatt agaagaatat cctgattcag gtgaaaatat tgttgatgcg 3480
   ctggcagtgt tcctgcgccg gttgcattcg attcctgttt gtaattgtcc ttttaacagc 3540
   gatcgcgtat ttcgtctcgc tcaggcgcaa tcacgaatga ataacggttt ggttgatgcg 3600
   agtgattttg atgacgagcg taatggctgg cctgttgaac aagtctggaa agaaatgcat 3660
   aagcttttgc cattctcacc ggattcagtc gtcactcatg gtgatttctc acttgataac 3720
   cttatttttg acgaggggaa attaataggt tgtattgatg ttggacgagt cggaatcgca 3780
   gaccgatacc aggatcttgc catcctatgg aactgcctcg gtgagttttc tccttcatta 3840
   cagaaacggc tttttcaaaa atatggtatt gataatcctg atatgaataa attgcagttt 3900
   catttgatgc tcgatgagtt tttctaatca gtactgacaa taaaaagatt cttgttttca 3960
   agaacttgtc atttgtatag tttttttata ttgtagttgt tctattttaa tcaaatgtta 4020
   gcgtgattta tatttttttt cgcctcgaca tcatctgccc agatgcgaag ttaagtgcgc 4080
   agaaagtaat atcatgcgtc aatcgtatgt gaatgctggt cgctatactg ctgtcgattc 4140
   gatactaacg ccgccatcca gtgtcgaaaa cgagctctcg agaaccctta atataacttc 4200
   gtataatgta tgctatacga agttattagg tgatatcgga tccggatcct cagcagcgaa 4260
   acgttgttct gggaaaccat ggagaaactg aaagcgcttg gcgccagctc gattctggta 4320
   ctgccgatcg agaagatgat ggagtgagca tgcaagcttg gcgtaatcat ggtcatagct 4380
   gtttcctgtg tgaaattgtt atccgctcac aattccacac aacatacgag ccggaagcat 4440
   aaagtgtaaa gcctggggtg cctaatgagt gagctaactc acattaattg cgttgcgctc 4500
   actgcccgct ttccagtcgg gaaacctgtc gtgccagctg cattaatgaa tcggccaacg 4560
   cgcggggaga ggcggtttgc gtattgggcg ctcttccgct tcctcgctca ctgactcgct 4620
   gcgctcggtc gttcggctgc ggcgagcggt atcagctcac tcaaaggcgg taatacggtt 4680
   atccacagaa tcaggggata acgcaggaaa gaacatgtga gcaaaaggcc agcaaaaggc 4740
   caggaaccgt aaaaaggccg cgttgctggc gtttttccat aggctccgcc cccctgacga 4800
   gcatcacaaa aatcgacgct caagtcagag gtggcgaaac ccgacaggac tataaagata 4860
   ccaggcgttt ccccctggaa gctccctcgt gcgctctcct gttccgaccc tgccgcttac 4920
   cggatacctg tccgcctttc tcccttcggg aagcgtggcg ctttctcata gctcacgctg 4980
   taggtatctc agttcggtgt aggtcgttcg ctccaagctg ggctgtgtgc acgaaccccc 5040
   cgttcagccc gaccgctgcg ccttatccgg taactatcgt cttgagtcca acccggtaag 5100
   acacgactta tcgccactgg cagcagccac tggtaacagg attagcagag cgaggtatgt 5160
   aggcggtgct acagagttct tgaagtggtg gcctaactac ggctacacta gaaggacagt 5220
   atttggtatc tgcgctctgc tgaagccagt taccttcgga aaaagagttg gtagctcttg 5280
   atccggcaaa caaaccaccg ctggtagcgg tggttttttt gtttgcaagc agcagattac 5340
   gcgcagaaaa aaaggatctc aagaagatcc tttgatcttt tctacggggt ctgacgctca 5400
   gtggaacgaa aactcacgtt aagggatttt ggtcatgaga ttatcaaaaa ggatcttcac 5460
   ctagatcctt ttaaattaaa aatgaagttt taaatcaatc taaagtatat atgagtaaac 5520
   ttggtctgac agttaccaat gcttaatcag tgaggcacct atctcagcga tctgtctatt 5580
   tcgttcatcc atagttgcct gactccccgt cgtgtagata actacgatac gggagggctt 5640
   accatctggc cccagtgctg caatgatacc gcgagaccca cgctcaccgg ctccagattt 5700
   atcagcaata aaccagccag ccggaagggc cgagcgcaga agtggtcctg caactttatc 5760
   cgcctccatc cagtctatta attgttgccg ggaagctaga gtaagtagtt cgccagttaa 5820
   tagtttgcgc aacgttgttg ccattgctac aggcatcgtg gtgtcacgct cgtcgtttgg 5880
   tatggcttca ttcagctccg gttcccaacg atcaaggcga gttacatgat cccccatgtt 5940
   gtgcaaaaaa gcggttagct ccttcggtcc tccgatcgtt gtcagaagta agttggccgc 6000
   agtgttatca ctcatggtta tggcagcact gcataattct cttactgtca tgccatccgt 6060
   aagatgcttt tctgtgactg gtgagtactc aaccaagtca ttctgagaat agtgtatgcg 6120
   gcgaccgagt tgctcttgcc cggcgtcaat acgggataat accgcgccac atagcagaac 6180
   tttaaaagtg ctcatcattg gaaaacgttc ttcggggcga aaactctcaa ggatcttacc 6240
   gctgttgaga tccagttcga tgtaacccac tcgtgcaccc aactgatctt cagcatcttt 6300
   tactttcacc agcgtttctg ggtgagcaaa aacaggaagg caaaatgccg caaaaaaggg 6360
   aataagggcg acacggaaat gttgaatact catactcttc ctttttcaat attattgaag 6420
   catttatcag ggttattgtc tcatgagcgg atacatattt gaatgtattt agaaaaataa 6480
   acaaataggg gttccgcgca catttccccg aaaagtgcca cctgacgtct aagaaaccat 6540
   tattatcatg acattaacct ataaaaatag gcgtatcacg aggccctttc gtcttcaaga 6600
   attagctttt caattcaatt catcattttt tttttattct tttttttgat ttcggtttct 6660
   ttgaaatttt tttgattcgg taatctccga acagaaggaa gaacgaagga aggagcacag 6720
   acttagattg gtatatatac gcatatgtag tgttgaagaa acatgaaatt gcccagtatt 6780
   cttaacccaa ctgcacagaa caaaaacatg caggaaacga agataaatca tgtcgaaagc 6840
   tacatataag gaacgtgctg ctactcatcc tagtcctgtt gctgccaagc tatttaatat 6900
   catgcacgaa aagcaaacaa acttgtgtgc ttcattggat gttcgtacca ccaaggaatt 6960
   actggagtta gttgaagcat taggtcccaa aatttgttta ctaaaaacac atgtggatat 7020
   cttgactgat ttttccatgg agggcacagt taagccgcta aaggcattat ccgccaagta 7080
   caatttttta ctcttcgaag acagaaaatt tgctgacatt ggtaatacag tcaaattgca 7140
   gtactctgcg ggtgtataca gaatagcaga atgggcagac attacgaatg cacacggtgt 7200
   ggtgggccca ggtattgtta gcggtttgaa gcaggcggca gaagaagtaa caaaggaacc 7260
   tagaggcctt ttgatgttag cagaattgtc atgcaagggc tccctatcta ctggagaata 7320
   tactaagggt actgttgaca ttgcgaagag cgacaaagat tttgttatcg gctttattgc 7380
   tcaaagagac atgggtggaa gagatgaagg ttacgattgg ttgattatga cacccggtgt 7440
   gggtttagat gacaagggag acgcattggg tcaacagtat agaaccgtgg atgatgtggt 7500
   ctctacagga tctgacatta ttattgttgg aagaggacta tttgcaaagg gaagggatgc 7560
   taaggtagag ggtgaacgtt acagaaaagc aggctgggaa gcatatttga gaagatgcgg 7620
   ccagcaaaac taaaaaactg tattataagt aaatgcatgt atactaaact cacaaattag 7680
   agcttcaatt taattatatc agttattacc caattctcat gtttgacagc ttatcatcga 7740
   tcgtccaact gcatggagat gagtcgtggc aagaatacca agagttcctc ggtttgccag 7800
   ttattaaaag actcgtattt ccaaaagact gcaacatact actcagtgca gcttcacaga 7860
   aacctcattc gtttattccc ttgtttgatt cagaagcagg tgggacaggt gaacttttgg 7920
   attggaactc gatttctgac tgggttggaa ggcaagagag ccccgagagc ttacatttta 7980
   tgttagctgg tggactgacg ccagaaaatg ttggtgatgc gcttagatta aatggcgtta 8040
   ttggtgttga tgtaagcgga ggtgtggaga caaatggtgt aaaagactct aacaaaatag 8100
   caaatttcgt caaaaatgct aagaaatagg ttattactga gtagtattta tttaagtatt 8160
   gtttgtgcac ttgcctgcaa gccttttgaa aagcaagcat aaaagatcta aacataaaat 8220
   ctgtaaaata acaagatgta aagataatgc taaatcattt ggctttttga ttgattgtac 8280
   aggaaaatat acatcgcagg gggttgactt ttaccatttc accgcaatgg aatcaaactt 8340
   gttgaagaga atgttcacag gcgcatacgc tacaatgacc cgattcttgc tagccttttc 8400
   tcggtcttgc aaacaaccgc cggcagctta gtatataaat acacatgtac atacctctct 8460
   ccgtatcctc gtaatcattt tcttgtattt atcgtctttt cgctgtaaaa actttatcac 8520
   acttatctca aatacactta ttaaccgctt ttactattat cttctacgct gacagtaata 8580
   tcaaacagtg acacatatta aacacagtgg tttctttgca taaacaccat cagcctcaag 8640
   tcgtcaagta aagatttcgt gttcatgcag atagataaca atctatatgt tgataattag 8700
   cgttgcctca tcaatgcgag atccgtttaa ccggacccta gtgcacttac cccacgttcg 8760
   gtccactgtg tgccgaacat gctccttcac tattttaaca tgtggaatta attctcatgt 8820
   ttgacagctt atcatcgaac tctaagaggt gatacttatt tactgtaaaa ctgtgacgat 8880
   aaaaccggaa ggaagaataa gaaaactcga actgatctat aatgcctatt ttctgtaaag 8940
   agtttaagct atgaaagcct cggcattttg gccgctccta ggtagtgctt tttttccaag 9000
   gacaaaacag tttctttttc ttgagcaggt tttatgtttc ggtaatcata aacaataaat 9060
   aaattatttc atttatgttt aaaaataaaa aataaaaaag tattttaaat ttttaaaaaa 9120
   gttgattata agcatgtgac cttttgcaag caattaaatt ttgcaatttg tgattttagg 9180
   caaaagttac aatttctggc tcgtgtaata tatgtatgct aaagtgaact tttacaaagt 9240
   cgatatggac ttagtcaaaa gaaattttct taaaaatata tagcactagc caatttagca 9300
   cttctttatg agatatatta tagactttat taagccagat ttgtgtatta tatgtattta 9360
   cccggcgaat catggacata cattctgaaa taggtaatat tctctatggt gagacagcat 9420
   agataaccta ggatacaagt taaaagctag tactgttttg cagtaatttt tttctttttt 9480
   ataagaatgt taccacctaa ataagttata aagtcaatag ttaagtttga tatttgattg 9540
   taaaataccg taatatattt gcatgatcaa aaggctcaat gttgactagc cagcatgtca 9600
   accactatat tgatcaccga tatatggact tccacaccaa ctagtaatat gacaataaat 9660
   tcaagatatt cttcatgaga atggcccagc gatatatgcg gtgtgaaata ccgcacagat 9720
   gcgtaaggag aaaataccgc atcaggcgcc attcgccatt caggctgcgc aactgttggg     9780
   aagggcgatc ggtgcgggcc tcttcgctat tacgccagct ggcgaaaggg ggatgtgctg     9840
   caaggcgatt aagttgggta acgccagggt tttcccagtc acgacgttgt aaaacgacgg     9900
   ccagtgaatt cgagctcatg ttagacaaca cccgcttacg catagctatt cagaaatcag     9960
   gccgtttaag cgatgattca cgagaattgc tggcccgctg cggcataaaa attaattggt    10020
   acc                                                                  10023
<210> 100
   <211> 1701
   <212> DNA
   <213> Saccharomyces cerevisiae
<400> 100
   atggcgattg caattggcct cgattttggc agtgattctg tgcgagcttt ggcggtggac 60
   tgcgctaccg gtgaagagat cgccaccagc gtagagtggt atccccgttg gcagaaaggg 120
   caattttgtg atgccccgaa taaccagttc cgtcatcatc cgcgtgacta cattgagtca 180
   atggaagcgg cactgaaaac cgtgcttgca gagcttagcg tcgaacagcg cgcagctgtg 240
   gtcgggattg gcgttgacag taccggctcg acgcccgcac cgattgatgc cgacggaaac 300
   gtgctggcgc tgcgcccgga gtttgccgaa aacccgaacg cgatgttcgt attgtggaaa 360
   gaccacactg cggttgaaga agcggaagag attacccgtt tgtgccacgc gccgggcaac 420
   gttgactact cccgctacat tggtggtatt tattccagcg aatggttctg ggcaaaaatc 480
   ctgcatgtga ctcgccagga cagcgccgtg gcgcaatctg ccgcatcgtg gattgagctg 540
   tgcgactggg tgccagctct gctttccggt accacccgcc cgcaggatat tcgtcgcgga 600
   cgttgcagcg ccgggcataa atctctgtgg cacgaaagct ggggcggcct gccgccagcc 660
   agtttctttg atgagctgga cccgatcctc aatcgccatt tgccttcccc gctgttcact 720
   gacacttgga ctgccgatat tccggtgggc accttatgcc cggaatgggc gcagcgtctc 780
   ggcctgcctg aaagcgtggt gatttccggc ggcgcgtttg actgccatat gggcgcagtt 840
   ggcgcaggcg cacagcctaa cgcactggta aaagttatcg gtacttccac ctgcgacatt 900
   ctgattgccg acaaacagag cgttggcgag cgggcagtta aaggtatttg cggtcaggtt 960
   gatggcagcg tggtgcctgg atttatcggt ctggaagcag gccaatcggc gtttggtgat 1020
   atctacgcct ggtttggtcg cgtactcggc tggccgctgg aacagcttgc cgcccagcat 1080
   ccggaactga aaacgcaaat caacgccagc cagaaacaac tgcttccggc gctgaccgaa 1140
   gcatgggcca aaaatccgtc tctggatcac ctgccggtgg tgctcgactg gtttaacggc 1200
   cgccgcacac cgaacgctaa ccaacgcctg aaaggggtga ttaccgatct taacctcgct 1260
   accgacgctc cgctgctgtt cggcggtttg attgctgcca ccgcctttgg cgcacgcgca 1320
   atcatggagt gctttaccga tcaggggatc gccgttaata acgtgatggc actgggcggc 1380
   atcgcgcgga aaaaccaggt cattatgcag gcctgctgcg acgtgctgaa tcgcccgctg 1440
   caaattgttg cctctgacca gtgctgtgcg ctcggtgcgg cgatttttgc tgccgtcgcc 1500
   gcgaaagtgc acgcagacat cccatcagct cagcaaaaaa tggccagtgc ggtagagaaa 1560
   accctgcaac cgtgcagcga gcaggcacaa cgctttgaac agctttatcg ccgctatcag 1620
   caatgggcga tgagcgccga acaacactat cttccaactt ccgccccggc acaggctgcc 1680
   caggccgttg cgactctata a 1701
<210> 101
   <211> 1803
   <212> DNA
   <213> Saccharomyces cerevisiae
<400> 101
   atgttgtgtt cagtaattca gagacagaca agagaggttt ccaacacaat gtctttagac      60
   tcatactatc ttgggtttga tctttcgacc caacaactga aatgtctcgc cattaaccag     120
   gacctaaaaa ttgtccattc agaaacagtg gaatttgaaa aggatcttcc gcattatcac     180
   acaaagaagg gtgtctatat acacggcgac actatcgaat gtcccgtagc catgtggtta     240
   gaggctctag atctggttct ctcgaaatat cgcgaggcta aatttccatt gaacaaagtt     300
   atggccgtct cagggtcctg ccagcagcac gggtctgtct actggtcctc ccaagccgaa     360
   tctctgttag agcaattgaa taagaaaccg gaaaaagatt tattgcacta cgtgagctct     420
   gtagcatttg caaggcaaac cgcccccaat tggcaagacc acagtactgc aaagcaatgt     480
   caagagtttg aagagtgcat aggtgggcct gaaaaaatgg ctcaattaac agggtccaga     540
   gcccatttta gatttactgg tcctcaaatt ctgaaaattg cacaattaga accagaagct     600
   tacgaaaaaa caaagaccat ttctttagtg tctaattttt tgacttctat cttagtgggc     660
   catcttgttg aattagagga ggcagatgcc tgtggtatga acctttatga tatacgtgaa     720
   agaaaattca gtgatgagct actacatcta attgatagtt cttctaagga taaaactatc     780
   agacaaaaat taatgagagc acccatgaaa aatttgatag cgggtaccat ctgtaaatat     840
   tttattgaga agtacggttt caatacaaac tgcaaggtct ctcccatgac tggggataat     900
   ttagccacta tatgttcttt acccctgcgg aagaatgacg ttctcgtttc cctaggaaca     960
   agtactacag ttcttctggt caccgataag tatcacccct ctccgaacta tcatcttttc    1020
   attcatccaa ctctgccaaa ccattatatg ggtatgattt gttattgtaa tggttctttg    1080
   gcaagggaga ggataagaga cgagttaaac aaagaacggg aaaataatta tgagaagact    1140
   aacgattgga ctctttttaa tcaagctgtg ctagatgact cagaaagtag tgaaaatgaa    1200
   ttaggtgtat attttcctct gggggagatc gttcctagcg taaaagccat aaacaaaagg    1260
   gttatcttca atccaaaaac gggtatgatt gaaagagagg tggccaagtt caaagacaag    1320
   aggcacgatg ccaaaaatat tgtagaatca caggctttaa gttgcagggt aagaatatct    1380
   cccctgcttt cggattcaaa cgcaagctca caacagagac tgaacgaaga tacaatcgtg    1440
   aagtttgatt acgatgaatc tccgctgcgg gactacctaa ataaaaggcc agaaaggact    1500
   ttttttgtag gtggggcttc taaaaacgat gctattgtga agaagtttgc tcaagtcatt    1560
   ggtgctacaa agggtaattt taggctagaa acaccaaact catgtgccct tggtggttgt    1620
   tataaggcca tgtggtcatt gttatatgac tctaataaaa ttgcagttcc ttttgataaa    1680
   tttctgaatg acaattttcc atggcatgta atggaaagca tatccgatgt ggataatgaa    1740
   aattgggatc gctataattc caagattgtc cccttaagcg aactggaaaa gactctcatc    1800
   taa                                                                   1803
<210> 102
   <211> 160
   <212> DNA
   <213> Saccharomyces cerevisiae
<400> 102
   ttctagagca cagctaacac cacgtcgtcc ctatctgctg ccctaggtct atgagtggtt      60
   gctggataac tttacgggca tgcataaggc tcgtataata tattcaggga gaccacaacg     120
   gtttccctct acaaataatt ttgtttaact tttactagag                           160